(19) Europäisches Patentamt
European Patent Office
Office européen des brevets

(11) EP 2 123 654 A1

(12) **EUROPEAN PATENT APPLICATION**
published in accordance with Art. 153(4) EPC

(43) Date of publication:
**25.11.2009 Bulletin 2009/48**

(21) Application number: **07860481.6**

(22) Date of filing: **28.12.2007**

(51) Int Cl.:
***C07D 487/04*** (2006.01) ***A61K 31/519*** (2006.01)
***A61K 31/5377*** (2006.01) ***A61K 31/538*** (2006.01)
***A61K 31/541*** (2006.01) ***A61K 31/55*** (2006.01)
***A61K 31/553*** (2006.01) ***A61P 19/02*** (2006.01)
***A61P 37/06*** (2006.01) ***A61P 37/08*** (2006.01)
***A61P 43/00*** (2006.01) ***C07D 519/00*** (2006.01)

(86) International application number:
**PCT/JP2007/075278**

(87) International publication number:
**WO 2008/081928 (10.07.2008 Gazette 2008/28)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **28.12.2006 JP 2006353781**

(71) Applicants:
- **Taisho Pharmaceutical Co. Ltd.**
  **Tokyo 170-8633 (JP)**
- **Nissan Chemical Industries, Ltd.**
  **Tokyo 101-0054 (JP)**

(72) Inventors:
- **YAGI, Makoto**
  **Toshima-ku**
  **Tokyo 170-8633 (JP)**
- **UMEMIYA, Hiroki**
  **Toshima-ku**
  **Tokyo 170-8633 (JP)**
- **ASANUMA, Hajime**
  **Toshima-ku**
  **Tokyo 170-8633 (JP)**
- **OKA, Yusuke**
  **Toshima-ku**
  **Tokyo 170-8633 (JP)**
- **NISHIKAWA, Rie**
  **Toshima-ku**
  **Tokyo 170-8633 (JP)**
- **HAYASHI, Keishi**
  **Funabashi-shi**
  **Chiba 274-8507 (JP)**
- **OKADA, Takumi**
  **Funabashi-shi**
  **Chiba 274-8507 (JP)**
- **SHIMIZU, Takanori**
  **Funabashi-shi**
  **Chiba 274-8507 (JP)**
- **SASAKO, Shigetada**
  **Funabashi-shi**
  **Chiba 274-8507 (JP)**

(74) Representative: **HOFFMANN EITLE**
**Patent- und Rechtsanwälte**
**Arabellastrasse 4**
**81925 München (DE)**

(54) **PYRAZOLOPYRIMIDINE COMPOUND**

(57) Disclosed is a novel compound having Syk and/or Abl inhibitory activities, which is useful for prevention/treatment of allergic diseases, autoimmune diseases, arthritides and cancers. Specifically disclosed is a pyrazolopyrimidine compound represented by the formula [I] or [III] below, a tautomer thereof, a stereoisomer thereof, a pharmaceutically acceptable salt thereof, or a solvent of any of them.

EP 2 123 654 A1

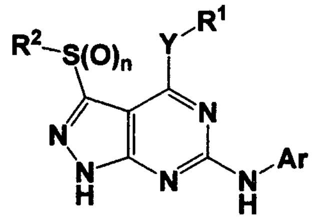

[I]

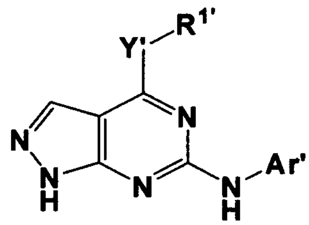

[III]

**Description**

TECHNICAL FIELD

The present invention relates to a novel pyrazolopyrimidine compound having Syk (spleen tyrosine kinase) and/or Abl (abelson tyrosine kinase) inhibitory activity, and to a medicine comprising the compound as an active ingredient.

BACKGROUND ART

Immediate allergic reactions playing a central role in allergic diseases typified by allergic rhinitis, atopic dermatitis, bronchial asthma and the like are known to be initiated by interaction between an antigen such as pollen, mite or house dust and immunoglobulin E (IgE) specific to the antigen.

Mast cells have a high-affinity IgE receptor (FcεRI) on their cell surface, abundantly contains, in the cytoplasm, granules containing histamine and other biologically active substances, and play an important role as cells as initiators of allergic disease.

Entrance of an antigen crosslinks the antigen-specific IgE on FcεRI and activates mast cells. Then, degranulation of intracellular granules occurs and lipid mediators such as prostaglandins and leukotrienes which are arachidonic acid metabolites, chemical mediators such as serotonin, and various cytokines are produced. Thus, an allergic reaction is induced.

FcεRI is composed of α, β and γ subunits (three subunits). The α-chain is the molecule exposed on the cell surface and constitutes the IgE-binding site. The β-chain is a four-transmembrane molecule and has, in the intracellular domain, an ITAM (immunoreceptor tyrosine-based activation motif) to transduce cell activation signals. The γ-chain is a single transmembrane molecule, forms a dimer, has an ITAM in the intracellular domain as does the β-chain, and is a molecule essential for expression on the cell membrane and activation signal transduction.

Syk is a non-receptor protein tyrosine kinase important for signal transduction via T-cell receptors and is a molecule classified, together with ZAP-70, into a subfamily referred to as Syk family.

Although Syk is not constitutively associated with FcεRI, Syk strongly binds to FcεRI through its SH2 domain when the intracellular tyrosine residues of the γ-chain are phosphorylated by Lyn which is a kinase of the Src family, due to aggregation of FcεRI. It has been found that because of this binding, Syk is autophosphorylated and phosphorylated by Lyn, multimerized, and therefore activated.

The activation of Syk causes degranulation, release of lipid mediators, and production of cytokines, active oxygen and the like. As a result of a study on Syk knockout mice, it has been observed that degranulation is inhibited and production of cytokines is inhibited in mast cells in an IgE-dependent manner, and production of active oxygen is inhibited in neutrophils (NON-PATENT DOCUMENT 1). The above-described inhibitory effects by Syk-specific inhibitors (NON-PATENT DOCUMENT 2) and Syk antisense oligodeoxynucleotides (NON-PATENT DOCUMENT 3) have been reported for an ovalbumin-sensitized asthma model.

On the other hand, it has been reported that differentiation of bone marrow cells obtained from Syk knockout mice into osteoclasts is inhibited (NON-PATENT DOCUMENT 4). Syk has also been suggested to be involved in signal transduction of immunoglobulin G receptors (FcγR). It has been reported that expression of Syk in the synovial tissue is significantly higher in human rheumatoid arthritis patients than in healthy subjects and osteoarthritis patients (NON-PATENT DOCUMENT 5). Further, Syk inhibitors have paw edema and joint destruction inhibitory effects in a mouse anti-collagen antibody arthritis model (NON-PATENT DOCUMENT 6).

Accordingly, Syk inhibitors are assumed to be effective for treating or preventing diseases such as allergic disease, autoimmune disease and arthritis.

Conventionally, some imidazopyrimidine compounds (PATENT DOCUMENT 1), naphthyridine compounds (PATENT DOCUMENT 2, NON-PATENT DOCUMENT 7), heterocyclic carboxamide compounds (PATENT DOCUMENT 3, NON-PATENT DOCUMENT 8), purine compounds (PATENT DOCUMENT 4), imidazopyrimidine compounds (PATENT DOCUMENT 5), triazole compounds (PATENT DOCUMENT 6), aminopyridine compounds (PATENT DOCUMENT 7) and aminopyridine compounds (PATENT DOCUMENT 8) have been reported as Syk inhibitors; however, it is not known that the compound of the present invention has a Syk inhibitory effect.

Abl is known to be activated by a growth factor such as PDGF (platelet-derived growth factor) or EGF (epidermal growth factor). Abl is activated by PDGF through activation of Src (sarcoma virus tyrosine kinase), and Src directly phosphorylates Y412 and Y245 necessary for activating Abl. It has become known that the PLCγ1 (phospholipase C gamma1) pathway, activated by PDGF stimulation as in Src, is also stimulated through activation of Abl. Activation of Abl is shown to be important for PDGF-induced cell growth, PDGF-derived cell membrane ruffling and PLCγ1-induced cell migration.

These growth factor receptors upstream of Abl, namely Src and PLCγ1, are often outside the physiological activity control in solid cancers such as breast cancer, and their activation is related with an increase in the tumor invasion

ability and a decrease in the efficacy of tumor treatment. Since Abl is involved in cytoskeletal reorganization and cell invasion, Abl may increase growth and invasion ability of cancer cells in solid cancer having constitutively activated growth factor receptors and activated Src (NON-PATENT DOCUMENT 9).

Recently, it has been shown that si-RNA of c-abl (cellular oncogene-abl) and ST-1571 having an Abl inhibitory effect can inhibit invasion of breast cancer cells, and cell transformation by Src needs Abl (NON-PATENT DOCUMENT 10). It has also been shown that Src/Abl inhibitory substances inhibited an increase in the cancer cell volume in tumor-bearing model mice (NON-PATENT DOCUMENT 11). Thus, Abl is assumed to be an important target for development of drugs for cancer involving activation of Src such as breast cancer.

PATENT DOCUMENT 1: WO 01/83485
PATENT DOCUMENT 2: WO 03/57695
PATENT DOCUMENT 3: WO 00/75113
PATENT DOCUMENT 4: WO 01/09134
PATENT DOCUMENT 5: JP-A-2004-203748
PATENT DOCUMENT 6: WO 06/047256
PATENT DOCUMENT 7: WO 06/093247
PATENT DOCUMENT 8: WO 03/063794
NON-PATENT DOCUMENT 1: Oncogene 1996 Dec 19; 13 (12), p. 2595-2605.
NON-PATENT DOCUMENT 2: J. Pharmacol. Exp. Ther. 2003 Sep; 306 (3), p. 1174-1181.
NON-PATENT DOCUMENT 3: J. Immunol. 2002 Jul 15; 169 (2), p. 1028-1036.
NON-PATENT DOCUMENT 4: Proc. Natl. Acad. Sci. USA. 2004 Apr 20; 101 (16), p. 6158-6163.
NON-PATENT DOCUMENT 5: J. Pharmacol. Exp. Ther. 2006 May; 317 (2), p. 571-578.
NON-PATENT DOCUMENT 6: J. Pharmacol. Exp. Ther. 2006 Aug 31; [Epub ahead of print]
NON-PATENT DOCUMENT 7: Bioorganic & Medicinal Chemistry Letters (2003), 13 (8), p. 1415
NON-PATENT DOCUMENT 8: Bioorganic & Medicinal Chemistry 13 (2005), p. 4936
NON-PATENT DOCUMENT 9: Oncogene 2007 Nov 15; 26 (52), p. 7313-7323.
NON-PATENT DOCUMENT 10: Cancer Res. 2006 Jun 1; 66 (11), p. 5648-5655.
NON-PATENT DOCUMENT 11: Cancer Res. 2007 Feb 15; 67 (4), p. 1580-1588.

WO 05/085249 and WO 05/028480 disclose substituted pyrazolopyrimidine compounds, but do not describe or suggest Syk and Abl inhibitory effects of these compounds. WO 07/070872 describes a group of compounds having Syk inhibitory activity; however, the document describes only one substituted pyrazolopyrimidine compound among them and does not describe that the compound has Syk inhibitory activity.

DISCLOSURE OF THE INVENTION

PROBLEMS TO BE SOLVED BY THE INVENTION

An object of the present invention is to provide a compound having Syk and/or Abl inhibitory effects and useful as a pharmaceutical agent.

MEANS FOR SOLVING THE PROBLEMS

As a result of extensive studies to find a compound having Syk and/or Abl inhibitory effects, the present inventors have found that the object can be achieved by a pyrazolopyrimidine compound represented by the following formula [I] or a pharmaceutically acceptable salt thereof. This finding has led to the completion of the present invention.
The present invention will be described below.

(1) A pyrazolopyrimidine compound represented by the formula [I]:

[Formula 1]

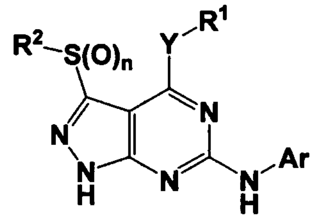

[I]

wherein

$R^1$ represents a $C_{1-8}$ alkyl group, a $C_{3-8}$ cycloalkyl group, a $C_{2-9}$ heterocyclyl group, a $C_{1-9}$ heteroaryl group or a phenyl group (wherein the $C_{1-8}$ alkyl group, the $C_{3-8}$ cycloalkyl group, the $C_{2-9}$ heterocyclyl group, the $C_{1-9}$ heteroaryl group and the phenyl group are unsubstituted or substituted with 1 to 4 substituents selected from the following Substituent Group $A^1$); Substituent Group $A^1$ represents
a group consisting of a halogen atom, a hydroxy group, an amino group, a cyano group, a carboxy group, a $C_{1-6}$ alkyl group, a trifluoromethyl group, a $C_{3-8}$ cycloalkyl group, a $C_{1-6}$ alkoxy group (wherein the $C_{1-6}$ alkoxy group is unsubstituted or substituted with a hydroxy group), a sulfanyl group, a $C_{1-6}$ alkylsulfanyl group, a $C_{1-6}$ alkoxycarbonyl group, a $C_{3-8}$ cycloalkoxy group, a ($C_{1-6}$ alkyl)amino group, a di($C_{1-6}$ alkyl)amino group, a $C_{1-6}$ alkoxycarbonylamino group, a hydroxyaminocarbonyl group, a ureido group, a carbamoyl group, a $C_{2-9}$ heterocyclyl group (wherein the $C_{2-9}$ heterocyclyl group is unsubstituted or substituted with a $C_{1-6}$ alkyl group or an oxo group), a $C_{1-9}$ heteroaryl group, a ($C_{1-6}$ acyl)amino group and an oxo group;
$R^2$ represents a $C_{1-6}$ alkyl group;
n represents an integer of 0, 1 or 2;
Y represents -O- or $-NR^3-$ (wherein $R^3$ represents a hydrogen atom or a $C_{1-6}$ alkyl group);
Ar represents a $C_{1-9}$ heteroaryl group (wherein the $C_{1-9}$ heteroaryl group is unsubstituted or substituted with 1 to 3 substituents selected from the group consisting of a sulfanyl group, a hydroxy group, a $C_{1-6}$ alkoxy group, a morpholinyl group, a trifluoromethyl group and an oxo group) or a phenyl group (wherein the phenyl group is unsubstituted or substituted with the same or different 1 to 3 $R^a$s); and
$R^a$ represents a hydroxy group, a cyano group, a carboxy group, a sulfanyl group, a $C_{1-6}$ alkoxycarbonyl group, a $C_{1-6}$ alkyl group (wherein the $C_{1-6}$ alkyl group is unsubstituted or substituted with a carboxy group or - $CONR^4R^5$ (wherein $R^4$ and $R^5$ are the same or different and each represent a hydrogen atom or a $C_{1-6}$ alkyl group)), a $C_{3-8}$ cycloalkyl group, a $C_{1-6}$ alkoxy group (wherein the $C_{1-6}$ alkoxy group is unsubstituted or substituted with a carboxy group or $-CONR^6R^7$ (wherein $R^6$ and $R^7$ are the same or different and each represent a hydrogen atom, a $C_{1-6}$ alkyl group, a $C_{3-8}$ cycloalkyl group, a $C_{1-6}$ alkoxy group, a $C_{2-9}$ heterocyclyl group or a $C_{1-9}$ heteroaryl group)), a $C_{1-9}$ heteroaryl group, a $C_{3-8}$ cycloalkoxy group, a halogen atom, a trifluoromethyl group, a trifluoromethoxy group, a phenoxy group, a phenyl group (wherein the phenyl group is unsubstituted or substituted with 1 to 3 substituents selected from the group consisting of a carboxy group, a $C_{1-6}$ alkoxycarbonyl group and $-SO_2NR^{14}R^{15}$ (wherein $R^{14}$ and $R^{15}$ are the same or different and each represent a hydrogen atom or a $C_{1-6}$ alkyl group)), $-NR^8R^9$, $-CONR^8R^9$, $-NR^8SO_2R^9$ or $-SO_2NR^8R^9$ (wherein $R^8$ and $R^9$ are the same or different and each represent a hydrogen atom, a $C_{1-6}$ acyl group, a $C_{1-6}$ alkoxycarbonyl group, a toluyl group, a naphthyl group, a $C_{1-9}$ heteroaryl group or a $C_{1-6}$ alkyl group (wherein the $C_{1-6}$ alkyl group is unsubstituted or substituted with 1 to 3 substituents selected from the group consisting of a hydroxy group, a carboxy group and a $C_{1-6}$ alkoxy group),
or when $R^8$ and $R^9$ are substituents on the adjacent nitrogen atom, they, together with the adjacent nitrogen atom, represent the formula [II]:

[Formula 2]

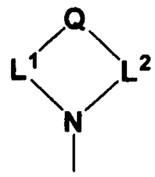

[II]

wherein Q represents -O-, $-NR^{10}-$, $-CHR^{11}$, -CO- (wherein the -CO- is unprotected or protected with ethylene ketal), -S-, -SO- or $-SO_2-$;

$L^1$ and $L^2$ are the same or different and each represent a linear $C_{1-3}$ alkylene group (wherein the linear $C_{1-3}$ alkylene group is unsubstituted or substituted with 1 to 3 substituents selected from the group consisting of a $C_{1-6}$ alkyl group and an oxo group);
$R^{10}$ represents a hydrogen atom, a $C_{1-6}$ acyl group, a $C_{1-6}$ alkyl group, a $C_{1-6}$ alkylsulfonyl group, a trifluoromethylsulfonyl group, a phenylcarbonyl group or a phenylsulfonyl group (wherein the phenylcarbonyl group and the phenylsulfonyl group are unsubstituted or substituted with 1 to 2 substituents selected from Substituent Group B);
Substituent Group B represents a group consisting of a halogen atom, a $C_{1-6}$ alkyl group and a $C_{1-6}$ alkoxy group; and
$R^{11}$ represents a hydrogen atom, a hydroxy group, a $C_{1-6}$ alkyl group (wherein the $C_{1-6}$ alkyl group is unsubstituted or substituted with a $C_{1-6}$ alkoxy group or a hydroxy group), a $C_{1-6}$ alkoxy group, a carboxy group, an amino group, a ($C_{1-6}$ alkyl) amino group, a di($C_{1-6}$ alkyl)amino group, a ($C_{1-6}$ acyl)amino group, a phenylcarbonyl group, $-CONR^{12}R^{13}$ (wherein $R^{12}$ and $R^{13}$ are the same or different and each represent a hydrogen atom or a $C_{1-6}$ alkyl group, or they, together with the adjacent nitrogen atom, represent a $C_{2-9}$ heterocyclyl group) or a ($C_{1-6}$ alkylsulfonyl) amino group); a tautomer or a stereoisomer of the compound, or a pharmaceutically acceptable salt thereof, or a solvate thereof.

(2) The pyrazolopyrimidine compound, a tautomer or a stereoisomer of the compound, or a pharmaceutically acceptable salt thereof, or a solvate thereof, according to (1) above, wherein $R^1$ is a $C_{1-8}$ alkyl group, a $C_{3-8}$ cycloalkyl group or a $C_{2-9}$ heterocyclyl group (wherein the $C_{1-8}$ alkyl group, the $C_{3-8}$ cycloalkyl group and the $C_{2-9}$ heterocyclyl group are unsubstituted or substituted with 1 to 4 substituents selected from the following Substituent Group A); Substituent Group A represents
a group consisting of a halogen atom, a hydroxy group, an amino group, a cyano group, a carboxy group, a $C_{1-6}$ alkyl group, a $C_{3-8}$ cycloalkyl group, a $C_{1-6}$ alkoxy group (wherein the $C_{1-6}$ alkoxy group is unsubstituted or substituted with a hydroxy group), a $C_{1-6}$ alkylsulfanyl group, a $C_{1-6}$ alkoxycarbonyl group, a $C_{3-8}$ cycloalkoxy group, a ($C_{1-6}$ alkyl)amino group, a di($C_{1-6}$ alkyl)amino group, a $C_{1-6}$ alkoxycarbonylamino group, a hydroxyaminocarbonyl group, a ureido group, a carbamoyl group, a $C_{2-9}$ heterocyclyl group (wherein the $C_{2-9}$ heterocyclyl group is unsubstituted or substituted with a $C_{1-6}$ alkyl group or an oxo group) and a $C_{1-9}$ heteroaryl group;
$R^2$ is a linear $C_{1-6}$ alkyl group;
Ar is a $C_{1-9}$ heteroaryl group (wherein the $C_{1-9}$ heteroaryl group is unsubstituted or substituted with 1 to 3 substituents selected from the group consisting of a sulfanyl group, a hydroxy group, a $C_{1-6}$ alkoxy group, a morpholinyl group and a trifluoromethyl group) or a phenyl group (wherein the phenyl group is unsubstituted or substituted with the same or different 1 to 3 $R^a$s); and
$R^a$ is a hydroxy group, a cyano group, a carboxy group, a sulfanyl group, a $C_{1-6}$ alkoxycarbonyl group, a $C_{1-6}$ alkyl group (wherein the $C_{1-6}$ alkyl group is unsubstituted or substituted with a carboxy group or $-CONR^4R^5$ (wherein $R^4$ and $R^5$ are the same or different and each represent a hydrogen atom or a $C_{1-6}$ alkyl group)), a $C_{3-8}$ cycloalkyl group, a $C_{1-6}$ alkoxy group (wherein the $C_{1-6}$ alkoxy group is unsubstituted or substituted with a carboxy group or $-CONR^6R^7$ (wherein $R^6$ and $R^7$ are the same or different and each represent a hydrogen atom, a $C_{1-6}$ alkyl group, a $C_{3-8}$ cycloalkyl group, a $C_{1-6}$ alkoxy group, a $C_{2-9}$ heterocyclyl group or a $C_{1-9}$ heteroaryl group)), a $C_{1-9}$ heteroaryl group, a $C_{3-8}$ cycloalkoxy group, a halogen atom, a trifluoromethyl group, a trifluoromethoxy group, a phenoxy group, $-NR^8R^9$, $-CONR^8R^9$, $-NR^8SO_2R^9$ or - $SO_2NR^8R^9$ (wherein $R^8$ and $R^9$ are the same or different and each represent a hydrogen atom, a $C_{1-6}$ acyl group, a $C_{1-6}$ alkoxycarbonyl group, a toluyl group, a $C_{1-9}$ heteroaryl group or a $C_{1-6}$ alkyl group (wherein the $C_{1-6}$ alkyl group is unsubstituted or substituted with 1 to 3 substituents selected from the group consisting of a hydroxy group, a carboxy group and a $C_{1-6}$ alkoxy group), or when $R^8$ and $R^9$ are substituents on the adjacent nitrogen atom, they,

together with the adjacent nitrogen atom, represent the formula [II]:
[Formula 3]

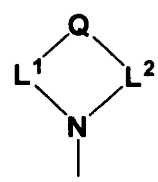

[II]

wherein Q represents -O-, $-NR^{10}-$, $-CHR^{11}-$, -CO- (wherein the -CO- is unprotected or protected with ethylene ketal), -S-, -SO- or $-SO_2-$;
$L^1$ and $L^2$ are the same or different and each represent a linear $C_{1-3}$ alkylene group (wherein the linear $C_{1-3}$ alkylene group is unsubstituted or substituted with 1 to 3 substituents selected from the group consisting of a $C_{1-6}$ alkyl group and an oxo group);
$R^{10}$ represents a hydrogen atom, a $C_{1-6}$ acyl group, a $C_{1-6}$ alkyl group, a $C_{1-6}$ alkylsulfonyl group, a trifluoromethylsulfonyl group, a phenylcarbonyl group or a phenylsulfonyl group (wherein the phenylcarbonyl group and the phenylsulfonyl group are unsubstituted or substituted with 1 to 2 substituents selected from Substituent Group B);
Substituent Group B represents a group consisting of a halogen atom, a $C_{1-6}$ alkyl group and a $C_{1-6}$ alkoxy group; and
$R^{11}$ represents a hydrogen atom, a hydroxy group, a $C_{1-6}$ alkyl group (wherein the $C_{1-6}$ alkyl group is unsubstituted or substituted with a $C_{1-6}$ alkoxy group or a hydroxy group), a $C_{1-6}$ alkoxy group, a carboxy group, an amino group, a ($C_{1-6}$ alkyl) amino group, a di($C_{1-6}$ alkyl)amino group, a ($C_{1-6}$ acyl)amino group, a phenylcarbonyl group, $-CONR^{12}R^{13}$ (wherein $R^{12}$ and $R^{13}$ are the same or different and each represent a hydrogen atom or a $C_{1-6}$ alkyl group, or they, together with the adjacent nitrogen atom, represent a $C_{2-9}$ heterocyclyl group) or a ($C_{1-6}$ alkylsulfonyl) amino group).

(3) The pyrazolopyrimidine compound, a tautomer or a stereoisomer of the compound, or a pharmaceutically acceptable salt thereof, or a solvate thereof according to (2) above, wherein Ar is a phenyl group (wherein the phenyl group is unsubstituted or substituted with the same or different 1 to 3 $R^a$s).
(4) The pyrazolopyrimidine compound, a tautomer or a stereoisomer of the compound, or a pharmaceutically acceptable salt thereof, or a solvate thereof according to (2) above, wherein Ar is a phenyl group (wherein the phenyl group is substituted at the 3-, 4- or 5-position with the same or different 1 to 3 $R^a$s).
(5) The pyrazolopyrimidine compound, a tautomer or a stereoisomer of the compound, or a pharmaceutically acceptable salt thereof, or a solvate thereof according to any one of (2) to (4) above, wherein Y is NH.
(6) The pyrazolopyrimidine compound, a tautomer or a stereoisomer of the compound, or a pharmaceutically acceptable salt thereof, or a solvate thereof according to any one of (2) to (5) above, wherein $R^1$ is a $C_{1-8}$ alkyl group (wherein the $C_{1-8}$ alkyl group is unsubstituted or substituted with an amino group, a ($C_{1-6}$ alkyl)amino group or a di($C_{1-6}$ alkyl)amino group).
(7) The pyrazolopyrimidine compound, a tautomer or a stereoisomer of the compound, or a pharmaceutically acceptable salt thereof, or a solvate thereof according to any one of (2) to (5) above, wherein $R^1$ is a $C_{3-8}$ cycloalkyl group (wherein the $C_{3-8}$ cycloalkyl group is unsubstituted or substituted with an amino group).
(8) The pyrazolopyrimidine compound, a tautomer or a stereoisomer of the compound, or a pharmaceutically acceptable salt thereof, or a solvate thereof according to (1) above, wherein $R^1$ is a $C_{1-9}$ heteroaryl group or a phenyl group (wherein the $C_{1-9}$ heteroaryl group and the phenyl group are unsubstituted or substituted with 1 to 3 substituents selected from the group consisting of a halogen atom, a hydroxy group, a cyano group, a $C_{1-6}$ alkyl group, a trifluoromethyl group, a $C_{1-6}$ alkoxy group, a sulfanyl group, a carbamoyl group, a ($C_{1-6}$ acyl)amino group and an oxo group); and Y is NH.
(9) A pyrazolopyrimidine compound, a tautomer or a stereoisomer of the compound, or a pharmaceutically acceptable salt thereof, or a solvate thereof, represented by the formula [III]:
[Formula 4]

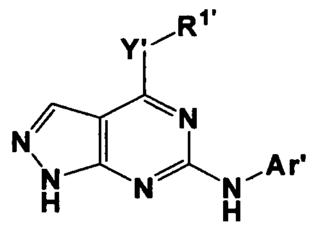

[III]

wherein

$R^{1'}$ represents a $C_{1-8}$ alkyl group or a $C_{3-8}$ cycloalkyl group (wherein the $C_{1-8}$ alkyl group and the $C_{3-8}$ cycloalkyl group are unsubstituted or substituted with 1 to 4 substituents selected from the following Substituent Group A'),
Substituent Group A' represents
a group consisting of a halogen atom, a hydroxy group, an amino group, a cyano group, a carboxy group, a $C_{1-6}$ alkyl group, a trifluoromethyl group, a $C_{3-8}$ cycloalkyl group, a $C_{1-6}$ alkoxy group (wherein the $C_{1-6}$ alkoxy group is unsubstituted or substituted with a hydroxy group), a sulfanyl group, a $C_{1-6}$ alkylsulfanyl group, a $C_{1-6}$ alkoxycarbonyl group, a $C_{3-8}$ cycloalkoxy group, a ($C_{1-6}$ alkyl)amino group, a di($C_{1-6}$ alkyl)amino group, a $C_{1-6}$ alkoxycarbonylamino group, a hydroxyaminocarbonyl group, a ureido group, a carbamoyl group, a $C_{2-9}$ heterocyclyl group (wherein the $C_{2-9}$ heterocyclyl group is unsubstituted or substituted with a $C_{1-6}$ alkyl group or an oxo group), a $C_{1-9}$ heteroaryl group, a ($C_{1-6}$ acyl)amino group and an oxo group;
Y' represents -O- or $-NR^{16}-$ (wherein $R^{16}$ represents a hydrogen atom or a $C_{1-6}$ alkyl group);
Ar' represents a $C_{1-9}$ heteroaryl group (wherein the $C_{1-9}$ heteroaryl group is unsubstituted or substituted with 1 to 3 substituents selected from the group consisting of a sulfanyl group, a hydroxy group, a $C_{1-6}$ alkoxy group, a morpholinyl group, a trifluoromethyl group and an oxo group) or a phenyl group (wherein the phenyl group is unsubstituted or substituted with the same or different 1 to 3 $R^b$s); and
$R^b$ represents a hydroxy group, a cyano group, a carboxy group, a sulfanyl group, a $C_{1-6}$ alkoxycarbonyl group, a $C_{1-6}$ alkyl group (wherein the $C_{1-6}$ alkyl group is unsubstituted or substituted with a carboxy group or - $CONR^{17}R^{18}$ (wherein $R^{17}$ and $R^{18}$ are the same or different and each represent a hydrogen atom or a $C_{1-6}$ alkyl group)), a $C_{3-8}$ cycloalkyl group, a $C_{1-6}$ alkoxy group (wherein the $C_{1-6}$ alkoxy group is unsubstituted or substituted with a carboxy group or $-CONR^{19}R^{20}$ (wherein $R^{19}$ and $R^{20}$ are the same or different and each represent a hydrogen atom, a $C_{1-6}$ alkyl group, a $C_{3-8}$ cycloalkyl group, a $C_{1-6}$ alkoxy group, a $C_{2-9}$ heterocyclyl group or a $C_{1-9}$ heteroaryl group)), a $C_{1-9}$ heteroaryl group, a $C_{3-8}$ cycloalkoxy group, a halogen atom, a trifluoromethyl group, a trifluoromethoxy group, a phenoxy group, a phenyl group (wherein the phenyl group is unsubstituted or substituted with a substituent selected from a carboxy group, a $C_{1-6}$ alkoxycarbonyl group and $-SO_2NR^{21}R^{22}$ (wherein $R^{21}$ and $R^{22}$ are the same or different and each represent a hydrogen atom or a $C_{1-6}$ alkyl group)), - $NR^{23}R^{24}$, $-CONR^{23}R^{24}$, $-NR^{23}SO_2R^{24}$ or $-SO_2NR^{23}R^{24}$ (wherein $R^{23}$ and $R^{24}$ are the same or different and each represent a hydrogen atom, a $C_{1-6}$ acyl group, a $C_{1-6}$ alkoxycarbonyl group, a toluyl group, a naphthyl group, a $C_{1-9}$ heteroaryl group or a $C_{1-6}$ alkyl group (wherein the $C_{1-6}$ alkyl group is unsubstituted or substituted with 1 to 3 substituents selected from the group consisting of a hydroxy group, a carboxy group and a $C_{1-6}$ alkoxy group), or when $R^{23}$ and $R^{24}$ are substituents on the adjacent nitrogen atom, they, together with the adjacent nitrogen atom, represent the formula [IV]:

[Formula 5]

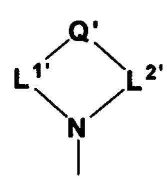

[IV]

wherein Q' represents -O-, $-NR^{25}-$, $-CHR^{26}-$, -CO- (wherein the -CO- is unprotected or protected with ethylene ketal), -S-, -SO- or $-SO_2-$;
$L^{1'}$ and $L^{2'}$ are the same or different and each represent a linear $C_{1-3}$ alkylene group (wherein the linear $C_{1-3}$ alkylene group is unsubstituted or substituted with 1 to 3 substituents selected from the group consisting of a $C_{1-6}$ alkyl group and an oxo group);
$R^{25}$ represents a hydrogen atom, a $C_{1-6}$ acyl group, a $C_{1-6}$ alkyl group, a $C_{1-6}$ alkylsulfonyl group, a trifluoromethylsulfonyl group, a phenylcarbonyl group or a phenylsulfonyl group (wherein the phenylcarbonyl group and the phenylsulfonyl group are unsubstituted or substituted with 1 to 2 substituents selected from Substituent Group B');
Substituent Group B' represents a group consisting of a halogen atom, a $C_{1-6}$ alkyl group and a $C_{1-6}$ alkoxy group; and
$R^{26}$ represents a hydrogen atom, a hydroxy group, a $C_{1-6}$ alkyl group (wherein the $C_{1-6}$ alkyl group is unsubstituted or substituted with a $C_{1-6}$ alkoxy group or a hydroxy group), a $C_{1-6}$ alkoxy group, a carboxy group, an amino group, a ($C_{1-6}$ alkyl)amino group, a di($C_{1-6}$ alkyl)amino group, a ($C_{1-6}$ acyl)amino group, a phenylcarbonyl group, $-CONR^{27}R^{28}$ (wherein $R^{27}$ and $R^{28}$ are the same or different and each represent a hydrogen atom or a $C_{1-6}$ alkyl group, or they, together with the adjacent nitrogen atom, represent a $C_{2-9}$ heterocyclyl group) or a ($C_{1-6}$ alkylsulfonyl) amino group).

(10) The pyrazolopyrimidine compound, a tautomer or a stereoisomer of the compound, or a pharmaceutically acceptable salt thereof, or a solvate thereof according to (9) above, wherein $R^{1'}$ is a $C_{1-8}$ alkyl group (wherein the $C_{1-8}$ alkyl group is unsubstituted or substituted with an amino group, a cyano group, a ($C_{1-6}$ alkyl) amino group or a di($C_{1-6}$ alkyl)amino group).

(11) The pyrazolopyrimidine compound, a tautomer or a stereoisomer of the compound, or a pharmaceutically acceptable salt thereof, or a solvate thereof according to (9) above, wherein $R^{1'}$ is a $C_{3-8}$ cycloalkyl group (wherein the $C_{3-8}$ cycloalkyl group is unsubstituted or substituted with a hydroxy group or an amino group).

(12) A Syk inhibitor comprising, as an active ingredient, the pyrazolopyrimidine compound, a tautomer or a stereoisomer of the compound, or a pharmaceutically acceptable salt thereof, or a solvate thereof according to any one of (1) to (11) above.

(13) A therapeutic agent or prophylactic agent for allergic disease, autoimmune disease or arthritis, comprising, as an active ingredient, the pyrazolopyrimidine compound, a tautomer or a stereoisomer of the compound, or a pharmaceutically acceptable salt thereof, or a solvate thereof according to any one of (1) to (11) above.

(14) An Abl inhibitor comprising, as an active ingredient, the pyrazolopyrimidine compound, a tautomer or a stereoisomer of the compound, or a pharmaceutically acceptable salt thereof, or a solvate thereof according to any one of (1) to (11) above.

(15) A therapeutic agent or prophylactic agent for cancer, comprising, as an active ingredient, the pyrazolopyrimidine compound, a tautomer or a stereoisomer of the compound, or a pharmaceutically acceptable salt thereof, or a solvate thereof according to any one of (1) to (11) above.

ADVANTAGEOUS EFFECTS OF INVENTION

The compound of the present invention has Syk and/or Abl inhibitory effects and is assumed to be effective for treating or preventing diseases such as allergic disease, autoimmune disease and arthritis, or cancer.

BEST MODE FOR CARRYING OUT THE INVENTION

The present invention will be described in more detail below.
First, the phrases used herein will be described.

In the present invention, "n" refers to normal, "i" refers to iso, "s" refers to secondary, "t" refers to tertiary, "c" refers to cyclo, "o" refers to ortho, "m" refers to meta, and "p" refers to para.

**[0041]** The "$C_{1-8}$ alkyl group" refers to a linear alkyl group having 1 to 8 carbon atoms or a branched alkyl group having 3 to 8 carbon atoms. Examples of the group include a methyl group, an ethyl group, an n-propyl group, an n-butyl group, an n-pentyl group, an n-hexyl group, an n-heptyl group, an n-octyl group, an i-propyl group, an i-butyl group, a t-butyl group, an s-butyl group, an i-pentyl group, a neopentyl group and a t-pentyl group.

The "$C_{3-8}$ cycloalkyl group" refers to a cycloalkyl group having 3 to 8 carbon atoms. Examples of the group include a c-propyl group, a c-butyl group, a c-pentyl group, a c-hexyl group, a c-heptyl group and a c-octyl group.

The "$C_{1-6}$ alkoxy group" refers to a linear alkoxy group having 1 to 6 carbon atoms or a branched alkoxy group having 3 to 6 carbon atoms. Examples of the group include a methoxy group, an ethoxy group, an n-propoxy group, an i-propoxy group, an n-butoxy group, an i-butoxy group, an s-butoxy group, a t-butoxy group, an n-pentyloxy group, an i-pentyloxy group and an n-hexyloxy group.

The "$C_{1-6}$ alkylsulfanyl group" refers to an alkylsulfanyl group having 1 to 6 carbon atoms and may include a

linear alkylsulfanyl group, a branched alkylsulfanyl group and a $C_{3-6}$ cycloalkylsulfanyl group. Examples of the group include a methylsulfanyl group, an ethylsulfanyl group, an n-propylsulfanyl group, an i-propylsulfanyl group, an n-butylsulfanyl group, an i-butylsulfanyl group, a t-butylsulfanyl group, an n-pentylsulfanyl group, an i-pentylsulfanyl group, a c-pentylsulfanyl group, an n-hexylsulfanyl group and a c-hexylsulfanyl group.

The "$C_{1-6}$ alkoxycarbonyl group" refers to a $C_{1-6}$ alkoxycarbonyl group having 1 to 6 carbon atoms and may include a linear alkoxycarbonyl group, a branched alkoxycarbonyl group and a $C_{3-6}$ cycloalkoxycarbonyl group. Examples of the group include a methoxycarbonyl group, an ethoxycarbonyl group, an i-propoxycarbonyl group and a c-pentyloxycarbonyl group.

The "$C_{3-8}$ cycloalkoxy group" refers to a cyclic alkoxy group having 3 to 8 carbon atoms. Examples of the group include a c-propoxy group, a c-butoxy group, a c-pentyloxy group, a c-hexyloxy group, a c-heptyloxy group and a c-octyloxy group.

The "halogen atom" is a fluorine atom, a chlorine atom, a bromine atom or an iodine atom.

The "($C_{1-6}$ alkyl)amino group" refers to an amino group having one $C_{1-6}$ alkyl group as a substituent and may include a linear alkylamino group, a branched alkylamino group and a ($C_{3-6}$ cycloalkyl)amino group. Examples of the group include a methylamino group, an ethylamino group, an n-propylamino group, an i-propylamino group, a c-propylamino group, an n-butylamino group, an i-butylamino group, an s-butylamino group, a t-butylamino group and a c-butylamino group.

The "di($C_{1-6}$ alkyl)amino group" refers to an amino group having the same or different two $C_{1-6}$ alkyl groups as substituents and may include a linear dialkylamino group, a branched dialkylamino group and a di($C_{3-6}$ cycloalkyl) amino group. Examples of the group include a dimethylamino group, a diethylamino group, a di-n-propylamino group, a di-i-propylamino group, a di-i-butylamino group, a di-s-butylamino group, a di-t-butylamino group, a (methyl, ethyl) amino group and a (methyl, n-propyl)amino group.

The "$C_{1-6}$ alkoxycarbonylamino group" refers to an amino group having a $C_{1-6}$ alkoxycarbonyl group and may include a linear alkoxycarbonylamino group, a branched alkoxycarbonylamino group and a $C_{3-6}$ cycloalkoxycarbonylamino group. Examples of the group include a methoxycarbonylamino group, an ethoxycarbonylamino group, an n-propoxycarbonylamino group, an i-propoxycarbonylamino group and a c-propoxycarbonylamino group.

The "$C_{2-9}$ heterocyclyl group" refers to a saturated monocyclic or fused bicyclic aliphatic heterocyclic group composed of one or more atoms selected from the group consisting of an oxygen atom, a sulfur atom and a nitrogen atom and 2 to 9 carbon atoms. Examples of the group include a tetrahydrofuranyl group, a pyrrolidinyl group, an imidazolinyl group, a pyrazolinyl group, a piperidyl group, a piperazinyl group and a morpholinyl group.

The "$C_{1-9}$ heteroaryl group" refers to a monocyclic aromatic heterocyclic group, a polycyclic aromatic heterocyclic group or a polycyclic heterocyclic group containing an aromatic ring in the structure, which is composed of one or more atoms selected from the group consisting of an oxygen atom, a sulfur atom and a nitrogen atom and 1 to 9 carbon atoms. Examples of the monocyclic aromatic heterocyclic group include an imidazolyl group, a pyrazolyl group, a thiazolyl group, an oxazolyl group, a furyl group, a thienyl group, a pyrrolyl group, a pyridyl group, a pyrimidinyl group and a pyrazinyl group. Examples of the polycyclic aromatic heterocyclic group include an indolyl group, a quinolyl group, a benzoimidazolyl group, a benzothiazolyl group, an indazolyl group and a benzotriazolyl group. Examples of the polycyclic heterocyclic group containing an aromatic ring in the structure include a benzoxazinyl group and a pyridooxazinyl group.

The "linear $C_{1-6}$ alkyl group" refers to a linear alkyl group having 1 to 6 carbon atoms. Examples of the group include a methyl group, an ethyl group, an n-propyl group, an n-butyl group, an n-pentyl group and an n-hexyl group.

The "$C_{1-6}$ alkyl group" refers to a linear alkyl group having 1 to 6 carbon atoms or a branched alkyl group having 3 to 6 carbon atoms. Examples of the group include a methyl group, an ethyl group, an n-propyl group, an i-propyl group, an n-butyl group, an i-butyl group, an n-pentyl group and an n-hexyl group.

The "$C_{1-6}$ acyl group" refers to an acyl group having 1 to 6 carbon atoms and may include a linear acyl group, a branched acyl group and a cyclic acyl group. Examples of the group include a formyl group, an acetyl group, a propionyl group, an n-butyryl group, an i-butyryl group, a c-butyryl group, an n-valeryl group, an i-valeryl group and a pivaloyl group.

Examples of the "$C_{1-3}$ alkylene group" include a methylene group, an ethylene group and a trimethylene group.

The "$C_{1-6}$ alkylsulfonyl group" refers to an alkylsulfonyl group having 1 to 6 carbon atoms and may include a linear alkylsulfonyl group, a branched alkylsulfonyl group and a $C_{3-6}$ cycloalkylsulfonyl group. Examples of the group include a methylsulfonyl group, an ethylsulfonyl group, a propyl-1-sulfonyl group, a 2-methylpropyl-1-sulfonyl group, a butyl-1-sulfonyl group, a hexyl-1-sulfonyl group and a 3-methylbutyl-1-sulfonyl group.

The "($C_{1-6}$ acyl)amino group" refers to an amino group having a $C_{1-6}$ acyl group as a substituent. Examples of the group include a formylamino group, an acetylamino group, a propionylamino group, an i-butyrylamino group, a c-butyrylamino group, an n-valerylamino group, an i-valerylamino group and a pivaloylamino group.

The "($C_{1-6}$ alkylsulfonyl)amino group" refers to an amino group having a $C_{1-6}$ alkylsulfonyl group as a substituent. Examples of the group include a methylsulfonylamino group, an ethylsulfonylamino group, a propyl-1-sulfonylamino group, a 2-methylpropyl-1-sulfonylamino group, a butyl-1-sulfonylamino group, a hexyl-1-sulfonylamino group and a 3-methylbutyl-1-sulfonylamino group.

Preferred embodiments of the compound of the present invention are as follows.

Specifically, preferable $R^1$ is a $C_{1-8}$ alkyl group, a $C_{3-8}$ cycloalkyl group, a $C_{1-9}$ heteroaryl group or a phenyl group (wherein the $C_{1-8}$ alkyl group, the $C_{3-8}$ cycloalkyl group, the $C_{1-9}$ heteroaryl group and the phenyl group are unsubstituted or substituted with 1 to 3 substituents selected from Substituent Group $A^1$).

A preferable substituent among Substituent Group $A^1$ is a halogen atom, a hydroxy group, an amino group, a hydroxyaminocarbonyl group, a ($C_{1-6}$ alkyl)amino group, a di($C_{1-6}$ alkyl)amino group or a carbamoyl group.

Preferable $R^2$ is a methyl group, an ethyl group or an isopropyl group.

Preferable n is 0, 1 or 2.

Preferable Y is $-NR^3-$.

Preferable $R^3$ is a hydrogen atom.

Preferable Ar is a $C_{1-9}$ heteroaryl group (wherein the $C_{1-9}$ heteroaryl group is unsubstituted or substituted with a sulfanyl group or a hydroxy group) or a phenyl group (wherein the phenyl group is substituted with the same or different 1 to 3 $R^a$s).

More preferable Ar is a phenyl group (wherein the phenyl group is substituted at the 3-, 4- or 5-position with the same or different 1 to 3 $R^a$s).

Preferable $R^a$ is a $C_{1-6}$ alkyl group, a $C_{1-6}$ alkoxy group (wherein the $C_{1-6}$ alkoxy group is unsubstituted or substituted with a carboxy group or - $CONR^6R^7$ (wherein $R^6$ and $R^7$ are the same or different and each represent a hydrogen atom or a $C_{1-6}$ alkyl group)), a halogen atom, $-NR^8R^9$, $-CONR^8R^9$ or $-SO_2NR^8R^9$ (wherein $R^8$ and $R^9$ are the same or different and each represent a hydrogen atom, an acyl group, a $C_{1-9}$ heteroaryl group or a $C_{1-6}$ alkyl group (wherein the $C_{1-6}$ alkyl group is unsubstituted or substituted with a hydroxy group), or they, together with the adjacent nitrogen atom, represent the formula [II]:

[Formula 6]

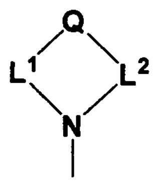

[II]

wherein Q represents -O-, $-NR^{10}-$, $-CHR^{11}-$, -CO- (wherein the -CO- is unprotected or protected with ethylene ketal), -S-, -SO- or $-SO_2-$,

$L^1$ and $L^2$ are the same or different and are each a linear $C_{1-3}$ alkylene group (wherein the linear $C_{1-3}$ alkylene group is unsubstituted or substituted with a $C_{1-6}$ alkyl group or an oxo group),

$R^{10}$ is a hydrogen atom, a $C_{1-6}$ acyl group, a $C_{1-6}$ alkyl group, a $C_{1-6}$ alkylsulfonyl group or a phenylcarbonyl group, and $R^{11}$ represents a hydroxy group, a $C_{1-6}$ alkyl group (wherein the $C_{1-6}$ alkyl group is substituted with a $C_{1-6}$ alkoxy group or a hydroxy group), a $C_{1-6}$ alkoxy group, a carboxy group or $-CONR^{12}R^{13}$ (wherein $R^{12}$ and $R^{13}$ are the same or different and each represent a hydrogen atom or a $C_{1-6}$ alkyl group, or they, together with the adjacent nitrogen atom, represent a $C_{2-9}$ heterocyclyl group)).

Preferable $R^{1'}$ is a $C_{1-8}$ alkyl group or a $C_{3-8}$ cycloalkyl group (wherein the $C_{1-8}$ alkyl group and the $C_{3-8}$ cycloalkyl group are unsubstituted or substituted with 1 to 3 substituents selected from Substituent Group A').

A preferable substituent among Substituent Group A' is a hydroxy group, an amino group, a cyano group, a ($C_{1-6}$ alkyl) amino group or a di($C_{1-6}$ alkyl)amino group.

Preferable Y' is $-NR^{16}-$.

Preferable $R^{16}$ is a hydrogen atom.

Preferable Ar' is a $C_{1-9}$ heteroaryl group (wherein the $C_{1-9}$ heteroaryl group is unsubstituted or substituted with a sulfanyl group or a hydroxy group) or a phenyl group (wherein the phenyl group is substituted with the same or different 1 to 3 $R^b$s).

More preferable Ar is a phenyl group (wherein the phenyl group is substituted at the 3-, 4- or 5-position with the same or different 1 to 3 $R^b$s).

Preferable $R^b$ is a $C_{1-6}$ alkyl group, a $C_{1-6}$ alkoxy group (wherein the $C_{1-6}$ alkoxy group is unsubstituted or substituted

with a carboxy group or - $CONR^{17}R^{18}$ (wherein $R^6$ and $R^7$ are the same or different and each represent a hydrogen atom or a $C_{1-6}$ alkyl group)), a halogen atom, $-NR^{23}R^{24}$, $-CONR^{23}R^{24}$ or $-SO_2NR^{23}R^{24}$ (wherein $R^{23}$ and $R^{24}$ are the same or different and each represent a hydrogen atom, an acyl group, a $C_{1-9}$ heteroaryl group or a $C_{1-6}$ alkyl group (wherein the $C_{1-6}$ alkyl group is unsubstituted or substituted with a hydroxy group), or $R^{23}$ and $R^{24}$, together with the adjacent nitrogen atom, represent the formula [IV]:

[Formula 7]

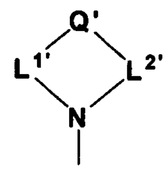

[IV]

wherein Q' represents -O-, $-NR^{25}-$, $-CHR^{26}-$, -CO- (wherein the -CO- is unprotected or protected with ethylene ketal), -S-, -SO- or $-SO_2-$,
$L^{1'}$ and $L^{2'}$ are the same or different and are each a linear $C_{1-3}$ alkylene group (wherein the linear $C_{1-3}$ alkylene group is unsubstituted or substituted with a $C_{1-6}$ alkyl group or an oxo group),
$R^{25}$ is a hydrogen atom, a $C_{1-6}$ acyl group, a $C_{1-6}$ alkyl group, a $C_{1-6}$ alkylsulfonyl group or a phenylcarbonyl group, and
$R^{26}$ represents a hydroxy group, a $C_{1-6}$ alkyl group (wherein the $C_{1-6}$ alkyl group is substituted with a $C_{1-6}$ alkoxy group or a hydroxy group), a $C_{1-6}$ alkoxy group, a carboxy group or $-CONR^{27}R^{28}$ (wherein $R^{27}$ and $R^{28}$ are the same or different and each represent a hydrogen atom or a $C_{1-6}$ alkyl group, or they, together with the adjacent nitrogen atom, represent a $C_{2-9}$ heterocyclyl group)).

Tautomers, stereoisomers such as geometric isomers, optical isomers, and prodrugs of the compound of the present invention may exist, and the present invention also encompasses them.

The present invention also encompasses various hydrates, solvates and crystalline polymorphs of the inventive compound and a salt thereof.

The prodrug is a derivative of the compound of the present invention having a chemically or metabolically decomposable group and is a compound converted to a pharmacologically active compound forming the present invention by solvolysis or in vivo under physiological conditions. Methods for selecting and preparing a suitable prodrug derivative are described in DESIGN OF PRODRUGS (Elsevier, Amsterdam 1985), for example. Examples of the prodrug of the compound of the present invention having a hydroxy group include an acyloxy derivative prepared by reacting the compound with a suitable acyl halide or a suitable acid anhydride. Examples of the acyloxy particularly preferable as a prodrug include $-OCOC_2H_5$, - OCO(t-Bu), $-OCOC_{15}H_{31}$, $-OCO(m-CO_2Na-Ph)$, $-OCOCH_2CH_2CO_2Na$, $-OCOCH(NH_2)CH_3$ and $-OCOCH_2N(CH_3)_2$. Examples of the prodrug of the compound forming the present invention which has an amino group include an amide derivative prepared by reacting the compound having an amino group with a suitable acid halide or a suitable mixed acid anhydride. Examples of the amide particularly preferable as a prodrug include $-NHCOCH(NH_2)CH_3$. Examples of the prodrug of the compound forming the present invention which has a carboxyl group include a carboxylic acid ester synthesized by reaction with an aliphatic alcohol; and a carboxylic acid ester obtained by reaction with a free alcoholic hydroxyl group of 1,2- or 1,3-diglyceride. Examples of the carboxylic acid ester particularly preferable as a prodrug include a methyl ester and an ethyl ester.

The pharmaceutically acceptable salt is a salt with an alkali metal, an alkali earth metal, ammonium, an alkylammonium or the like, or a salt with an inorganic acid or organic acid. Examples of the salt include sodium salts, potassium salts, calcium salts, ammonium salts, aluminum salts, triethylammonium salts, acetates, propionates, butyrates, formates, trifluoroacetates, maleates, tartrates, citrates, stearates, succinates, ethylsuccinates, lactobionates, gluconates, glucoheptonates, benzoates, methanesulfonates, ethanesulfonates, 2-hydroxyethanesulfonates, benzenesulfonates, p-toluenesulfonates, lauryl sulfates, malates, aspartates, glutamates, adipates, salts with cysteine, salts with N-acetylcysteine, hydrochlorides, hydrobromides, phosphates, sulfates, hydroiodides, nicotinates, oxalates, picrates, thiocyanates, undecanoates, salts with acrylic acid polymers, and salts with carboxyvinyl polymers.

The compound of the present invention can be synthesized by the method shown below, for example.

(1) A compound represented by the following formula (a):

[Formula 8]

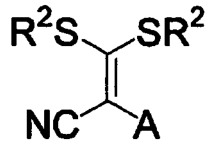

(a)

wherein $R^2$ is a $C_{1-6}$ alkyl group and A represents a cyano group, a carboxy group, a $C_{1-6}$ alkoxycarbonyl group or a carbamoyl group, is reacted with a compound represented by the formula $R'NHNH_2$, wherein R' represents a hydrogen atom or a $C_{1-8}$ alkyl group (wherein the $C_{1-8}$ alkyl group is unsubstituted or substituted with an Ar group), in a solvent or without a solvent to obtain a compound represented by the following formula (b):

[Formula 9]

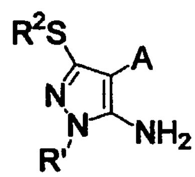

(b)

wherein A, $R^2$ and R' are as defined above.

(2) The compound represented by the formula (b), wherein A, $R^2$ and R' are as defined above, is appropriately subjected to hydrolysis of A and is then reacted with urea in a solvent or without a solvent to obtain a compound represented by the following formula (c):

[Formula 10]

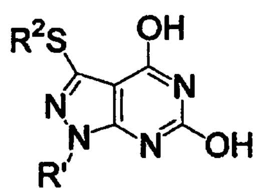

(c)

wherein $R^2$ and R' are as defined above.

(3) The compound represented by the formula (c) is halogenated in a solvent or without a solvent in the presence or absence of a base.

[Formula 11]

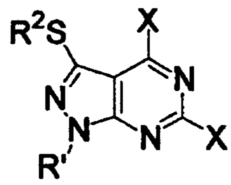

(d)

Thus, a compound represented by the above formula (d) is obtained, wherein $R^2$ and R' are as defined above and Xs are the same or different and each represent a halogen atom.

(4) The compound represented by the formula (d) is reacted with $R^1$-YH in a solvent or without a solvent in the presence or absence of a base, an acid or chlorotrimethylsilane, or an additive.

[Formula 12]

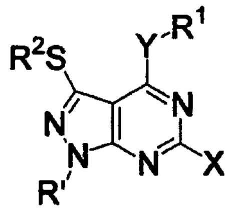

(e)

Thus, a compound represented by the above formula (e) is obtained, wherein $R^1$, $R^2$, R', X and Y are as defined above.

(5) The compound represented by the formula (e) is oxidized with an oxidizing agent in a solvent or without a solvent to obtain a compound represented by the following formula (f):

[Formula 13]

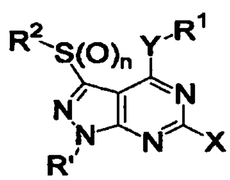

(f)

wherein n represents an integer of 1 or 2 and $R^1$, $R^2$, R', X and Y are as defined above.

(6) The compound represented by the formula (e) or (f) is aminated with Ar-$NH_2$ in a solvent or without a solvent in the presence or absence of a base, an acid or chlorotrimethylsilane, or an additive to obtain the compound of the present invention which is represented by the following formula (g):

[Formula 14]

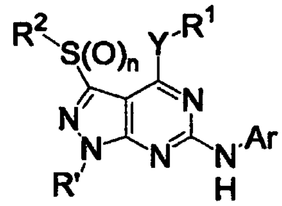

(g)

wherein n represents an integer of 0, 1 or 2 and $R^1$, $R^2$, R', X, Y and Ar are as defined above.
The compound of the present invention can also be synthesized by the method shown below, for example.

(7) Pyrimidine-2,4,6(1H,3H,5H)-trione is halogenated in DMF in the presence or absence of a base to obtain a compound represented by the following formula (h):

[Formula 15]

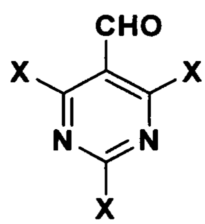

(h)

wherein X is as defined above.
(8) The compound represented by the formula (h) is reacted with a compound represented by the formula $R'NHNH_2$, wherein R' represents a hydrogen atom or a $C_{1-8}$ alkyl group (wherein the $C_{1-8}$ alkyl group is unsubstituted or substituted with an Ar group), in a solvent or without a solvent, or the compound is reacted with hydrazine, and then a protecting group is introduced as necessary, to obtain a compound represented by the following formula (i):
[Formula 16]

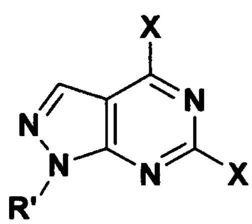

(i)

wherein X and R' are as defined above.
(9) The compound represented by the formula (i) is reacted with $R^{1'}$-Y'H in a solvent or without a solvent in the presence or absence of a base, an acid or chlorotrimethylsilane, or an additive to obtain a compound represented by the formula (j):
[Formula 17]

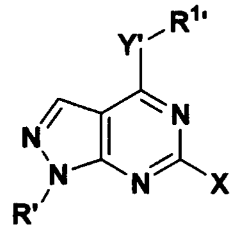

wherein $R^{1'}$, R', Y' and X are as defined above.

(10) The compound represented by the formula (j) is aminated with Ar'-$NH_2$ in a solvent or without a solvent in the presence or absence of a base, an acid or chlorotrimethylsilane, or an additive to obtain the compound of the present invention which is represented by the following formula (k):

[Formula 18]

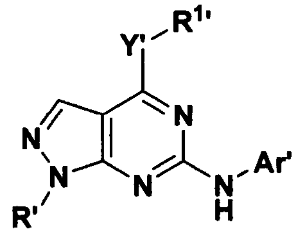

wherein $R^{1'}$, R', Y' and Ar' are as defined above.

The protecting group on $R^1$, Ar, $R^{1'}$ or Ar', and R' can be appropriately deprotected in each step. The order of reaction steps such as substituent introduction can be changed as necessary when it is inappropriate to carry out the steps in the synthesis method shown above.

Examples of the base, when used in the above reaction, include alkali metal salts such as sodium carbonate, potassium carbonate, cesium carbonate, sodium bicarbonate, potassium bicarbonate, sodium hydroxide, sodium hydride, sodium amide, t-butylpotassium, lithium hydroxide, lithium hydride, t-butoxypotassium and t-butoxysodium; amines such as triethylamine, diisopropylamine, dimethylaniline, diethylaniline, pyrrolidine and piperidine; sodium acetate and potassium acetate.

Examples of the acid used include inorganic acids such as hydrochloric acid, hydrobromic acid, sulfuric acid, nitric acid, phosphoric acid and polyphosphoric acid; and organic acids such as p-toluenesulfonic acid, methanesulfonic acid, trifluoroacetic acid, formic acid and acetic acid.

Examples of the additive include $Pd_2(dba)_3$, $Pd(PPh_3)_4$, BINAP, 4,5-bis(diphenylphosphino)-9,9-dimethylxanthene, CuI, CuCl and copper powder.

Examples of the oxidizing agent used include organic peracids such as m-chloroperbenzoic acid, magnesium monoperphthalate hexahydrate, peracetic acid and performic acid; inorganic and organic peroxides such as hydrogen peroxide, urea-hydrogen peroxide adduct/phthalic anhydride, t-butyl hydroperoxide and cumene hydroperoxide; sodium periodate, Oxone(R), N-bromosuccinimide, N-chlorosuccinimide, chloramine-T, t-butyl hypochlorite, iodobenzene diacetate and bromine-1,4-diazabicyclo[2,2,2]octane addition complex.

Examples of the protecting group include alkoxymethyl groups such as methoxymethyl and 2-methoxyethoxymethyl; aryloxymethyl groups such as benzyloxymethyl and p-methoxybenzyloxymethyl; acyl groups such as formyl, acetyl and trifluoroacetyl; arylcarbonyl groups such as benzoyl, benzoylformyl and benzoylpropionyl; alkoxycarbonyl groups such as methoxycarbonyl, ethoxycarbonyl, n-propoxycarbonyl, i-propoxycarbonyl, n-butoxycarbonyl, i-butoxycarbonyl and t-butoxycarbonyl; alkylaminocarbonyl groups such as methylcarbamoyl, ethylcarbamoyl and n-propylcarbamoyl; trialkylsilyl groups such as trimethylsilyl, triethylsilyl, triisopropylsilyl, diethylisopropylsilyl, dimethylisopropylsilyl, di-t-butylmethylsilyl and t-butyldimethylsilyl; trialkylarylsilyl groups such as diphenylmethylsilyl, t-butyldiphenylsilyl and t-butyldimethoxyphenylsilyl; alkylsulfonyl groups such as methanesulfonyl and ethanesulfonyl; arylsulfonyl groups such as benzenesulfonyl, p-toluenesulfonyl, p-chlorobenzenesulfonyl and p-methoxybenzenesulfonyl; acetal

groups such as methyleneacetal and isopropylideneketal; a t-butyl group, an allyl group, a benzyl group, a tetrahydropyranyl group and a tetrahydrofuranyl group.

The reaction solvent is not particularly limited insofar as it is stable under the reaction conditions and is inert and does not inhibit the reaction. Examples of the solvent include alcohols such as methanol, ethanol, i-propanol, n-butanol, t-butanol and ethylene glycol; ethers such as diethyl ether, 1,4-dioxane, tetrahydrofuran and 1,2-dimethoxyethane; hydrocarbons such as toluene, benzene and xylene; esters such as ethyl acetate and ethyl formate; ketones such as acetone, methyl ethyl ketone and methyl i-butyl ketone; halogenated carbon solvents such as chloroform and dichloromethane; amides such as N,N-dimethylformamide, N,N-dimethylacetamide and N-methylpyrrolidone; acetonitrile, dimethyl sulfoxide, water, and mixed solvents thereof.

The reaction can be carried out under normal pressure, under pressure or under microwave irradiation, for example, at a suitable temperature selected within a range from -78˚C to the boiling point of the solvent used for the reaction.

When the compound of the present invention is used as a medicine, a common excipient, bulking agent, pH adjuster or solubilizer is added to the compound of the present invention, and the mixture is formulated into tablets, granules, pills, capsules, a powder, a solution, a suspension or an injection by a common technique, for example. Thus, the compound of the present invention can be administered as an oral formulation, or an injection or an application.

The compound of the present invention can be administered to an adult patient at 1 to 2000 mg per day in one to several doses. The dose can be appropriately increased and reduced according to the type of the disease the age, body weight and symptom of the patient, for example.

EXAMPLES

Next, the present invention will be described in more detail by way of Examples and Test Example. The compounds of the present invention are not limited to the compounds described in the following Examples.

The instruments used for measurement of the respective analysis data are as follows.
[$^1$H-NMR]
600 MHz: JEOL JNM-ECA600
500 MHz: JEOL JNM-ECP500
300 MHz: JEOL JNM-ECX300
[MS]
Waters, Shimadzu
The abbreviations in the Examples are shown below.
DEAD: Diethyl azodicarboxylate
THF: Tetrahydrofuran
BINAP: 2,2'-Bis(diphenylphosphino)-1,1-binaphthyl
dba: Bis(dibenzylideneacetone)
TFA: Trifluoroacetic acid
Boc: t-Butoxycarbonyl
mCPBA: m-Chloroperbenzoic acid
DMSO: Dimethyl sulfoxide
ESI: Electrospray ionization

Reference Example 1

[Formula 19]

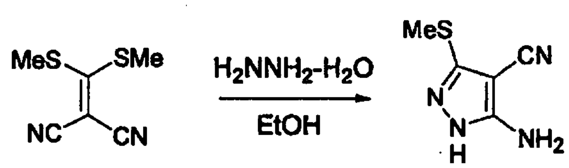


[Bis(methylsulfanyl)methylene]malononitrile (10 g) was added to a solution of hydrazine monohydrate (4.5 g) in ethanol (10 ml) under ice-cooling. After stirred at room temperature for 30 minutes and refluxed at 80˚C for one hour,

water was added dropwise to the reaction solution under ice-cooling. The precipitated solid was separated by filtration and dried to obtain 5-amino-3-(methylsulfanyl)-1H-pyrazole-4-carbonitrile (colorless solid) (7.6 g, 85%).
MS(ESI):155$(M+H)^+$,153$(M-H)^-$
$^1H$ NMR(600MHz,DMSO-D6)δppm 2.44(s,3H),6.47(bs,2H), 12.00(bs,1H)

Reference Example 2

[Formula 20]

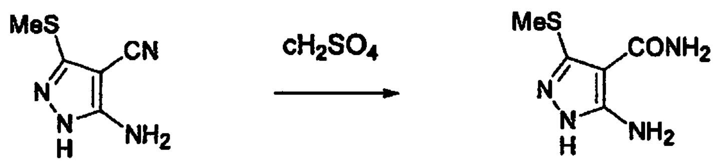


5-Amino-3-(methylsulfanyl)-1H-pyrazole-4-carbonitrile (7.6 g) was added to concentrated sulfuric acid (25 ml) under ice-cooling, and the mixture was stirred at room temperature for five hours. The reaction solution was added dropwise to ice water (50 ml), and subsequently aqueous ammonia was added to precipitate crystals (pH 8 to 9). The precipitated solid was separated by filtration and dried to obtain 5-amino-3-(methylsulfanyl)-1H-pyrazole-4-carboxamide (colorless solid) (7.6 g, 90%).
MS(ESI):173$(M+H)^+$,171$(M-H)^-$
$^1H$ NMR(600MHz,DMSO-D6)δppm 2.38(s,3H),6.00(bs,2H), 6.71(br,2H),11.84(br,1H)

Example 1

$N^4$-Cyclopropyl-3-(methylsulfonyl)-$N^6$-(4-morpholin-4-ylphenyl)-1H-pyrazolo[3,4-d]pyrimidine-4,6-diamine (Compound 6)

[Formula 21]

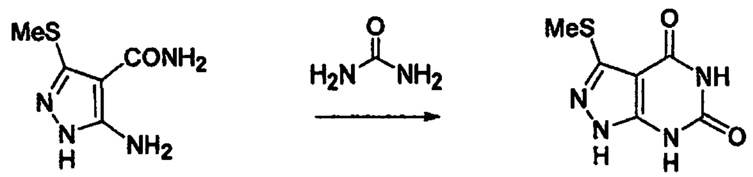

(1) 5-Amino-3-(methylsulfanyl)-1H-pyrazole-4-carboxamide obtained in Reference Example 2 (3.0 g) and urea (5.3 g) were stirred at 170°C for two hours. After cooling to room temperature, a 1 M sodium hydroxide solution was added and the solid was dissolved. Then, acetic acid was added to precipitate a solid. The precipitated solid was separated by filtration and dried to obtain 3-(methylsulfanyl)-1H-pyrazolo[3,4-d]pyrimidine-4,6(5H,7H)-dione (colorless solid) (3.4 g, quant.).
MS(ESI):197$(M-H)^-$

[Formula 22]

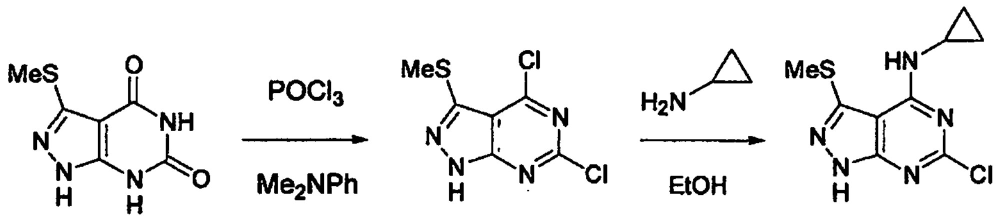


(2) N,N-dimethylaniline (2.0 ml) was added dropwise to a suspension of 3-(methylsulfanyl)-1H-pyrazolo[3,4-d]pyrimidine-4,6(5H,7H)-dione obtained in Example 1-(1) (2.0 g) and phosphorus oxychloride (10 ml) under ice-cooling, and then the mixture was stirred at 100°C for two hours. The reaction solution was dissolved in chloroform and the insoluble matter was separated by filtration. The objective product was extracted with chloroform. Cyclopropylamine (0.88 ml) was added to a solution of 4,6-dichloro-3-(methylsulfanyl)-1H-pyrazolo[3,4-d]pyrimidine obtained by concentrating the extract in ethanol (3.0 ml) under ice-cooling. The mixture was stirred at room temperature for 30 minutes and then 1 M hydrochloric acid was added, followed by extraction with ethyl acetate. The extract was washed with brine and then dried over anhydrous magnesium sulfate. The drying agent was separated by filtration, and then the solvent was concentrated under reduced pressure. The residue was washed with a diethyl ether-methanol mixed solution and separated by filtration to obtain 6-chloro-N-cyclopropyl-3-(methylsulfanyl)-1H-pyrazolo[3,4-d]pyrimidin-4-amine (pale yellow solid) (0.29 g, 11% (2 steps)).
MS(ESI):256$(M+H)^+$,254$(M-H)^-$
$^1H$ NMR(600MHz,DMSO-D6)δppm 0.62-0.70(m,2H),0.73-0.81(m,2H),2.52(s,3H),2.86-3.03(m,1H),7.01(s,1H), 13.63(s, 1H)
[Formula 23]

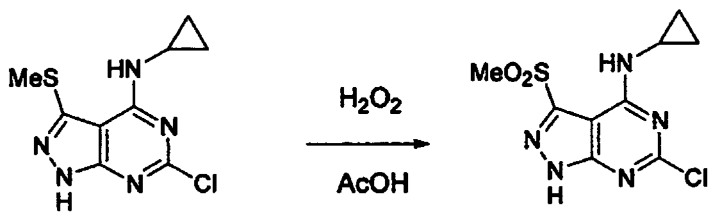


(3) A 35% hydrogen peroxide solution (3.0 ml) was added to a solution of 6-chloro-N-cyclopropyl-3-(methylsulfanyl)-1H-pyrazolo[3,4-d]pyrimidin-4-amine obtained in Example 1-(2) (0.29 g) in acetic acid (3.0 ml) at room temperature. The mixture was stirred at 80°C for 2.5 hours and then cooled to room temperature. Water was added and the precipitate was separated by filtration and dried to obtain 6-chloro-N-cyclopropyl-3-(methylsulfonyl)-1H-pyrazolo[3,4-d]pyrimidin-4-amine (colorless solid) (0.23 g, 72%).
MS(ESI):288$(M+H)^+$,286$(M-H)^-$
$^1H$ NMR(600MHz,DMSO-D6)δppm 0.51-0.62(m,2H),0.81-0.91(m,2H),2.96-3.05(m,1H),3.44(s,3H), 8.02(d,J=3.7Hz, 1H),14.81(bs,1H)
[Formula 24]

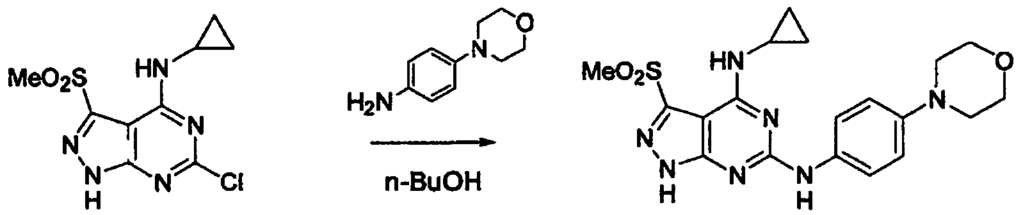


(4) A mixture of 6-chloro-N-cyclopropyl-3-(methylsulfonyl)-1H-pyrazolo[3,4-d]pyrimidin-4-amine obtained in Example 1-(3) (0.23 g), 4-morpholinoaniline (0.19 g) and n-butanol (3.0 ml) was reacted at 130˚C for two hours. The reaction solution was concentrated, and then the resulting crude product was purified by silica gel chromatography, followed by recrystallization from chloroform-methanol to obtain Compound 6 (colorless solid) (313 mg, 90%).
MS(ESI):430$(M+H)^+$,428$(M-H)^-$
$^1$H NMR(300MHz,DMSO-D6)δppm 0.62-0.67(m,2H),0.92-0.98(m,2H),3.03(bs,1H),3.44(s,3H),3.54-3.60(m,4H), 4.07-4.13(m,4H),7.78-7.80(m,2H),7.90(s,1H),8.03-8.05(m,2H),9.99(s,1H),14.28(bs,1H)

Example 2

$N^4$-Cyclopropyl-3-(methylsulfonyl)-$N^6$-(4-morpholin-4-ylphenyl)-1H-pyrazolo[3,4-d]pyrimidine-4,6-diamine phosphate (Compound 9)

(1) The compound 6 obtained in Example 1-(4) (29 g) was dissolved in a 10% phosphoric acid solution (570 ml). Water (570 ml) was added and the mixture was stirred at room temperature for 13 hours. The precipitate was separated by filtration and dried to obtain Compound 9 (yellow solid) (32 g, 91%).
$^1$H NMR(600MHz,DMSO-D6)δppm 0.51-0.56(m,2H),0.82-0.88(m,2H),2.94-2.98(m,1H),2.98-3.02(m,4H),3.26-3.33(m, 3H),3.37(s,3H),3.69-3.73(m,4H),6.82-6.90(m,2H), 7.53-7.58(m,1H),7.66-7.74(m,2H),9.14(s,1H),13.85(s,1H)

Example 3

$N^4$-Cyclopropyl-$N^6$-[4-(4-methylpiperazin-1-yl)phenyl]-3-(methylsulfonyl)-1H-pyrazolo[3,4-d]pyrimidine-4,6-diamine (Compound 131)

[Formula 25]

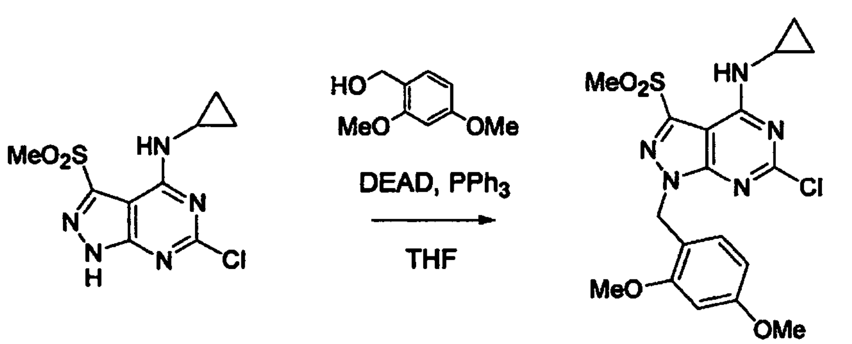


(1) Diethyl azodicarboxylate (40% solution in toluene, 19 g) was added dropwise to a mixture of 6-chloro-N-cyclopropyl-3-(methylsulfonyl)-1H-pyrazolo[3,4-d]pyrimidin-4-amine obtained in Example 1-(3) (8.4 g), 2,4-dimethoxybenzyl alcohol (7.4 g) and triphenylphosphine (12 g) in tetrahydrofuran (0.40 1) under ice-cooling, and the mixture was reacted at room temperature for 15 hours. The reaction solution was concentrated, and then the resulting crude product was purified by silica gel chromatography (hexane/chloroform = 1/4) to obtain 6-chloro-N-cyclopropyl-1-(2,4-dimethoxybenzyl)-3-(methylsulfonyl)-1H-pyrazolo[3,4-d]pyrimidin-4-amine (colorless solid) (3.2 g, 25%).

MS(ESI):438(M+H)+,436(M-H)-

[Formula 26]

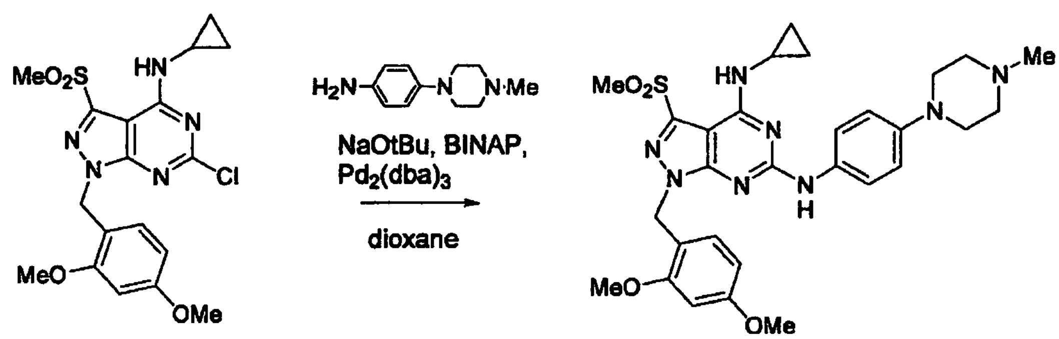


(2) A suspension of 6-chloro-N-cyclopropyl-1-(2,4-dimethoxybenzyl)-3-(methylsulfonyl)-1H-pyrazolo[3,4-d]pyrimidin-4-amine obtained in Example 3-(1) (0.60 g), sodium t-butoxide (0.21 g), BINAP (0.13 g), $Pd_2(dba)_3$ (63 mg) and 4-(N-methylpiperazinyl)aniline (0.31 g) in dioxane (20 ml) was stirred at 100˚C for 15 hours. The reaction solution was concentrated, and then the resulting crude product was purified by silica gel chromatography (NH silica gel, hexane/ethyl acetate = 7/3) to obtain $N^4$-cyclopropyl-$N^6$-[4-(4-methylpiperazin-1-yl)phenyl]-1-(2,4-dimethoxybenzyl)-3-(methylsulfonyl)-1H-pyrazolo[3,4-d]pyrimidine-4,6-diamine (colorless solid) (369 mg).

[Formula 27]

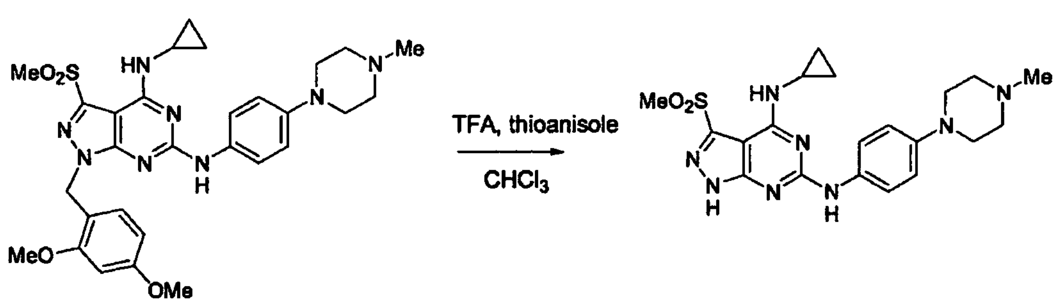


(3) Trifluoroacetic acid (1.0 ml) and thioanisole (2.0 ml) were added to a solution of $N^4$-cyclopropyl-$N^6$-[4-(4-methylpiperazin-1-yl)phenyl]-1-(2,4-dimethoxybenzyl)-3-(methylsulfonyl)-1H-pyrazolo[3,4-d]pyrimidine-4,6-diamine obtained in Example 3-(2) (0.37 g) in chloroform (4.0 ml) under ice-cooling, and the mixture was stirred at 60˚C for two days. The reaction solution was concentrated, and the resulting crude product was purified by silica gel chromatography (NH silica gel, chloroform/methanol = 99/1), followed by recrystallization from chloroform-methanol to obtain Compound 131 (colorless solid) (58 mg, 10%).

MS(ESI):443(M+H)+,441(M-H)-

$^1H$ NMR(600MHz,DMSO-D6)δppm 0.45-0.59(m,2H),0.85(m,2H), 2.18(s,3H),2.31-2.45(m,4H),2.93-3.00(m,1H), 2.99-3.11(m,4H),3.37(s,3H),6.78-6.92(m,2H),7.54(s,1H),7.60-7.81(m,2H),9.11(s,1H),13.85(bs,1H)

Example 4

$N^4$-(cis-4-Aminocyclohexyl)-3-(methylsulfonyl)-$N^6$-(4-morpholin-4-ylphenyl)-1H-pyrazolo[3,4-d]pyrimidine-4,6-diamine (Compound 117)

[Formula 28]

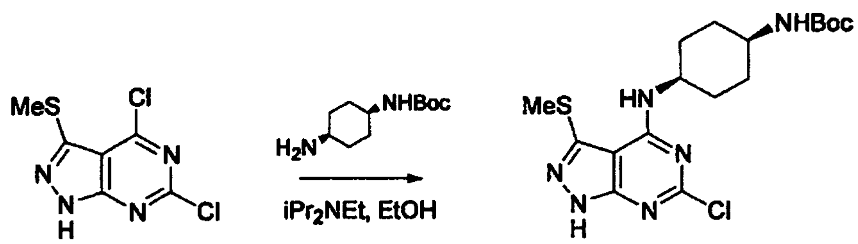


(1) t-Butyl (cis-4-aminocyclohexyl)carbamate (1.8 g) and diisopropylethylamine (1.5 ml) were added to a solution of 4,6-dichloro-3-(methylsulfanyl)-1H-pyrazolo[3,4-d]pyrimidine obtained in Example 1-(2) (1.0 g) in ethanol (43 ml), and the mixture was stirred at room temperature overnight. The reaction solution was concentrated, and the resulting crude product was purified by silica gel chromatography (hexane/ethyl acetate = 1/1) to obtain t-butyl (cis-4-{[6-chloro-3-(methylsulfanyl)-1H-pyrazolo[3,4-d]pyrimidin-4-yl]amino}cyclohexyl)carbamate (colorless powder) (1.6 g, 89%).
MS(ESI):413$(M+H)^+$,411$(M-H)^-$
$^1H$ NMR(600MHz,DMSO-D6)δppm 1.36(s,9H),1.41-1.54(m,2H), 1.55-1.71(m,4H),1.73-1.85(m,2H)2.56(s,3H), 3.40-3.58(m,1H),4.08-4.32(m,1H),6.47(d,J=7.8Hz,1H), 6.77(d,J=7.8Hz,1H),13.70(s,1H)
[Formula 29]

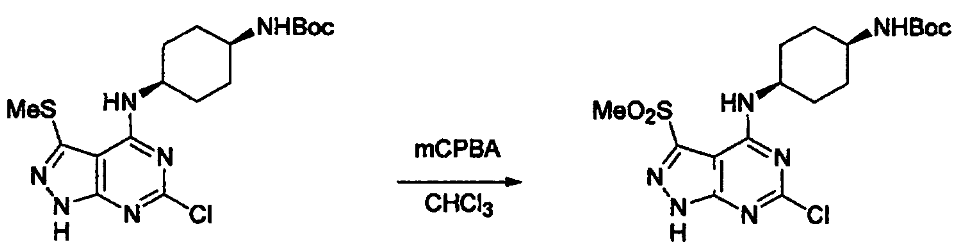


(2) m-Chloroperbenzoic acid (2.5 g) was added to a solution of t-butyl (cis-4-{[6-chloro-3-(methylsulfanyl)-1H-pyrazolo[3,4-d]pyrimidin-4-yl]amino}cyclohexyl)carbamate obtained in Example 4-(1) (1.6 g) in chloroform (37 ml) under ice-cooling, and the mixture was stirred at room temperature overnight. The reaction solution was washed with a 1 M sodium hydroxide solution and brine, dehydrated over anhydrous magnesium sulfate, separated by filtration and concentrated. The resulting crude product was purified by silica gel chromatography (hexane/ethyl acetate = 3/2) to obtain t-butyl (cis-4-{[6-chloro-3-(methylsulfonyl)-1H-pyrazolo[3,4-d]pyrimidin-4-yl]amino}cyclohexyl)carbamate (colorless powder) (0.78 g, 47%).
MS(ESI):445$(M+H)^+$,443$(M-H)^-$
$^1H$ NMR(600MHz,DMSO-D6)δppm 1.35(s,9H),1.40-1.51(m,2H),1.58-1.72(m,4H),1.71-1.81(m,2H),3.31-3.41(m,1H), 3.46(s,3H),4.10-4.19(m,1H), 6.93(d,J=7.3Hz,1H),8.13(d,J=7.3Hz,1H),14.76(s,1H)
[Formula 30]

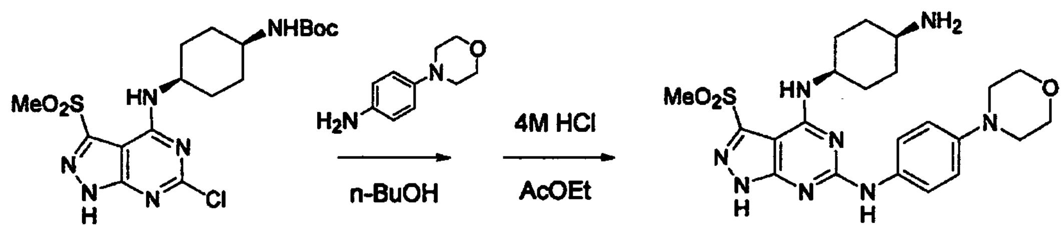


(3) 4-Morpholinoaniline (81 mg) was added to a suspension of t-butyl (cis-4-{[6-chloro-3-(methylsulfonyl)-1H-pyrazolo[3,4-d]pyrimidin-4-yl]amino}cyclohexyl)carbamate obtained in Example 4-(2) (0.16 g) in n-butanol (3.0 ml), and the mixture was stirred at 130˚C for four hours. The reaction solution was concentrated, and the resulting crude product was purified by silica gel chromatography (chloroform/methanol = 99/1). Thereafter, a 4 M hydrochloric acid/ethyl acetate solution (2.0 ml) was added and the mixture was stirred at 80˚C for 30 minutes. The reaction solution was concentrated, and the resulting crude product was purified by silica gel chromatography (chloroform/methanol = 99/1), followed by recrystallization to obtain Compound 117 (pale purple powder) (95 mg, 56%).
MS(ESI):487$(M+H)^+$,485$(M-H)^-$
$^1H$ NMR(600MHz,DMSO-D6)δppm 1.29-1.45(m,2H),1.54-1.72(m,4H),1.72-1.95(m,2H),2.75-2.85(m,1H),2.91-3.05(m,4H),3.36(s,3H),3.65-3.75(m,4H),4.15-4.30(m,1H),6.76-6.91(m,2H),7.55-7.68(m,3H),8.91(s,1H)

Example 5

$N^4$-Cyclopropyl-3-(methylsulfinyl)-$N^6$-(4-morpholin-4-ylphenyl)-1H-pyrazolo[3,4-d]pyrimidine-4,6-diamine (Compound 4)

[Formula 31]

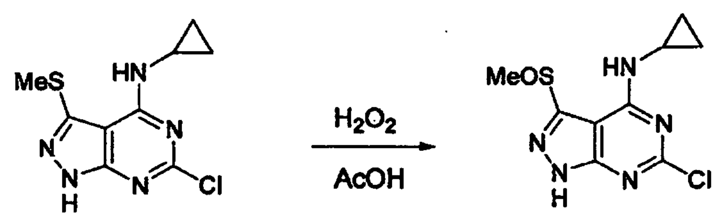


(1) A 35% hydrogen peroxide solution (1.0 ml) was added to a solution of 6-chloro-N-cyclopropyl-3-(methylsulfanyl)-1H-pyrazolo[3,4-d]pyrimidin-4-amine obtained in Example 1-(2) (0.10 g) in acetic acid (1.0 ml) at room temperature, and the mixture was stirred at room temperature for six hours. Then, water was added, and the precipitate was separated by filtration and dried to obtain 6-chloro-N-cyclopropyl-3-(methylsulfinyl)-1H-pyrazolo[3,4-d]pyrimidin-4-amine (pale yellow solid) (0.10 g, 94%).
MS(ESI):272$(M+H)^+$,270$(M-H)^-$
$^1H$ NMR(300MHz,DMSO-D6)δppm 0.50-0.65(m,2H),0.80-0.95(m,2H),2.96(s,3H),3.03(m,1H),8.92(d,J=4.1Hz,1H),14. 4 (bs,1H)
[Formula 32]

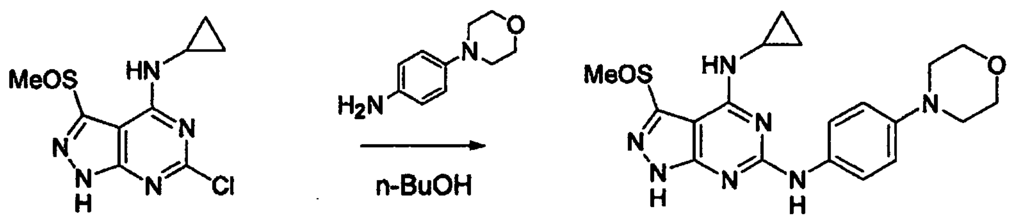


(2) A mixture of 6-chloro-N-cyclopropyl-3-(methylsulfinyl)-1H-pyrazolo[3,4-d]pyrimidin-4-amine obtained in Example 5-(1) (15 mg), 4-morpholinoaniline (10 mg) and n-butanol (0.5 ml) was reacted under microwave irradiation at 130˚C for two hours. The reaction solution was concentrated, and then the resulting crude product was washed with diethyl ether to obtain Compound 4 (gray solid) (9 mg, 40%).
MS(ESI):414$(M+H)^+$,412$(M-H)^-$
$^1H$ NMR(500MHz,DMSO-D6)δppm 0.49-0.58(m,2H),0.82-0.90(m,2H),2.90(s,3H),2.99(bs,1H),3.05-3.15(m,4H), 3.72-3.78(m,4H),6.89(d,J=8.0Hz,2H),7.74(d,J=8.6Hz,2H), 8.33(s,1H),9.11(s,1H),13.49(s,1H)

Example 6

$N^4$-Cyclopropyl-3-(methylsulfanyl)-$N^6$-(4-morpholin-4-ylphenyl)-1H-pyrazolo[3,4-d]pyrimidine-4,6-diamine (Compound 13)

[Formula 33]

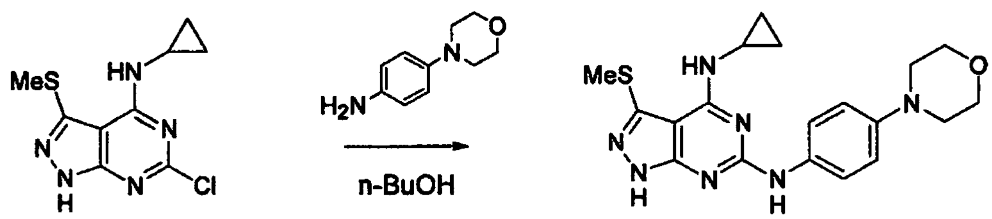


(1) A mixture of 6-chloro-N-cyclopropyl-3-(methylsulfanyl)-1H-pyrazolo[3,4-d]pyrimidin-4-amine obtained in Example 1-(2) (30 mg), 4-morpholinoaniline (27 mg) and n-butanol (1.0 ml) was reacted under microwave irradiation at 130˚C for two hours. The reaction solution was concentrated, and then the resulting crude product was washed with diethyl ether to obtain Compound 13 (colorless solid) (23 mg, 49%).
MS(ESI):398$(M+H)^+$,396$(M-H)^-$
$^1H$ NMR(300MHz,DMSO-D6)δppm 0.77-0.80(m,2H),0.92-0.95(m,2H),2.56(s,3H),2.96(bs,1H),3.05-3.25(m,4H), 3.75-3.85(m,4H),7.10-7.20(m,2H),7.27(bs,1H),7.60-7.80(m,2H), 9.88(bs,1H),12.89(bs,1H)

Example 7

4-Ethoxy-3-(methylsulfanyl)-N-(4-morpholin-4-ylphenyl)-1H-pyrazolo[3,4-d]pyrimidin-6-amine (Compound 20)

[Formula 34]

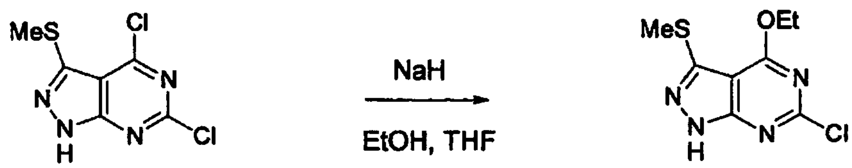


(1) 40% sodium hydride (0.34 g) and methyl aziridine-2-carboxylate (0.65 g) were added to a mixed solution of 4,6-dichloro-3-(methylsulfanyl)-1H-pyrazolo[3,4-d]pyrimidine obtained in Example 1-(2) (1.0 g) in THF (10 ml) and ethanol (10 ml), and the mixture was reacted at 70˚C for three hours. Water was added to the reaction solution, followed by extraction with ethyl acetate and washing with brine. The extract was dried over anhydrous magnesium sulfate. The drying agent was separated by filtration, and then the solvent was concentrated under reduced pressure. The residue was washed with a methanol-chloroform mixed solution and separated by filtration to obtain 6-chloro-4-ethoxy-3-(methylsulfanyl)-1H-pyrazolo[3,4-d]pyrimidine (green powder) (0.4 g, 38%).

[Formula 35]

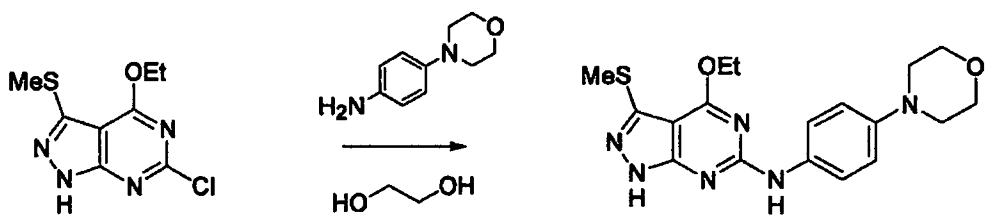

(2) A mixture of 6-chloro-4-ethoxy-3-(methylsulfanyl)-1H-pyrazolo[3,4-d]pyrimidine obtained in Example 7-(1) (50 mg), 4-morpholinoaniline (44 mg) and ethylene glycol (1.0 ml) was reacted under microwave irradiation at 130˚C for one hour. Water was added to the reaction solution, followed by extraction with ethyl acetate and washing with brine. The extract was dried over anhydrous magnesium sulfate. The drying agent was separated by filtration, and then the solvent was concentrated under reduced pressure. The residue was washed with a methanol-acetonitrile mixed solution and separated by filtration to obtain Compound 20 (colorless powder) (39 mg, 49%).
MS(ESI):387$(M+H)^+$,385$(M-H)^-$
$^1$H NMR(500MHz,DMSO-D6)δppm 1.39(t,J=7.0Hz,3H), 2.52(s,3H),3.04(t,J=4.9Hz,4H),3.74(t,J=4.9Hz,4H),4.51(q , J=7.0Hz,2H),6.89(d,J=9.2Hz,2H),7.63(d,J=8.9Hz,2H),9.29 (s,1H),12.99(s,1H)

Example 8

$N^4$-Cyclopropyl-$N^6$-(4-morpholin-4-ylphenyl)-1H-pyrazolo[3,4-d]pyrimidine-4,6-diamine (Compound 177)

[Formula 36]

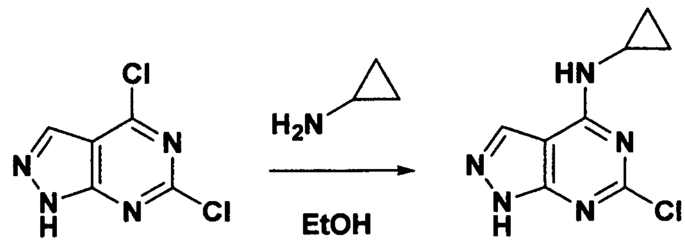


(1) Cyclopropylamine (0.12 g) was added to a solution of 4,6-dichloro-1H-pyrazolo[3,4-d]pyrimidine synthesized according to the method of JP-A-48-99194 (0.40 g) in ethanol (4.0 ml) at room temperature. The mixture was stirred at the same temperature overnight. The precipitated solid was separated by filtration and dried to obtain 6-chloro-N-cyclopropyl-1H-pyrazolo[3,4-d]pyrimidin-4-amine (yellow solid) (0.43 g, 97%).
MS(ESI):210$(M+H)^+$,208$(M-H)^-$
$^1$H NMR(300MHz,DMSO-D6)δppm 0.55-0.73(m,2H),0.74-1.06(m,2H),2.97(bs,1H),8.18(bs,1H),8.51(bs,1H), 13.37(bs, 1H)

[Formula 37]

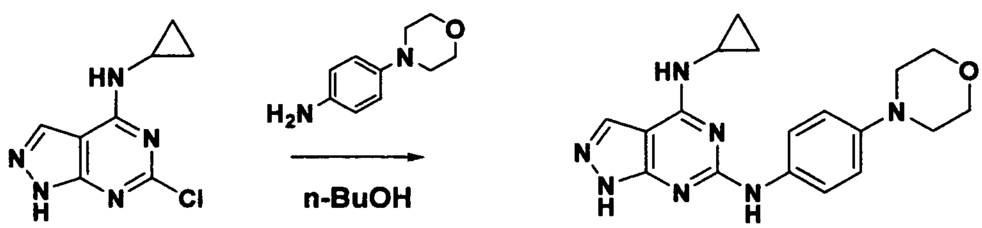


(2) A mixture of 6-chloro-N-cyclopropyl-1H-pyrazolo[3,4-d]pyrimidin-4-amine obtained in Example 8-(1) (50 mg), 4-morpholinoaniline (55 mg) and n-butanol (1.0 ml) was reacted under microwave irradiation at 130°C for two hours. The reaction solution was concentrated, and then the resulting crude product was crystallized from diethyl ether to obtain Compound 177 (colorless solid) (13 mg, 15%).
MS(ESI):352$(M+H)^+$,350$(M-H)^-$
$^1$H NMR(300MHz,DMSO-D6)δppm 0.58-0.64(m,2H),0.80-0.85(m,2H),2.95(bs,1H),3.02(t,J=2.7Hz, 4H), 3.73(t,J=2.7Hz, 4H),6.84(d,J=5.3Hz, 2H),7.55-7.75(m,3H),7.90(bs,1H),8.44(bs,1H),12.53(bs,1H)

Example 9

$N^4$-(cis-4-Aminocyclohexyl)-$N^4$-(4-morpholinophenyl)-1H-pyrazolo[3,4-d]pyrimidine-4,6-diamine (Compound 216)

[Formula 38]

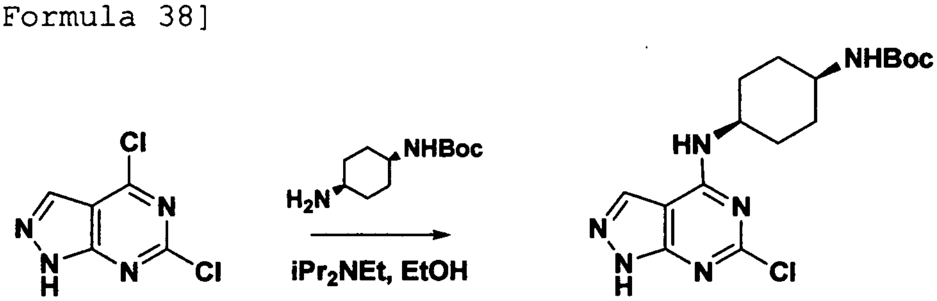

(1) t-Butyl (cis-4-aminocyclohexyl)carbamate (680 mg) and diisopropylethylamine (552 μL) were added to a solution of 4,6-dichloro-1H-pyrazolo[3,4-d]pyrimidine synthesized according to the method of JP-A-48-99194 (300 mg) in ethanol (16 mL), and the mixture was stirred at room temperature for 3.5 hours. The reaction solution was concentrated, and the resulting crude product was purified by silica gel chromatography (hexane/ethyl acetate = 7/3 to 3/7) to obtain t-butyl [cis-4-(6-chloro-1H-pyrazolo[3,4-d]pyrimidin-4-ylamino)cyclohexyl]carbamate (yellow powder) (354 mg, 61%).
MS(ESI):389(M+Na)$^+$,365(M-H)$^-$
$^1$H NMR(600MHz,DMSO-D6)δppm 1.36(s,9H),1.48-1.77(m,8H),3.35-3.45(m,1H),3.93-4.04(m,1H),6.74(bs,1H), 8.16 (bs,1H),8.29(d,J=6.9Hz,1H),13.46(bs,1H)
[Formula 39]

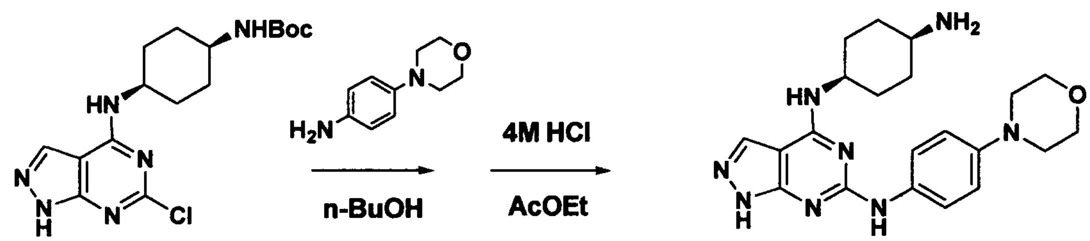


(2) 4-Morpholinoaniline (221 mg) was added to a solution of t-butyl [cis-4-(6-chloro-1H-pyrazolo[3,4-d]pyrimidin-4-ylamino)cyclohexyl]carbamate obtained in Example 9-(1) (350 mg) in n-butanol (5.0 mL), and the mixture was stirred at 130°C for four hours. The reaction solution was concentrated and dissolved in methanol/chloroform (10 mL, methanol/ chloroform = 1/9). A 4.0 M hydrochloric acid/ethyl acetate solution (4.0 mL) was added and the mixture was stirred at room temperature for one hour. Methanol (25 mL) was added to the reaction solution, and the precipitate was dissolved. Then, NH silica gel was added and the solvent was evaporated. The residue was purified by silica gel chromatography (NH silica gel, methanol/chloroform = 0 to 10%), followed by recrystallization (chloroform/hexane) to obtain Compound 216 (gray powder) (308 mg, 79%).
MS(ESI):409(M+H)$^+$,407(M-H)$^-$
$^1$H NMR(600MHz,DMSO-D6)δppm 1.47-1.81(m,8H),2.88-2.99(m,5H),3.64-3.70(m,4H),4.02-4.13(m,1H),6.74-6.82(m, 2H),7.47-7.55(m,1H),7.57-7.63(m,2H),7.91(s,1H), 8.62(bs,1H),12.57(bs,1H)

Example 10

4-Ethoxy-3-(methylsulfonyl)-N-(4-morpholin-4-ylphenyl)-1H-pyrazolo[3,4-d]pyrimidin-6-amine (Compound 179)

[Formula 40]

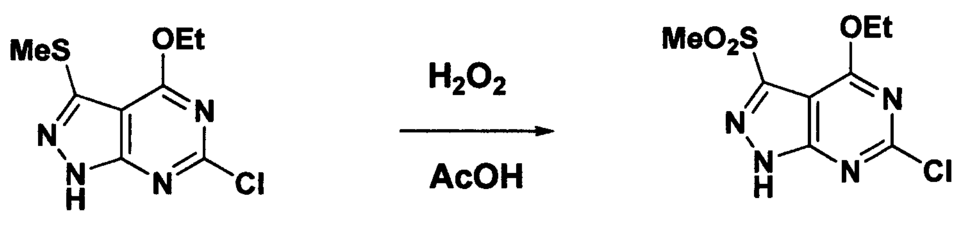


(1) A 35% hydrogen peroxide solution (2.0 ml) was added to a solution of 6-chloro-4-ethoxy-3-(methylsulfanyl)-1H-pyrazolo[3,4-d]pyrimidine obtained in Example 7-(1) (0.27 g) in acetic acid (2.0 ml) at room temperature, and the mixture was stirred at the same temperature for two days. Then, water was added, and the precipitate was separated by filtration and dried to obtain 6-chloro-4-ethoxy-3-(methylsulfonyl)-1H-pyrazolo[3,4-d]pyrimidine (colorless solid) (0.15 g, 49%).
MS(ESI):277(M+H)$^+$,275(M-H)$^-$

$^1$H NMR(300MHz,DMSO-D6)δppm 1.42(t,J=7.2Hz, 3H),2.50(s, 3H),4.62(q,J=7.2Hz,2H),15.05(s,1H)

[Formula 41]

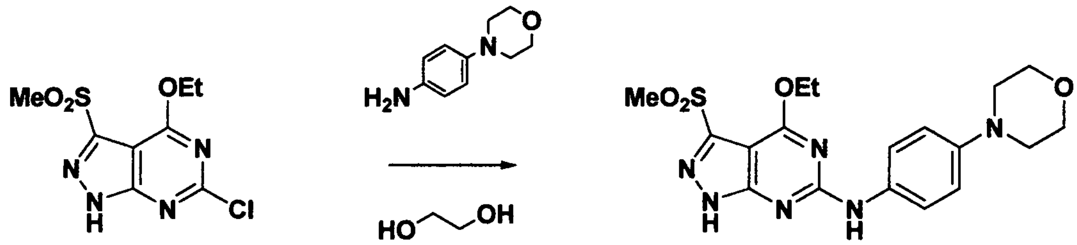

(2) A mixture of 6-chloro-4-ethoxy-3-(methylsulfonyl)-1H-pyrazolo[3,4-d]pyrimidine obtained in Example 10-(1) (50 mg), 4-morpholinoaniline (39 mg) and n-butanol (1.0 ml) was reacted under microwave irradiation at 130°C for 45 minutes. A 1 M hydrochloric acid solution was added to the reaction solution, followed by extraction with ethyl acetate. The extract was washed with brine and then dried over anhydrous magnesium sulfate. The drying agent was separated by filtration, and then the solvent was concentrated under reduced pressure. The resulting crude product was purified by silica gel chromatography, followed by recrystallization from chloroform-methanol to obtain Compound 179 (colorless solid) (3.0 mg, 4%). MS(ESI):419(M+H)$^+$,417(M-H)$^-$

Example 11

cis-4-(4-Aminocyclohexyloxy)-N-(4-morpholinophenyl)-1H-pyrazolo[3,4-d]pyrimidin-6-amine (Compound 217)

[Formula 42]

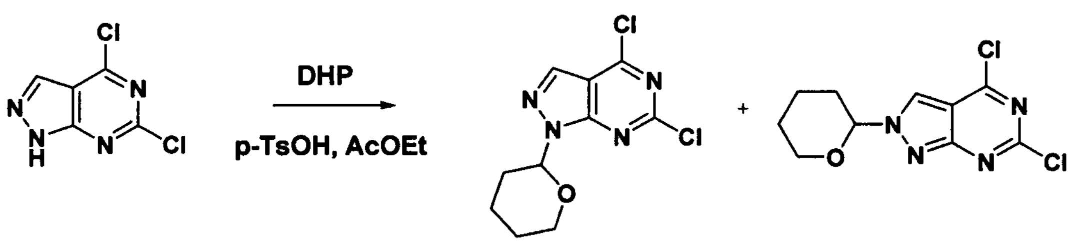


(1) Dihydropyrane (724 μl) and a catalytic amount of p-toluenesulfonic acid monohydrate were added to a solution of 4,6-dichloro-1H-pyrazolo[3,4-d]pyrimidine synthesized according to the method of JP-A-48-99194 (1.00 g) in ethyl acetate (30 ml). The mixture was stirred at room temperature for 35 minutes and then stirred at 50°C for 25 minutes. Silica gel was added to the reaction mixture, followed by concentration. Then, the residue was purified by silica gel chromatography (hexane/ethyl acetate = 8/1) to obtain 4,6-dichloro-1-(tetrahydro-2H-pyran-2-yl)-1H-pyrazolo[3,4-d]pyrimidine (872 mg, colorless powder) and 4,6-dichloro-2-(tetrahydro-2H-pyran-2-yl)-2H-pyrazolo[3,4-d]pyrimidine (672 mg, yellow oil). 4,6-Dichloro-1-(tetrahydro-2H-pyran-2-yl)-1H-pyrazolo[3,4-d]pyrimidine

$^1$H NMR(600MHz,CDCl3)δppm 1.63-1.68(m,1H),1.73-1.83(m,2H),1.93-1.98(m,1H),2.12-2.18(m,1H),2.52-2.60(m,1H), 3.78-3.84(m,1H),4.10-4.15(m,1H), 6.01(dd,J=10.6,2.8Hz,1H),8.20(s,1H) 4,6-Dichloro-2-(tetrahydro-2H-pyran-2-yl)-2H-pyrazolo[3,4-d]pyrimidine

$^{11}$H NMR(600MHz,CDC13)δppm 1.70-1.83(m,3H),1.92-2.05(m,2H),2.39-2.45(m,1H),3.78-3.85(m,1H),4.14-4.19(m, 1H),5.71(dd,J=8.9,3.0Hz,1H),8.45(s,1H)

[Formula 43]

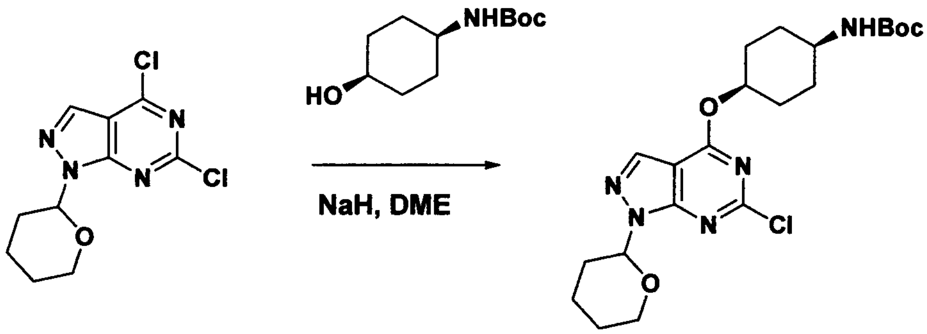


(2) cis-t-Butyl-4-hydroxycyclohexyl carbamate (680 mg) and sodium hydride (151 mg, ~60% in mineral oil) were added to a solution of 4,6-dichloro-1-(tetrahydro-2H-pyran-2-yl)-1H-pyrazolo[3,4-d]pyrimidine obtained in Example 11-(1) (870 mg) in dimethoxyethane (16.0 mL) under ice-cooling, and the mixture was stirred at room temperature overnight. The reaction mixture was filtered and silica gel was added to the filtrate, followed by concentration. Then, the residue was purified by silica gel chromatography to obtain cis-t-butyl-4-(6-chloro-1-(tetrahydro-2H-pyran-2-yl)-1H-pyrazolo[3,4-d]pyrimidin-4-yloxy)cyclohexyl carbamate (843 mg, colorless powder).
MS(ESI):474$(M+Na)^+$,450$(M-H)^-$
$^1$HNMR(600MHz,CDCl3)δppm 1.45(s,9H),1.58-2.15(m,14H),2.50-2.59(m,1H),3.58-3.67(m,1H),3.77-3.83(m,1H), 4.09-4.14(s,1H),4.52-4.59(m,1H),5.52-5.57(m,1H),5.93(dd,J=10.6,2.8Hz,1H),8.04(s,1H)

[Formula 44]

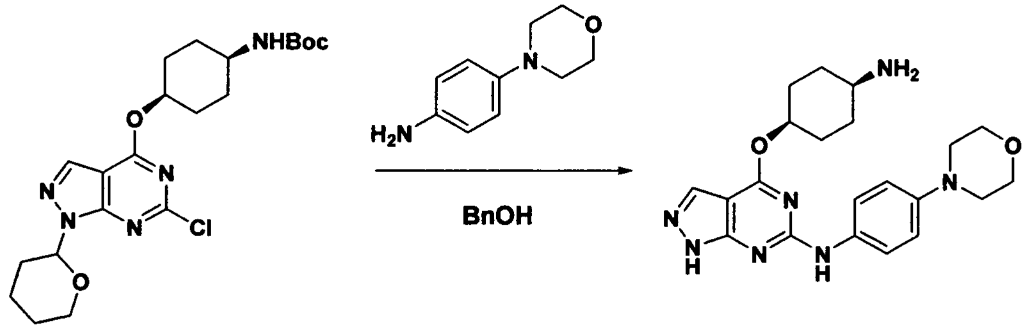


(3) A solution of cis-t-butyl-4-(6-chloro-1-(tetrahydro-2H-pyran-2-yl)-1H-pyrazolo[3,4-d]pyrimidin-4-yloxy)cyclohexyl carbamate obtained in Example 11-(2) (100 mg) and 4-morpholinoaniline (47 mg) in n-butanol (2.0 mL) was stirred at 130˚C for five hours. The green precipitate was filtered, washed with ethyl acetate and then purified by silica gel chromatography (NH silica gel, methanol/chloroform = 1 to 10%), followed by recrystallization (hexane/chloroform) to obtain Compound 217 (15 mg, pale gray powder).
MS(ESI):410$(M+H)^+$
$^1$HNMR(600MHz,DMSO-D6) δppm1.46-1.74(m,6H),1.98-2.04(m,2H),2.48-2.54(m,1H),2.77-2.83(m,1H),3.02-3.06(m, 4H),3.72-3.76(m,4H),6.87-6.91(m,2H),7.62-7.66(m,2H),7.85(s,1H),9.22(s,1H)

Example 12

N-(4-(3-(Methylthio)-6-(4-morpholinophenylamino)-1H-pyrazolo[3,4-d]pyrimidin-4-ylamino)phenyl)acetamide (Compound 234)

[Formula 45]

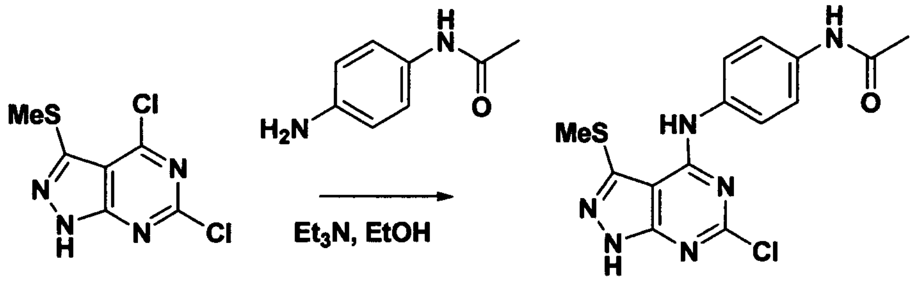


(1) Triethylamine (0.5 mL) was added to a solution of 4,6-dichloro-3-(methylsulfanyl)-1H-pyrazolo[3,4-d]pyrimidine obtained in Example 1-(2) (300 mg) and N-(4-aminophenyl)acetamide (211 mg) in ethanol (3.0 mL), and the mixture was stirred at 70˚C for 15 hours. After cooling to room temperature, the precipitated crystals were collected by filtration, washed with ethanol and dried to obtain N-(4-(6-chloro-3-(methylthio)-1H-pyrazolo[3,4-d]pyrimidin-4-ylamino)phenyl)acetamide (pale yellow powder) (424 mg, 94%).

[Formula 46]

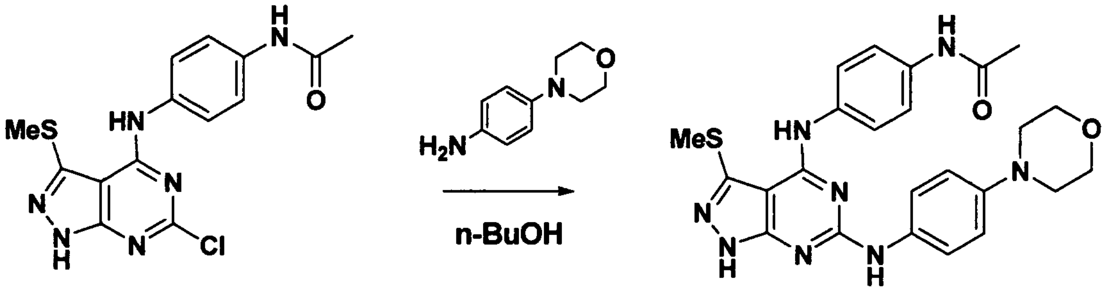


(2) A mixture of N-(4-(6-chloro-3-(methylthio)-1H-pyrazolo[3,4-d]pyrimidin-4-ylamino)phenyl)acetamide obtained in Example 12-(1) (100 mg), 4-morpholinoaniline (56 mg) and n-butanol (3.0 mL) was reacted at 130˚C for two hours. The reaction solution was concentrated, and then the resulting crude product was purified by silica gel chromatography (NH silica gel, methanol/chloroform = 0 to 10%), followed by recrystallization from hexanechloroform-methanol to obtain Compound 234 (colorless solid) (11 mg, 7.8%).
$^1$HNMR (600MHz,DMSO-D6) δppm 2.05(s,3H),2.58(s,3H),3.01-3.07(m,4H),3.72-3.77(m,4H),6.80-7.78(m,8H),8.10(s,1H), 9.02(s,1H),9.92(s,1H),13.05(brs,1H)

Example 13

3-(3-(Methylsulfonyl)-6-(4-morpholinophenylamino)-1H-pyrazolo[3,4-d]pyrimidin-4-ylamino)benzonitrile hydrochloride (Compound 194)

[Formula 47]

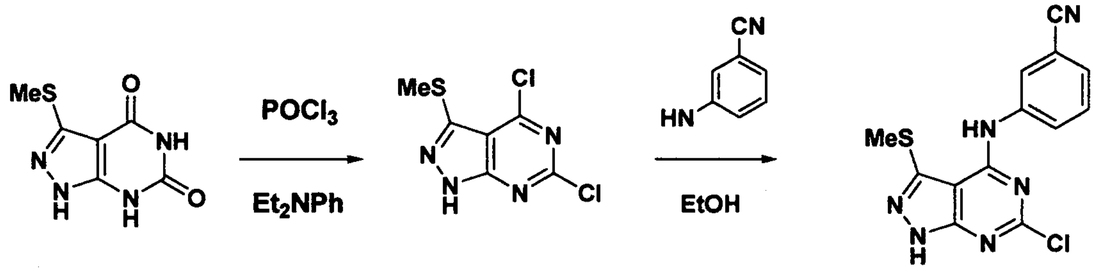


(1) N,N-diethylaniline (1.5 ml) was added dropwise to a suspension of 3-(methylsulfanyl)-1H-pyrazolo[3,4-d]pyrimidine-4,6(5H,7H)-dione obtained in Example 1-(1) (0.78 g) and phosphorus oxychloride (7.8 mL) under ice-cooling, and then the mixture was stirred at 110°C for 4.5 hours. The reaction solution was concentrated and then dissolved in chloroform, and the insoluble matter was separated by filtration. The objective product was extracted with chloroform. Triethylamine (1.4 mL) and 3-aminobenzonitrile (0.44 g) were added to a solution of 4,6-dichloro-3-(methylsulfanyl)-1H-pyrazolo[3,4-d]pyrimidine obtained by concentrating the extract in ethanol (11 mL) at room temperature. The mixture was stirred with heating under reflux for 13 hours. After cooling to room temperature, the precipitated solid was washed with ethanol and separated by filtration to obtain a crude product of 3-(6-chloro-3-(methylsulfanyl)-1H-pyrazolo[3,4-d]pyrimidin-4-ylamino)benzonitrile (pale yellow solid) (0.26 g, 21% (2 steps)).

[Formula 48]

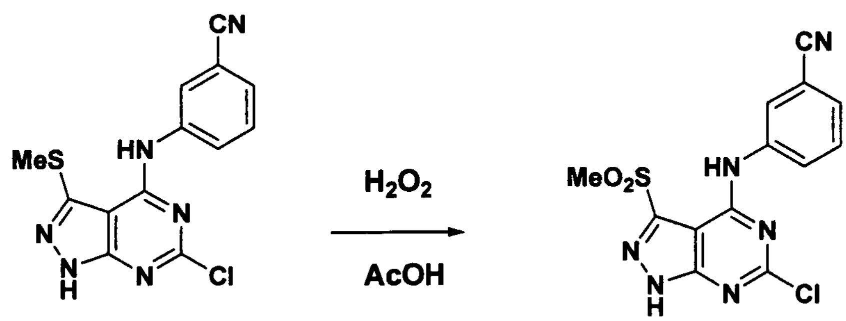


(2) A 35% hydrogen peroxide solution (2.0 mL) was added to a solution of the crude 3-(6-chloro-3-(methylsulfanyl)-1H-pyrazolo[3,4-d]pyrimidin-4-amino)benzonitrile obtained in Example 13-(1) (0.16 g) in acetic acid (2.0 mL) at room temperature, and the mixture was stirred at 80°C for 10.5 hours. After cooling to room temperature, water was added and the precipitate was separated by filtration and dried to obtain 3-(6-chloro-3-(methylsulfonyl)-1H-pyrazolo[3,4-d]pyrimidin-4-ylamino)benzonitrile (colorless solid) (0.11 g, 61%).

MS(ESI):349$(M+H)^+$,347$(M-H)^-$

$^1$HNMR(300MHz,DMSO-D6) δppm 3.58(s,3H),7.50-7.72(m,2H),7.90(bs,1H),8.31(s,1H),10.16(s,1H),15.18(bs, 1H)

[Formula 49]

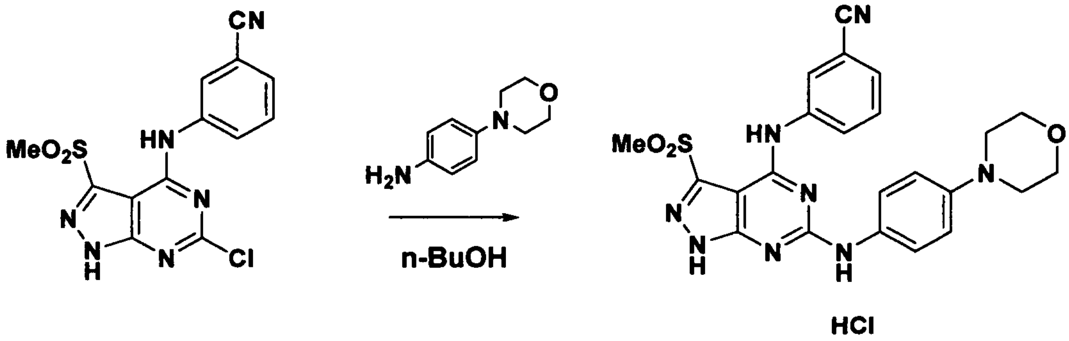


(3) A mixture of 3-(6-chloro-3-(methylsulfonyl)-1H-pyrazolo[3,4-d]pyrimidin-4-ylamino)benzonitrile obtained in Example 13-(2) (0.10 g), 4-morpholinoaniline (0.053 g), trimethylsilyl chloride (0.040 g) and n-butanol (1.5 mL) was reacted under microwave irradiation at 130˚C for two hours. The reaction solution was concentrated, and then the resulting crude product was washed with methanol and then separated by filtration and dried to obtain Compound 194 (pale blue solid) (0.076 g, 54%).
MS(ESI):491(M+H)$^+$,489(M-H)$^-$
$^1$HNMR(300MHz,DMSO-D6) δppm 3.10-3.40(m,4H),3.54(s,3H), 3.75-3.95(m,4H),7.05-7.95(m,6H),8.51(s,1H),9.71(bs, 1H), 9.99(s,1H),14.28(s,1H)

Example 14

3-(Methylthio)-N6-(4-morpholinophenyl)-N4-(thiazol-2-yl)-1H-pyrazolo[3,4-d]pyrimidine-4,6-diamine (Compound 235)

[Formula 50]

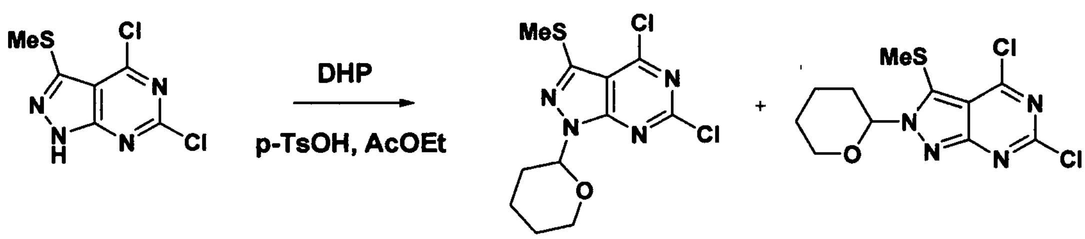


(1) Dihydropyrane (268 mg) and a catalytic amount of p-toluenesulfonic acid monohydrate were added to a solution of 4,6-dichloro-3-(methylsulfanyl)-1H-pyrazolo[3,4-d]pyrimidine obtained in Example 1-(2) (500 mg) in ethyl acetate (30 mL), and the mixture was stirred at room temperature for three hours. The reaction mixture was concentrated, and then the residue was purified by silica gel chromatography (hexane/ethyl acetate = 9/1 to 8/2) to obtain a mixture of 4,6-dichloro-3-(methylthio)-1-(tetrahydro-2H-pyran-2-yl)-1H-pyrazolo[3,4-d]pyrimidine and 4,6-dichloro-3-(methylthio)-1-(tetrahydro-2H-pyran-2-yl)-2H-pyrazolo[3,4-d]pyrimidine (colorless powder) (623 mg, 92%).
MS(ESI):319(M+H)$^+$
[Formula 51]

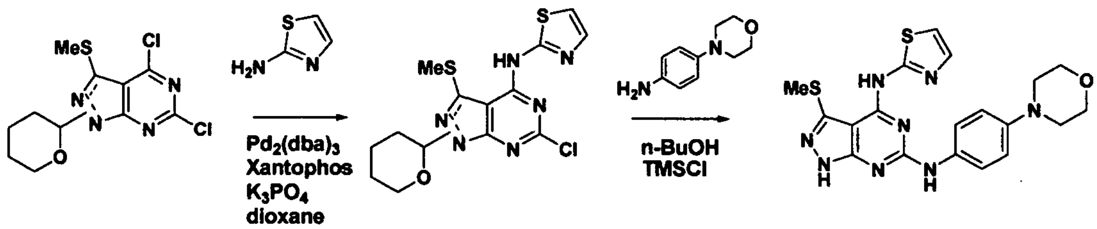


(2) A suspension of the mixture of 4,6-dichloro-3-(methylthio)-1-(tetrahydro-2H-pyran-2-yl)-1H-pyrazolo[3,4-d]pyrimidine and 4,6-dichloro-3-(methylthio)-1-(tetrahydro-2H-pyran-2-yl)-2H-pyrazolo[3,4-d]pyrimidine obtained in Example 14-(1) (70 mg), 2-aminothiazole (26 mg), $Pd_2(dba)_3$ (10 mg), 4,5-bis(diphenylphosphino)-9,9-dimethylxanthene (6 mg) and potassium phosphate (153 mg) in dioxane (2.0 mL) was stirred at 100˚C for 2.5 hours. After cooling to room temperature, water was added, followed by extraction with chloroform. The extract was dried over anhydrous magnesium sulfate. The drying agent was separated by filtration, and then the solvent was concentrated under reduced pressure. The residue was purified by silica gel chromatography (hexane/ethyl acetate = 2/1 to 1/1) to obtain a pale brown amorphous compound (46 mg). A solution of the resulting compound (31 mg) and 4-morpholinoaniline (16 mg) in n-butanol (1.0 mL) was stirred at 130˚C for two hours. Then, one drop of trimethylsilyl chloride was added and the mixture was stirred at 130˚C for 16 hours. The reaction solution was concentrated, and the resulting residue was purified by silica gel chromatography (NH silica gel, methanol/chloroform = 0 to 2%), followed by recrystallization from ethanol-ether to obtain Compound 235 (brown powder) (5 mg, 7.8% (2 steps)).
MS(ESI):441$(M+H)^+$,439$(M-H)^-$
$^1$HNMR(600MHz,$CDCl_3$) δppm 3.13-3.18(m,4H),3.86-3.91(m,4H),6.90-7.04(m,3H),7.44-7.55(m,3H), 9.25(bs,1H), 10.17(bs,1H)

The compounds shown in Tables 1-1 to 1-33 were prepared by the same methods as in the above Examples 1 to 14 using the corresponding raw materials, respectively. An Example No. indicates any of the above Examples based on which a compound shown in Tables 1-1 to 1-33 was prepared. A slash in the Tables indicates that measurement was not carried out.
The abbreviations in the Tables are shown below.
Free: Not forming a salt (free form)
MsOH: Methanesulfonic acid
BsOH: Benzenesulfonic acid
[Table 1-1]

| Compound No. | Compound | Salt | NMR | ESI MS $(M+H)^+$ | ESI MS$(M-H)^-$ | Example No. |
|---|---|---|---|---|---|---|
| 1 | 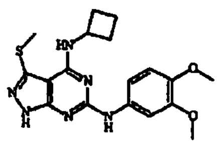 | Free | $^1H$ NMR (500 MHz, DMSO-D6) δ ppm 1.65-1.70 (m, 2H), 2.05-2.18 (m, 2H), 2.32-2.40 (m, 2H), 2.56 (s, 3H), 3.73 (s, 3H), 3.79 (s, 3H), 4.68-4.75 (m, 1H), 6.37 (bs, 1H), 6.87 (d, J=8.6Hz, 1H), 7.20-7.35 (m, 1H), 7.54 (s, 1H), 8.87 (bs, 1H), 12.84 (bs, 1H) | 387 | 385 | 6 |
| 2 | 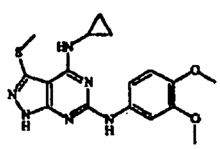 | Free | $^1H$ NMR (500 MHz, DMSO-D6) δ ppm 0.72-0.80 (m, 2H), 0.85-0.95 (m, 2H), 2.55 (s, 3H), 3.00 (bs, 1H), 3.74 (s, 3H), 3.76 (s, 3H), 6.91 (d, J=8.7Hz, 1H), 7.32 (d, J=8.3Hz, 1H), 7.41 (s, 1H), 9.48 (bs, 1H), 13.0 (bs, 1H) | 373 | 371 | 6 |

(continued)

| Compound No. | Compound | Salt | NMR | ESI MS $(M+H)^+$ | ESI MS$(M-H)^-$ | Example No. |
|---|---|---|---|---|---|---|
| 3 | 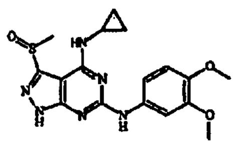 | Free | $^1H$ NMR (300 MHz, DMSO-D6) δ ppm 0.53-0.58 (m, 2H), 0.82-0.87 (m, 2H), 2.91 (s, 3H), 3.03 (bs, 1H), 3.72 (s, 3H), 3.75 (s, 3H), 6.87 (d, J=8.7Hz, 1H), 7.40 (d, J=8.7Hz 1H), 7.54 (d, J=2.1Hz, 1H), 8.46 (s, 1H), 9.12 (bs, 1H), 13.48 (bs, 1H) | 389 | 387 | 5 |
| 4 | 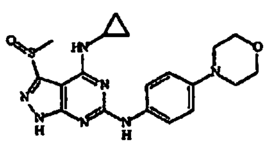 | Free | $^1H$ NMR (500 MHz, DMSO-D6) δ ppm 0.49-0.58 (m, 2H), 0.82-0.90 (m, 2H), 2.90 (s, 3H), 2.99 (bs, 1H), 3.05-3.15 (m, 4H), 3.72-3.78 (m, 4H), 6.89 (d, J=8.0Hz, 2H), 7.74 (d, J=8.6Hz, 2H), 8.33 (s, 1H), 9.11 (s, 1H), 13.49 (s, 1H) | 414 | 412 | 5 |

(continued)

| Compound No. | Compound | Salt | NMR | ESI MS $(M+H)^+$ | ESI MS$(M-H)^-$ | Example No. |
|---|---|---|---|---|---|---|
| 5 | 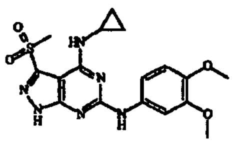 | Free | $^1H$ NMR (500 MHz, DMSO-D6) δ ppm 0.55-0.60 (m, 2H), 0.85-0.89 (m, 2H), 3.04 (bs, 1H), 3.38 (s, 3H), 3.72 (s, 3H), 3.75 (s, 3H), 6.87 (d, J=8.6Hz, 1H), 7.42 (d, J=8.6Hz, 1H), 7.52 (s, 1H), 7.57 (d, J=3.4Hz, 1H), 9.05 (s, 1H), 13.82 (s, 1H) | 405 | 403 | 1 |
| 6 | 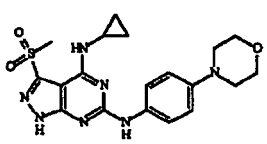 | Free | $^1H$ NMR (300 MHz, DMSO-D6) δ ppm 0.62-0.67 (m, 2H), 0.92-0.98 (m, 2H), 3.03 (bs, 1H), 3.44 (s, 3H), 3.54-3.60 (m, 4H), 4.07-4.13 (m, 4H), 7.78-7.80 (m, 2H), 7.90 (s, 1H), 8.03-8.05 (m, 2H), 9.99 (s, 1H), 14.28 (bs, 1H) | 430 | 428 | 1 |

(continued)

| Compound No. | Compound | Salt | NMR | ESI MS $(M+H)^+$ | ESI MS$(M-H)^-$ | Example No. |
|---|---|---|---|---|---|---|
| 7 | 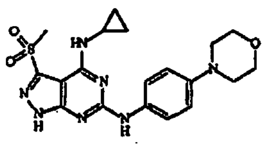 | HCl | $^1H$ NMR (600 MHz, DMSO-D6) δ ppm 0.57-0.65 (m, 2H), 0.88-0.94 (m, 2H), 2.99-3.04 (m, 1H), 3.34-3.51 (m, 4H), 3.42 (s, 3H), 3.95-4.01 (m, 4H), 7.49-7.55 (m, 2H), 7.73 (bs, 1H), 7.95-7.98 (m, 2H), 9.65 (bs, 1H), 14.08 (bs, 1H) | 430 | 428 | 2 |

[Table 1-2]

| Compound No. | Compound | Salt | NMR | ESI MS $(M+H)^+$ | ESI MS $(M-H)^-$ | Example No. |
|---|---|---|---|---|---|---|
| 8 | 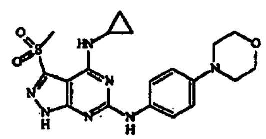 | $H_2SO_4$ | $^1H$ NMR (600 MHz, DMSO-D6) δ ppm 0.46-0.74 (m, 2H), 0.82-0.97 (m, 2H), 2.99-3.05 (m, 1H), 3.27-3.37 (m, 4H), 3.42 (s, 3H), 3.82-3.94 (m, 4H), 5.21 (s, 3 H), 7.17-7.35 (m, 2H), 7.68 (bs, 1H), 7.82-8.00 (m, 2H), 9.48 (bs, 1H), 14.00 (bs, 1H) | | | 2 |
| 9 | 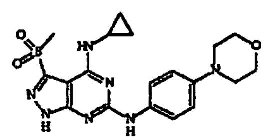 | $H_3PO_4$ | $^1H$ NMR (600 MHz, DMSO-D6) δ ppm 0.54-0.59 (m, 2H), 0.85-0.91 (m, 2H), 2.97-3.01 (m, 1H), 3.01-3.05 (m, 4H), 3.29-3.36 (m, 3H), 3.40 (s, 3H), 3.72-3.76 (m, 4H), 6.85-6.93 (m, 2H), 7.56-7.61 (m, 1H), 7.69-7.77 (m, 2H), 9.17 (s, 1H), 13.88 (s, 1H) | | | 2 |
| 10 | 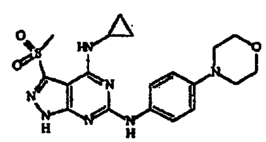 | BsOH | $^1H$ NMR (600 MHz, DMSO-D6) δ ppm 0.51-0.69 (m, 2H), 0.82-0.99 (m, 2H), 2.94-3.10 (m, 1H), 3.18-3.37 (m, 4H), 3.41 (s, 3H), 3.75-3.98 (m, 4H), 4.41 (m, 2H), 7.10-7.24 (m, 2H), 7.25-7.38 (m, 3H), 7.55-7.62 (m, 2H), 7.63-7.72 (m, 1H), 7.79-7.99 (m, 2H), 13.98 (s, 1H) | | | 2 |
| 11 | 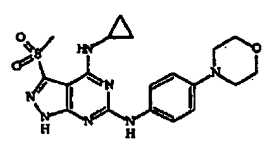 | MsOH | $^1H$ NMR (600 MHz, DMSO-D6) δ ppm 0.57-0.68 (m, 2H), 0.87-0.96 (m, 2H), 2.36 (s, 3H), 2.93-3.06 (m, 1H), 3.35-3.50 (m, 4H), 3.42 (s, 3H), 3.69-4.10 (m, 4H), 7.33-7.54 (m, 2H), 7.68-7.76 (bs, 1H), 7.87-8.07 (m, 2H), 9.60 (bs, 1H), 14.05 (bs, 1H) | | | 2 |
| 12 | 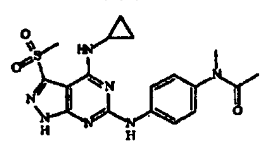 | Free | $^1H$ NMR (300 MHz, DMSO-D6) δ ppm 0.56-0.59 (m, 2H), 0.88-0.91 (m, 2H), 1.78 (s, 3H), 2.91 (bs, 1H), 3.03 (s, 3H), 3.38 (s, 3H), 7.18-7.22 (m, 2H), 7.63 (s, 1H), 7.93-7.96 (m, 2H), 9.43 (bs, 1H), 13.91 (bs, 1H) | 416 | 414 | 1 |
| 13 | 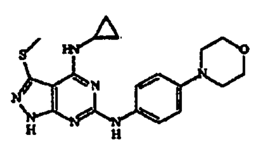 | Free | $^1H$ NMR (300 MHz, DMSO-D6) δ ppm 0.77-0.80 (m, 2H), 0.92-0.95 (m, 2H), 2.56 (s, 3H), 2.96 (bs, 1H), 3.05-3.25 (m, 4H), 3.75-3.85 (m, 4H), 7.10-7.20 (m, 2H), 7.27 (bs, 1H), 7.60-7.80 (m, 2H), 9.88 (bs, 1H), 12.89 (bs, 1H) | 398 | 396 | 6 |
| 14 | 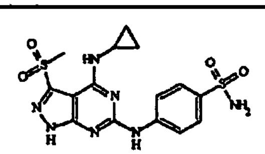 | Free | $^1H$ NMR (300 MHz, DMSO-D6) δ ppm 0.57-0.61 (m, 2H), 0.89-0.92 (m, 2H), 3.03 (bs, 1H), 3.39 (s, 3H), 7.16 (bs, 2H), 7.67-7.76 (m, 2H), 8.05-8.13 (m, 2H), 9.82 (bs, 1H), 14.12 (bs, 1H) | 424 | 422 | 1 |

[Table 1-3]

| Compound No. | Compound | Salt | NMR | ESI MS $(M+H)^+$ | ESI MS $(M-H)^-$ | Example No. |
|---|---|---|---|---|---|---|
| 15 | 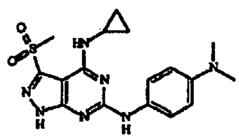 | Free | $^1H$ NMR (500 MHz, DMSO-D6) δ ppm 0.57-0.62 (m, 2H), 0.87-0.83 (m, 2H), 2.98-3.10 (m, 7H), 3.39 (s, 3H), 7.15-7.55 (brm, 2H), 7.64 (s, 1H), 7.85-8.15 (m, 2H), 9.40 (bs, 1H), 13.91 (s, 1H) | 388 | 386 | 1 |
| 16 | 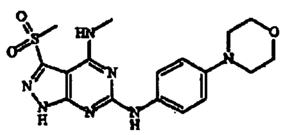 | Free | $^1H$ NMR (300 MHz, DMSO-D6) δ ppm 3.08 (s, 3H), 3.20-3.40 (m, 4H), 3.39 (s, 3H), 3.75-3.95 (m, 4H), 7.10-7.35 (m, 2H), 7.46 (s, 1H), 7.70-7.85 (m, 2H), 9.28 (bs, 1H), 13.87 (bs, 1H) | 404 | 402 | 1 |

(continued)

| Compound No. | Compound | Salt | NMR | ESI MS $(M+H)^+$ | ESI MS $(M-H)^-$ | Example No. |
|---|---|---|---|---|---|---|
| 17 | 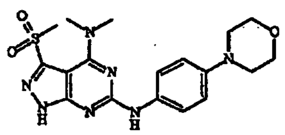 | Free | $^1H$ NMR (300 MHz, DMSO-D6) δ ppm 3.10-3.36 (m, 10H), 3.43 (s, 3H), 3.70-3.94 (m, 4H), 7.05-7.33 (m, 2H), 7.74 (d, J=8.7Hz, 2H), 9.19 (bs, 1H), 13.72 (bs, 1H) | 418 | 416 | 1 |
| 18 | 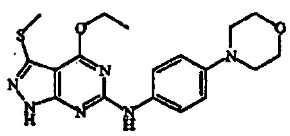 | Free | $^1H$ NMR (500 MHz, DMSO-D6) δ ppm 1.39 (t, J=7.0Hz, 3H), 2.52 (s, 3H), 3.04 (t, J=4.9Nz, 4H), 3.74 (t, J=4.9Hz, 4H), 4.51 (q, J=7.0Hz, 2H), 6.89 (d, J=9.2Hz, 2H), 7.63 (d, J=8.9Hz, 2H), 9.29 (s, 1H), 12.99 (s, 1H) | 387 | 385 | 7 |

(continued)

| Compound No. | Compound | Salt | NMR | ESI MS $(M+H)^+$ | ESI MS $(M-H)^-$ | Example No. |
|---|---|---|---|---|---|---|
| 19 | 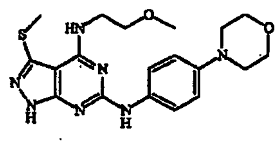 | Free | $^1H$ NMR (500 MHz, DMSO-D6) δ ppm 2.53 (s, 3H), 3.02 (t, J=4.9Hz, 4H), 3.31 (s, 3H), 3.56 (t, J=5.5Hz, 2H), 3.70 (t, J=5.5Hz, 2H), 3.73 (t, J=4.9Hz, 4H), 6.38 (s, 1H), 6.86 (d, J=9.2Hz, 2H), 7.63 (d, J=8.9Hz, 2H), 8.88 (s, 1H), 12.84 (s, 1H) | 416 | 414 | 6 |
| 20 | 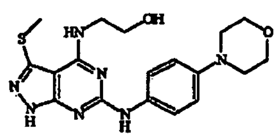 | Free | $^1H$ NMR (500 MHz, DMSO-D6) δ ppm 2.54 (s, 3H), 3.02 (t, J=4.9Hz, 4H), 3.58-3.64 (m, 4H), 3.73 (t, J=4.9Hz, 4H), 4.89 (bs, 1H), 6.40 (s, 1H), 6.86 (d, J=8.9Hz, 2H), 7.63 (d, J=8.9Hz, 2H), 8.87 (s, 1H), 12.84 (s, 1H) | 402 | 400 | 6 |

(continued)

| Compound No. | Compound | Salt | NMR | ESI MS $(M+H)^+$ | ESI MS $(M-H)^-$ | Example No. |
|---|---|---|---|---|---|---|
| 21 | 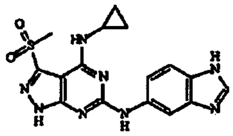 | Free | $^1H$ NMR (500 MHz, DMSO-D6) δ ppm 0.59-0.61 (m, 2H), 0.90-0.98 (m, 2H), 3.05 (bs, 1H), 3.42 (s, 3H), 7.38-7.70 (m, 3H), 8.09 (s, 1H), 8.30-8.42 (bs, 1H), 9.30-9.40 (bs, 1H), 12.28 (s, 1H), 13.94 (s, 1H) | 385 | 383 | 1 |

[Table 1-4]

| Compound No. | Compound | Salt | NMR | ESI MS $(M+H)^+$ | ESI MS $(M-H)^-$ | Example No. |
|---|---|---|---|---|---|---|
| 22 | 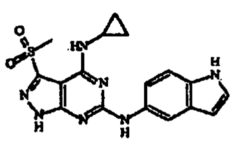 | Free | $^1H$ NMR (500 MHz, DMSO-D6) δ ppm 0.5-0.61 (m, 2H), 0.87-0.91 (m, 2H), 3.03-3.05 (bs, 1H), 3.41 (s, 3H), 6.33 (s, 1H), 7.26-7.29 (m, 2H), 7.40 (d, J=7.3Hz, 1H), 7.56 (s, 1H), 8.14 (bs, 1H), 9.13 (s, 1H), 10.91 (bs, 1H), 13.85 (bs, 1H) | 384 | 382 | 1 |

(continued)

| Compound No. | Compound | Salt | NMR | ESI MS $(M+H)^+$ | ESI MS $(M-H)^-$ | Example No. |
|---|---|---|---|---|---|---|
| 23 | 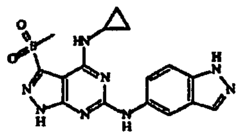 | Free | $^1H$ NMR (500 MHz, DMSO-D6) δ ppm 0.59-0.62 (m, 2H), 0.90-0.93 (m, 2H), 3.04 (bs, 1H), 3.42 (s, 3H), 7.45 (d, J=8.9Hz, 1H), 7.62 (s, 1H), 7.66 (d, J=8.9Hz, 1H), 7.98 (s, 1H), 8.43 (bs, 1H), 9.37 (s, 1H), 12.90 (bs, 1H), 13.94 (bs, 1H) | 385 | 383 | 1 |
| 24 | 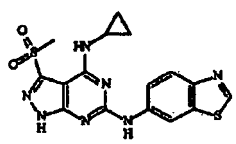 | Free | $^1H$ NMR (300 MHz, DMSO-D6) δ ppm 0.50-0.70 (m, 2H), 0.75-0.95 (m, 2H), 3.04 (bs, 1H), 3.40 (s, 3H), 7.67 (s, 1H), 7.75-8.00 (m, 2H), 8.92 (s, 1H), 9.16 (s, 1H), 9.61 (s, 1H), 13.94 (s, 1H) | 402 | 400 | 1 |

(continued)

| Compound No. | Compound | Salt | NMR | ESI MS (M+H)$^+$ | ESI MS (M-H)$^-$ | Example No. |
|---|---|---|---|---|---|---|
| 25 | 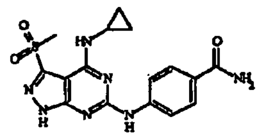 | Free | $^1$H NMR (500 MHz, DMSO-D6) δ ppm 0.58-0.62 (m, 2H), 0.89-0.93 (m, 2H), 3.04 (bs, 1H), 3.43 (s, 3H), 7.16 (s, 1H), 7.72 (8, 1H), 7.74-7.87 (m, 3H), 8.00 (d, J=8.9Hz, 2H), 9.72 (s, 1H), 14.10 (s, 1H) | 388 | 386 | 1 |
| 26 | 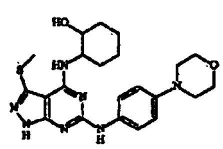 | Free | $^1$H NMR (500 MHz, DMSO-D6) δ ppm 1.25-1.45 (m, 2H), 1.25-1.70 (m, 4H), 1.70-1.80 (m, 2H), 2.54 (s, 3H), 3.02 (t, J=4.9Hz, 4H), 3.73(t, J=4.9Hz, 4H), 3.89 (bs, 1H), 4.14 (bs, 1H), 5.03 (s, 1H), 6.3 (s, 1H), 6.85 (d, J=9.2Hz, 2H), 7.63 (d, J=8.9Hz, 2H), 8.86 (s, 1H), 12.83 (s, 1H) | 456 | 454 | 6 |

(continued)

| Compound No. | Compound | Salt | NMR | ESI MS $(M+H)^+$ | ESI MS $(M-H)^-$ | Example No. |
|---|---|---|---|---|---|---|
| 27 | 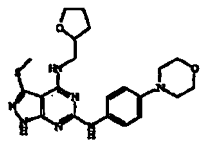 | Free | $^1H$ NMR (500 MHz, DMSO-D6) δ ppm 1.54-2.10 (m, 4H), 2.54 (s, 3H), 3.02 (t, J=4.9Hz, 4H), 3.50-4.20 (m, 9H), 6.31 (s, 1H), 6.86 (d, J=9.2Hz, 2H), 7.63 (d, J=8.9Hz, 2H), 8.89 (s, 1H), 12.86 (s, 1H) | 442 | 440 | 6 |
| 28 | 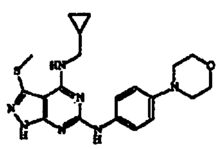 | Free | $^1H$ NMR (500 MHz, DMSO-D6) δ ppm 0.29-033(m, 2H), 0.44-0.49 (m, 2H), 1.21 (bs, 1H), 2.54 (s, 3H), 3.02 (t, J=4.9Hz, 4H), 3.41 (t, J=6.4Hz, 2H), 3.73 (t, J=4.9Hz, 4H), 6.42 (s, 1H), 6.86 (d, J=9.2Hz, 2H), 7.64 (d, J=8.9Hz, 2H), 8.85 (s, 1H), 12.82 (s, 1H) | 412 | 410 | 6 |

[Table 1-5]

| Compound No. | Compound | Salt | NMR | ESI MS $(M+H)^+$ | ESI MS (M-H)· | Example No. |
|---|---|---|---|---|---|---|
| 29 | 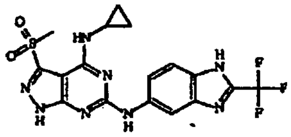 | Free | $^1$H NMR (500 MHz, DMSO-D6) δ ppm 0.58-0.62 (m. 2H), 0.95-1.02 (m, 2H), 3.06 (bs, 1H), 3.43 (s, 3H), 7.60-7.78 (m, 3H), 8.55 (s, 1H), 9.63 (s, 1H), 14.03 (s, 1H) | 453 | 451 | 1 |
| 30 | 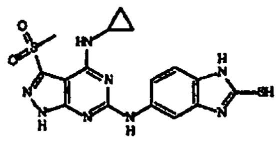 | Free | $^1$HNMR (500 MHz, DMSO-D6) δ ppm 0.55-0.58 (m, 2H), 0.95-1.00 (m, 2H), 3.04 (bs, 1H), 3.42 (s, 3H), 7.03 (d, J=8.6Hz, 1H), 7.41 (d, J=8.0Hz, 1H), 7.67 (d, J=2.5Hz, 1H), 8.13 (bs, 1H), 9.46 (s, 1H), 12.37 (s, 1H), 12.48 (s, 1H), 13.94 (s, 1H) | 417 | 415 | 1 |

(continued)

| Compound No. | Compound | Salt | NMR | ESI MS (M+H)+ | ESI MS (M-H)- | Example No. |
|---|---|---|---|---|---|---|
| 31 | 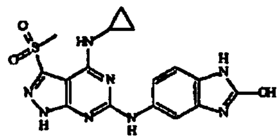 | Free | $^{1}$H NMR (500 MHz, DMSO-D6) δ ppm 0.54-0.58 (m, 2H), 0.89-0.94 (m, 2H), 3.01 (bs, 1H), 3.41 (s, 3H), 6.81 (d, J=8.3Hz, 1H), 7.29 (d, J=8.3Hz, 1H), 7.60 (s, 1H), 7.72 (bs, 18), 9.24 (s, 1H), 10.40 (s, 1H), 10.56 (s, 1H), 13.89 (s, 1H) | 401 | 399 | 1 |
| 32 | 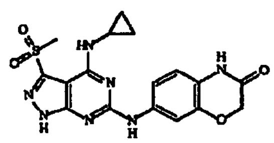 | Free | $^{1}$H NMR (300 MHz, DMSO-D6) δ ppm 0.50-0.65 (m, 2H), 0.80-0.95 (m, 2H), 2.99 (bs, 1H), 3.38 (s, 3H), 4.52 (s, 2H), 6.78 (d, J=8.4Hz, 1H), 7.32 (d, J=9.0Hz, 1H), 7.59 (s, 1H), 7.77 (s, 1H), 9.22 (s, 1H), 10.43 (s, 1H), 13.86 (s, 1H) | 416 | 414 | 1 |

(continued)

| Compound No. | Compound | Salt | NMR | ESI MS (M+H)+ | ESI MS (M-H)- | Example No. |
|---|---|---|---|---|---|---|
| 33 | 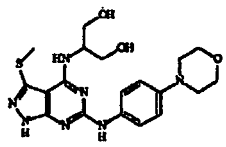 | Free | 1H NMR (500 MHz, DMSO-D6) δ ppm 253 (s, 3H), 3.02 (t, J=4.9Hz, 4H), 3.54-3.70 (m, 4H), 3.73 (t, J=4.9Hz, 4H), 4.27 (bs, 1H), 4.90 (t, J=4.9Hz, 2H), 6.23 (d, J=8.3Hz, 1H), 6.86 (d, J=8.9Hz, 2H), 7.63 (d, J=8.9Hz, 2H), 8.86 (s, 1H), 12.85 (s, 1H) | 432 | 430 | 6 |
| 34 | 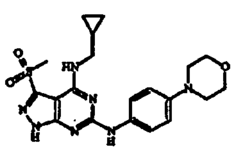 | Free | 1H NMR (500 MHz, DMSO-D6) δ ppm 0.27-0.32 (m, 2H), 0.49-0.54 (m, 2H), 1.07-1.16 (m, 1H), 3.02-3.08 (m, 4H), 3.38-3.45 (m, 5H), 3.72-3.77 (m, 4H), 6.90 (d, J=8.6Hz, 2H), 7.55-7.70 (m, 3H), 9.11 (s, 1H), 13.89 (s, 1H) | 444 | 442 | 1 |

(continued)

| Compound No. | Compound | Salt | NMR | ESI MS $(M+H)^+$ | ESI MS $(M-H)^-$ | Example No. |
|---|---|---|---|---|---|---|
| 35 | 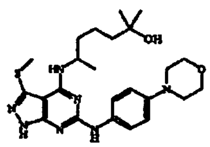 | Free | $^1$H NMR (500 MHz, DMSO-D6) δ ppm 1.02-1.06 (m, 6H), 1.25 (d, J=6.4Hz, 3H), 1.30-1.70 (m, 6H), 2.53 (s, 3H), 3.02 (t, J=4.9Hz, 4H), 3.73 (t, J=4.9Hz, 4H), 4.09 (s, 1H), 4.34 (bs, 1H), 5.86 (d, J=8.3Hz, 1H), 6.86 (d, J=9.2Hz, 2H), 7.63 (d, J=9.2Hz, 2H), 8.86 (s, 1H), 12.84 (s, 1H) | 486 | 484 | 6 |

[Table 1-6]

| Compound No. | Compound | Salt | NMR | ESI MS $(M+H)^+$ | ESI MS $(M-H)^-$ | Example No. |
|---|---|---|---|---|---|---|
| **36** | 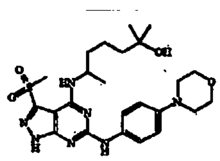 | Free | $^1$H NMR (500 MHz, DMSO-D6) δ ppm 1.02-1.08 (m, 6H), 1.23 (d, J=6.4Hz, 3H), 1.30-1.60 (m, 6H), 3.03 (t, J=4.9Hz, 4H), 3.41 (s, 3H), 3.74 (t, J=4.9Hz, 4H), 4.09 (s, 1H), 4.30 (bs, 1H), 6.88 (d, J=9.2Hz, 2H), 7.41 (d, J=7.4Hz, 1H), 7.63 (d, J=9.2Hz, 2H), 9.06 (s, 1H), 13.84 (s, 1H) | 518 | 516 | 1 |

(continued)

| Compound No. | Compound | Salt | NMR | ESI MS $(M+H)^+$ | ESI MS $(M-H)^-$ | Example No. |
|---|---|---|---|---|---|---|
| **37** | 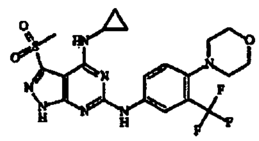 | HCl | $^1H$ NMR (300 MHz, DMSO-D6) δ ppm 0.45-0.60 (m, 2H), 0.75-0.91 (m, 2H), 2.65-2.85 (m, 4H), 3.02 (bs, 1H), 3.39 (s, 3H), 3.52-3.72 (m, 4H), 7.50 (d, J=8.4Hz, 1H), 7.67 (s, 1H), 8.08 (s, 1H), 8.30 (s, 1H), 9.55 (s, 1H), 13.94 (bs, 1H) | 498 | 496 | 1 |
| **38** | 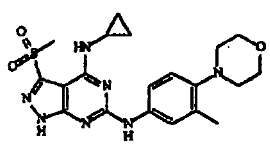 | HCl | $^1H$ NMR (500 MHz, DMSO-D6) δ ppm 0.55-0.65 (m, 2H), 0.83-0.91 (m, 2H), 2.27 (s, 3H), 2.80-2.87 (m, 4H), 3.03 (bs, 1H), 3.38 (s, 3H), 3.72-3.78 (m, 4H), 7.00 (d, J=8.6Hz, 1H), 7.55-7.73 (m, 3H), 9.05 (s, 1H), 13.81 (s, 1H) | 444 | 442 | 1 |

(continued)

| Compound No. | Compound | Salt | NMR | ESI MS $(M+H)^+$ | ESI MS $(M-H)^-$ | Example No. |
|---|---|---|---|---|---|---|
| **39** | 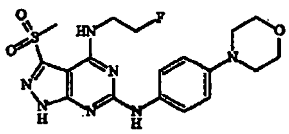 | Free | $^1H$ NMR (300 MHz, DMSD-D6) δ ppm 3.04-3.05 (m, 4H), 3.42 (s, 3H), 3.74-3.76 (m, 4H), 3.82-3.84 (m, 1H), 3.92-3.93 (m, 1H), 4.57-4.60 (m, 1H), 4.73-4.76 (m, 1H), 6.89 (d, J=9.0Hz, 2H), 7.61 (d, J=9.0Hz, 2H), 7.67-7.71 (m, 1H), 9.15 (bs, 1H) | 436 | 434 | 1 |
| **40** | 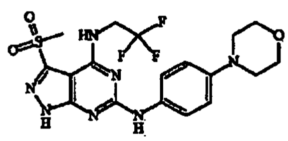 | Free | $^1H$ NMR (300 MHz, DMSO-D6) δ ppm 2.73-2.74 (m, 4H), 3.45 (s, 3H), 3.73-3.76 (m, 4H), 4.48-4.60 (m, 2H), 6.90 (d, J=9.0Hz, 2H), 7.60 (d, J=9.0Hz, 2H), 7.74-7.79 (m, 1H), 9.30 (bs, 1H), 14.07 (bs, 1H) | 472 | 470 | 1 |

(continued)

| Compound No. | Compound | Salt | NMR | ESI MS (M+H)+ | ESI MS (M-H)- | Example No. |
|---|---|---|---|---|---|---|
| **41** | 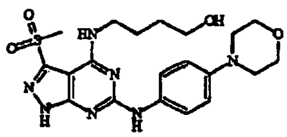 | Free | $^1H$ NMR (300 MHz, DMSO-D6) δ ppm 1.08-1.24 (m, 4H), 3.02-3.05 (m, 4H), 3.40-3.48 (m, 5H), 3.32-3.58 (m, 2H), 3.72-3.76 (m, 4H), 6.88 (d, J=9.3Hz, 2H), 7.46-7.52 (m, 1H), 7.64 (d, J=9.0Hz, 2H), 9.10 (bs, 1H), 13.86 (bs, 1H) | 462 | 460 | 1 |
| **42** | 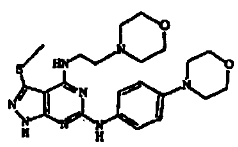 | Free | $^1H$ NMR (300 MHz, DMSO-D6) δ ppm 2.47-2.61 (m, 6H), 3.00-3.03 (m, 4H), 3.30 (s, 3H), 3.60-3.63 (m, 6H), 3.72-3.75 (m, 4H), 6.64-6.67 (m, 1H), 6.84-6.87 (m, 2H), 7.62-7.65 (m, 2H), 8.86 (bs, 1H), 12.83 (bs, 1H) | 471 | 469 | 6 |

[Table 1-7]

| Compound No. | Compound | Salt | NMR | ESI MS $(M+H)^+$ | ESI MS $(M-H)^-$ | Example No. |
|---|---|---|---|---|---|---|
| **43** | 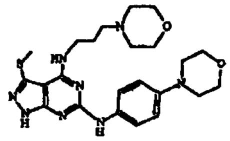 | Free | $^1H$ NMR (300 MHz, DMSO-D6) δ ppm 1.79-1.81 (m, 2H), 2.32-2.40 (m, 6H), 3.00-3.04 (m, 4H), 3.30 (s, 3H), 3.57-3.61 (m, 6H), 3.72-3.75 (m, 4H), 6.47 (bs, 1H), 6.84-6.87 (m, 2H), 7.62-7.65 (m, 2H), 8.82(bs, 1H), 12.80 (bs, 1H) | 485 | Not detected | 6 |
| **44** | 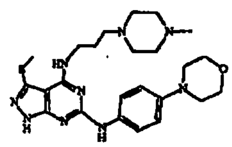 | Free | $^1H$ NMR (300 MHz, DMSO-D6) δ ppm 1.75-1.80 (m, 2H), 2.14 (s, 3H), 2.35-2.39 (m, 10H), 3.00-3.04 (m, 4H), 3.32 (s, 3H), 3.53-3.57 (m, 2H), 3.72-3.75 (m, 4H), 6.82-6.87 (m, 2H), 7.63-7.66 (m, 2H), 8.82 (bs, 1H), 12.80 (bs, 1H) | 499 | 496 | 6 |

(continued)

| Compound No. | Compound | Salt | NMR | ESI MS $(M+H)^+$ | ESI MS $(M-H)^-$ | Example No. |
|---|---|---|---|---|---|---|
| **45** | 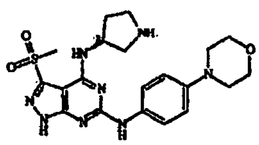 | Free | $^1H$ NMR (300 MHz, DMSO-D6) δ ppm 1.66-1.70 (m, 1H), 2.12-2.17 (m, 1H), 2.98-3.01 (m, 4H), 3.03-3.08 (m, 1H), 3.16 (s, 3H), 3.59-3.65 (m, 1H), 3.72-3.75 (m, 6H), 4.55-4.56 (m, 1H), 6.82-6.85 (m, 2H), 7.41-7.43 (m, 1H), 7.77-7.90 (m, 2H), 8.16 (bs, 1H) | 459 | 457 | 4 |
| **46** | 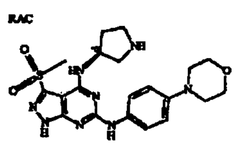 | Free | $^1H$ NMR (300 MHz, DMSO-D6) δ ppm 1.67-1.71 (m, 1H), 2.15-2.17 (m, 1H), 2.98-3.00 (m, 4H), 3.04-3.09 (m, 1H), 3.16 (s, 3H), 3.60-3.65 (m, 1H), 3.72-3.73 (m, 6H), 4.57-4.59 (m, 1H), 6.82-6.85 (m, 2H), 7.41-7.43 (m, 1H), 7.77-7.80 (m, 2H), 8.17 (bs, 1H) | 459 | 457 | 4 |

(continued)

| Compound No. | Compound | Salt | NMR | ESI MS $(M+H)^+$ | ESI MS $(M-H)^-$ | Example No. |
|---|---|---|---|---|---|---|
| **47** | 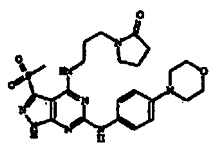 | Free | $^1H$ NMR (300 MHz, DMSO-D6) δ ppm 1.72-1.98 (m, 4H), 2.20 (t, J=8.3Hz, 2H), 2.90-3.05 (m, 4H), 3.20-3.40 (m, 4H), 3.42 (s, 3H), 3.45-3.60 (m, 2H), 3.62-3.85 (m, 4H), 6.89 (d, J=9.1Hz, 2H), 7.49 (m, 1H), 7.63 (d, J=8.6Hz, 2H), 9.11 (s, 1H), 13.85 (s, 1H) | 515 | 513 | 1 |
| **48** | 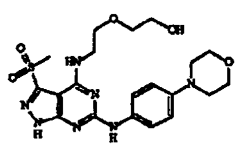 | Free | $^1H$ NMR (300 MHz, DMSO-D6) δ ppm 2.90-3.12 (m, 4H), 3.20-3.60 (m, 9H), 3.60-3.90 (m, 6H), 4.55 (m, 1H), 6.89 (d, J=9.1Hz, 2H), 7.50-7.70 (m, 3H), 9.11 (s, 1H), 13.87 (s, 1H) | 478 | 476 | 1 |

(continued)

| Compound No. | Compound | Salt | NMR | ESI MS $(M+H)^+$ | ESI MS $(M-H)^-$ | Example No. |
|---|---|---|---|---|---|---|
| 49 | 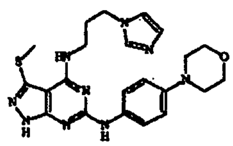 | Free | $^1H$ NMR (300 MHz, DMSO-D6) δ ppm 2.02-2.17 (m, 2H), 2.95-3.10 (m, 4H), 3.32 (s, 3H), 3.45-3.56 (m, 2H), 3.65.3.80 (m, 4H), 3.95-4.10 (m, 2H), 6.54 (m, 1H), 6.80-6.90 (m, 3H), 7.20 (s, 1H), 7.50-7.70 (m, 3H), 8.77 (s, 1H), 12.80 (s, 1H) | 466 | 464 | 6 |

[Table 1-8]

| Compound No. | Compound | Salt | NMR | ESI MS $(M+H)^+$ | ESI MS $(M-H)^-$ | Example No. |
|---|---|---|---|---|---|---|
| 50 | 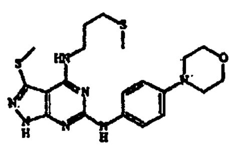 | Free | $^1H$ NMR (300 MHz, DMSO-D6) δ ppm 1.80-1.95 (m, 2H), 2.06 (s, 3H), 2.50 (s, 3H), 2.90-3.15 (m, 4H), 3.22-3.47 (m, 2H), 3.53-3.65 (m, 2H), 3.65-3.85 (m, 4H), 6.53 (s, 1H), 6.87 (d, J=8.7Hz, 2H), 7.64 (d, J=7.8Hz, 2H), 8.85 (s, 1H), 12.81 (s, 1H) | 446 | 444 | 6 |

(continued)

| Compound No. | Compound | Salt | NMR | ESI MS (M+H)+ | ESI MS (M-H)- | Example No. |
|---|---|---|---|---|---|---|
| **51** | 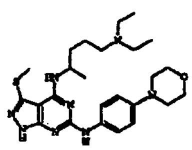 | HCl | $^1$H NMR (300 MHz, DMSO-D6) δ ppm 1.07-1.24 (m, 6H), 1.25-1.37 (m, 3H), 1.60-1.85 (m, 4H), 2.60 (s, 3H), 2.90-3.10 (m, 6H), 3.20-3.40 (m, 4H), 3.75-3.95 (m, 4H), 4.32-4.50 (m, 1H), 6.84 (bs, 1H), 7.20-7.40 (m, 2H), 7.55-7.72 (m, 2H), 10.12 (bs, 1H), 10.43 (bs, 1H) | 499 | 497 | 6 |
| **52** | 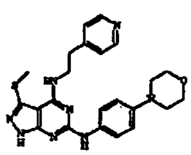 | Free | $^1$H NMR (300 MHz, DMSO-D6) δ ppm 2.85-3.15 (m, 6H), 3.32 (s, 3H), 3.60-3.90 (m, 6H), 6.53 (s, 1H), 6.84 (d, 1=9.0HZ, 2H), 7.30 (d, J=5.4Hz, 2H), 7.61 (d, J=8.7Hz, 2H), 8.49 (d, J=5.4Hz, 2H), 8.86 (s, 1H), 12.80 (s, 1H) | 463 | 461 | 6 |

(continued)

| Compound No. | Compound | Salt | NMR | ESI MS $(M+H)^+$ | ESI MS $(M-H)^-$ | Example No. |
|---|---|---|---|---|---|---|
| **53** | 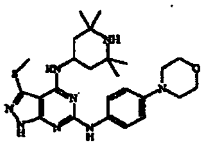 | HCl | $^1$H NMR (300 MHz, DMSO-D6) δ ppm 1.15-1.60 (m, 12H), 1.70-2.15 (m, 4H), 2.57 (s, 3H), 3.05-3.30 (m, 4H), 3.70-3.95 (m, 4H), 4.55-4.74 (m, 1H), 6.95-7.35 (m, 2H), 7.40-7.73 (m, 2H), 8.33 (bs, 1H), 9.30-9.60 (m, 2H) | 497 | 495 | 6 |
| **54** | 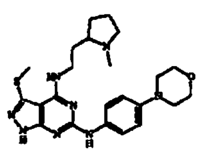 | HCl | $^1$H NMR (300 MHz, DMSO-D6) δ ppm 1.60-2.10 (m, 4H), 2.20-2.40 (m, 2H), 2.65-2.83 (m, 2H), 2.87-3.13 (m, 5H), 3.15-3.45 (m, 6H), 3.46-3.67 (m, 2H), 3.67-3.88 (m, 4H), 6.63 (s, 1H), 6.89 (d, J=8.7Hz, 2H), 7.60 (d, J=9.0Hz, 2H), 8.84 (s, 1H), 10.10-10.45 (m, 1H), 12.92 (s, 1H) | 469 | 467 | 6 |

(continued)

| Compound No. | Compound | Salt | NMR | ESI MS $(M+H)^+$ | ESI MS $(M-H)^-$ | Example No. |
|---|---|---|---|---|---|---|
| **55** | 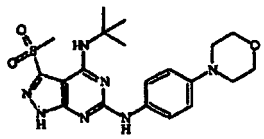 | Free | $^1$H NMR (300 MHz, DMSO-D6) δ ppm 1.49 (s, 9H), 2.95-3.15 (m, 4H), 3.41 (s, 3H), 3.60-3.80 (m, 4H), 6.90 (d, J=9.0Hz, 2H), 7.54 (s, 1H), 7.60 (d, J=9.0Hz, 2H), 9.00 (s, 1H), 13.84 (s, 1H) | 446 | 444 | 1 |
| **56** | 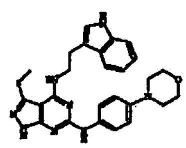 | HCl | $^1$H NMR (300 MHz, DMSO-D6) δ ppm 3.04-3.09 (m, 6H), 3.64-3.85 (m, 9H), 6.95-7.00 (m, 3H), 7.05-7.10 (m, 2H), 7.22 (bs, 1H), 7.34-737 (m, 2H), 7.58-7.61 (m, 3H), 10.90 (bs, 1H) | 501 | 499 | 6 |

[Table 1-9]

| Compound No. | Compound | Salt | NMR | ESI MS $(M+H)^+$ | ESI MS $(M-H)^-$ | Example No. |
|---|---|---|---|---|---|---|
| **57** | 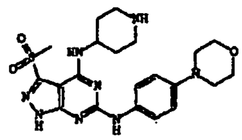 | HCl | $^1$H NMR (300 MHz, DMSO-D6) δ ppm 1.77-1.84 (m, 2H), 2.22-2.28 (m, 2H), 3.03-3.13 (m, 2H), 3.28-3.52 (m, 5H), 3.98-4.29 (m, 9H), 7.43-7.54 (m, 2H), 7.66-7.68 (m, 1H), 7.82-7.87 (m, 2H), 8.92-8.97 (m, 1H), 9.09-9.14 (m, 1H), 9.52-9.54 (m, 1H) | 473 | 471 | 4 |
| **58** | 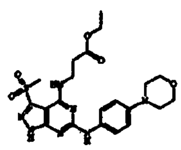 | Free | $^1$H NMR (300 MHz, DMSO-D6) δ ppm 1.19 (t, J=2Hz, 3H), 2.69-2.73 (m, 2H), 3.02-3.05 (m, 4H), 3.39 (s, 3H), 3.72-3.82 (m, 6H), 4.09 (q, J=7.2Hz, 2H), 6.87-6.90 (m, 2H), 7.61-7.64 (m, 3H), 9.14 (bs, 1H) | 490 | 488 | 1 |
| **59** | 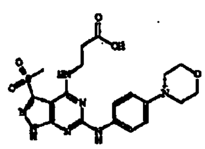 | Free | $^1$H NMR (300 MHz, DMSO-D6) δ ppm 2.61-2.66 (m, 2H), 3.02-3.05 (m, 4H), 3.38 (s, 3H), 3.73-3.76 (m, 6H), 6.87-6.90 (m, 2H), 7.61-7.65 (m, 3H), 9.14 (bs, 1H) | 462 | 460 | 1 |

(continued)

| Compound No. | Compound | Salt | NMR | ESI MS (M+H)+ | ESI MS (M-H)- | Example No. |
|---|---|---|---|---|---|---|
| **60** | 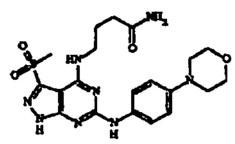 | Free | $^1H$ NMR (300 MHz, DMSO-D6) δ ppm 1.79-1.88 (m, 2H), 2.15-2.20 (m, 2H), 3.02-3.05 (m, 4H), 3.42 (s, 3H), 3.49-3.56 (m, 2H), 3.72-3.75 (m, 4H), 6.77 (bs, 1H), 6.87-6.90 (m, 2H), 7.30 (bs, 1H), 7.48 (bs, 1H), 7.62-7.65 (m, 2H), 9.10 (bs, 1H) | 475 | 473 | 1 |
| **61** | 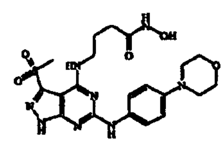 | Free | $^1H$ NMR (300 MHz, DMSO-D6) δ ppm 1.80-1.90 (m, 2H), 2.06-2.11 (m, 2H), 3.03-3.06 (m, 4H), 3.42 (s, 3H), 3.50-3.56 (m, 2H), 3.73-3.76 (m, 4H), 6.88-6.91 (m, 2H), 7.49 (bs, 1H), 7.62-7.65 (m, 2H), 9.10 (bs, 1H), 10.4 (bs, 1H) | 491 | 489 | 1 |
| **62** | 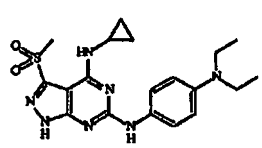 | Free | $^1H$ NMR (600 MHz, CHLOROFORM-D) δ ppm 0.62-0.72 (m, 2H), 0.83-0.94 (m, 2H), 1.15 (t, J=7.1Hz, 6H), 2.96-3.04 (m, 1H), 3.28 (s, 3H), 3.33 (q, J=7.1Hz, 4H), 6.68 (bs, 2H), 6.97 (bs, 1H), 7.45 (bs, 2H), 7.63 (bs, 1H) | 416 | 414 | 1 |

(continued)

| Compound No. | Compound | Salt | NMR | ESI MS $(M+H)^+$ | ESI MS $(M-H)^-$ | Example No. |
|---|---|---|---|---|---|---|
| 63 | 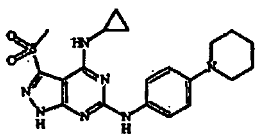 | Free | $^1$H NMR (600 MHz, DMSO-D6) δ ppm 0.53-0.59 (m, 2H), 0.85-0.90 (m, 2H), 1.47-1.54 (m, 2H),1.59-1.65 (m, 4H), 2.99 (bs, 1H), 3.03 (t, J=5.5Hz, 4H), 339 (s, 36), 6,84-6.88 (m, 2H), 7.56 (s, 1H), 7.65-7.71 (m, 2H), 9.12 (s, 1H), 13.86 (bs, 1H) | 428 | 426 | 1 |

[Table 1-10]

| Compound No. | Compound | Salt | NMR | ESI MS $(M+H)^+$ | ESI MS $(M-H)^-$ | Example No. |
|---|---|---|---|---|---|---|
| 64 | 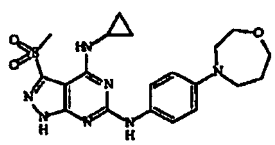 | Free | $^1$H NMR (600 MHz, DMSO-D6) δ ppm 0.53-0.60 (m, 2H), 0.83-0.91 (m, 2H), 1.85-1.94 (m, 2H), 3.00 (m, 1H), 3.39 (s, 3H), 3.49-3.61 (m, 6H), 3.71 (t, J=5.0Hz. 2H), 6.63-6.74 (m, 2H), 7.49-7.68 (m, 3H), 8.99 (s, 1H), 13.81 (bs, 1H) | 444 | 442 | 1 |

(continued)

| Compound No. | Compound | Salt | NMR | ESI MS $(M+H)^+$ | ESI MS $(M-H)^-$ | Example No. |
|---|---|---|---|---|---|---|
| **65** | 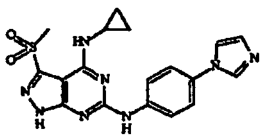 | Free | $^1$H NMR (600 MHz, DMSO-D6) δ ppm 0.50-0.60 (m, 2H), 0.84-0.94 (m, 2H), 3.23-3.38 (m, 1H), 3.32 (s, 3H), 5.61-5.74 (m, 1H), 6.72 (m, 2H), 7.50-7.62 (m, 2H), 8.21-8.27 (m, 1H), 8.55-8.62 (m, 1H), 10.13-1027 (m, 1H) | 411 | 409 | 1 |
| **66** | 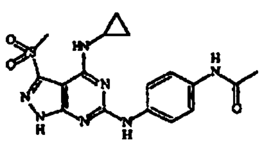 | Free | $^1$H NMR (600 MHz, DMSO-D6) δ ppm 0.56-0.62 (m, 2H), 0.85-0.91 (m, 2H), 2.01 (s, 3H), 2.98-3.04 (m, 1H), 3.41 (s, 3H), 7.44-7.49 (m, 2H), 7.68 (bs, 1H), 7.74-7.79 (m, 2H), 9.41 (bs, 1H), 9.82 (bs, 1H), 13.98 (bs, 1H) | 402 | 400 | 1 |

(continued)

| Compound No. | Compound | Salt | NMR | ESI MS $(M+H)^+$ | ESI MS $(M-H)^-$ | Example No. |
|---|---|---|---|---|---|---|
| **67** | 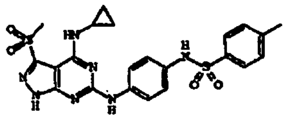 | Free | $^1H$ NMR (600 MHz, DMSO-D6) δ ppm 0.52-0.63 (m, 2H), 0.80-0.92 (m, 2H), 2.33 (s, 3H), 2.94-3.01 (m, 1H), 3.41 (s, 3H), 6.91-7.03 (m, 2H), 7.28-7.40 (m, 2H), 7.57-7.65 (m, 2H), 7.66-7.73 (m, 3H), 9.45 (bs, 1H), 9.93 (bs, 1H) | 514 | 512 | 1 |
| **68** | 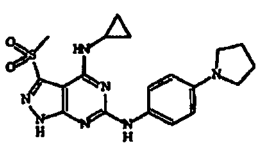 | Free | $^1H$ NMR (600 MHz, DMSO-b6) δ ppm 0.50-0.63 (m, 2H), 0.81-0.94 (m, 2H), 1.87-2.01 (m, 4H), 2.99 (m, 1H), 3.14-3.25 (m, 4H), 3.39 (s, 3H), 6.41-6.59 (m, 2H), 7.42-7.75 (m, 3H), 8.98 (s, 1H), 13.81 (bs, 1H) | 414 | 412 | 1 |

(continued)

| Compound No. | Compound | Salt | NMR | ESI MS (M+H)+ | ESI MS (M-H)- | Example No. |
|---|---|---|---|---|---|---|
| **69** | 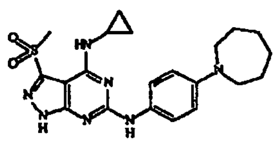 | Free | $^1H$ NMR (600 MHz, DMSO-D6) δ ppm 0.52-0.59 (m, 2H), 0.83-0.91 (m, 2H), 1.43-1.51 (m, 4H), 1.66-1.78 (m, 4H), 3.00 (m, 1H), 3.40 (s, 3H), 3.42 (t, J=6.0Hz, 4H), 6.54-6.66 (m, 2H), 7.46-7.65 (m, 3H), 8.96 (s, 1H), 13.80 (bs, 1H) | 442 | 440 | 1 |
| **70** | 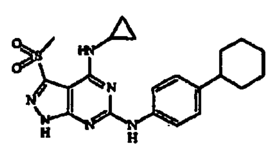 | Free | $^1H$ NMR (600 MHz, DMSO-D6) δ ppm 0.55-0.61 (m, 2H), 0.86-0.91 (m, 2H), 1.17-1.44 (m, 5H), 1.66-1.83 (m, 5H), 2.39-2.46 (m, 1H), 2.98-3.04 (m, 1H), 3.41 (s, 3H), 7.08-7.15 (m, 2H), 7.63 (bs, 1H), 7.75-7.80 (m, 2H), 9.32-9.33 (m, 1H), 13.95 (bs, 1H) | 427 | 425 | 1 |

[Table 1-11]

| Compound No. | Compound | Salt | NMR | ESI MS $(M+H)^+$ | ESI MS $(M-H)^-$ | Example No. |
|---|---|---|---|---|---|---|
| 71 | 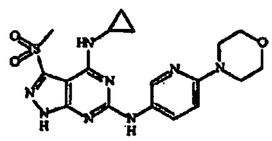 | Free | $^1$H NMR (600 MHz, CHLOROFORM-D) δ ppm 0.63-0.68 (m, 2H), 0.86-0.92 (m, 2H), 2.93-3.01 (m, 1H), 3.30(s, 3H), 3.44-3.52 (m, 4H), 3.79-3.90 (m, 4H), 6.65-6.70 (m, 1H), 7.01 (bs, 1H), 7.71 (bs, 1H), 7.93 (bs, 1H), 8.48 (bs, 1H) | 431 | 429 | 1 |
| 72 | 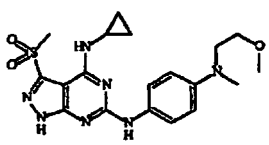 | Free | $^1$H NMR (600 MHz, DMSO-D6) δ ppm 0.53-0.60 (m, 2H), 0.83-0.91 (m, 2H), 2.87 (s, 3H), 3.00 (m, 1H), 3.25 (s, 3H), 3.40 (s, 3H), 3.41-3.52 (m, 4H), 6.61-6.71 (m, 2H), 7.55 (s, 1H), 7.57-7.72 (m, 2H), 9.01 (s, 1H), 13.47 (bs, 1H) | 432 | 430 | 1 |
| 73 | 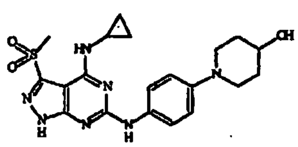 | Free | $^1$H NMR (600 MHz, DMSO-D6) δ ppm 0.52-0.60 (m, 2H), 0.83-0.92 (m, 2H), 1.44-1.54 (m, 2H), 1.76-1.86 (m, 2H), 2.69-2.79 (m, 2H), 2.98 (m, 1H), 3.38-3.48 (m, 2H), 3.39 (s, 3H), 3.58 (m, 1H), 4.65 (d, J=4.6Hz, 1H), 6.87 (d, J=8.7Hz, 2H), 757 (s, 1H), 7.61-7.76 (m, 2H), 9.12 (s, 1H), 13.86 (bs, 1H) | 444 | 442 | 1 |

(continued)

| Compound No. | Compound | Salt | NMR | ESI MS $(M+H)^+$ | ESI MS $(M-H)^-$ | Example No. |
|---|---|---|---|---|---|---|
| **74** | 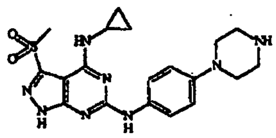 | HCl | $^1$H NMR (600 MHz, DMSO-D6) δ ppm 0.50-0.65 (m, 2H), 0.83-0.94 (m, 2H), 2.95-3.04 (m, 1H), 3.15-3.35 (m, 9H), 3.40 (s, 3H), 6.88-6.99 (m, 2H), 7.60 (bs, 1H) 7.72-7.82 (m, 2H), 9.03 (bs, 1H), 9.23 (bs, 1H) | 429 | 427 | 1 |
| **75** | 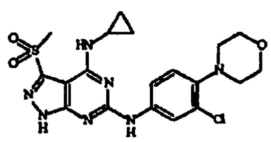 | HCl | $^1$H NMR (600 MHz, DMSO-D6) δ ppm 0.56-0.65 (m, 2 H), 0.90-0.98 (m, 2H), 2.87-2.95 (m, 4H), 2.96-3.02 (m, 1H), 3.42 (s, 3H), 3.69-3.78 (m, 4H), 4.33 (bs, 1H), 7.64-7.72 (m, 2H), 825 (bs, 1H), 9.50 (bs, 1H), 14.01 (bs, 1H). | 464 | 462 | 1 |
| **76** | 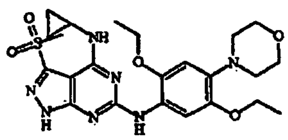 | Free | $^1$H NMR (600 MHz, DMSO-D6) δ ppm 0.52-0.60 (m, 2H), 0.83-0.88 (m, 2H), 1.28-1.37 (m, 6H), 2.95-3.01 (m, 4H), 3.01-3.07 (m, 1H), 3.40 (s, 3H), 3.68-3.77 (m, 4H), 4.00 (q, J=7.0Hz, 2H), 4.09 (q, J=6.9Hz, 2H), 6.60 (s, 1H), 7.63 (bs, 1H), 7.66 (bs, 1H), 8.06(bs, 1H), 13.93 (bs, 1H) | 518 | 516 | 1 |

(continued)

| Compound No. | Compound | Salt | NMR | ESI MS (M+H)+ | ESI MS (M-H)- | Example No. |
|---|---|---|---|---|---|---|
| **77** | 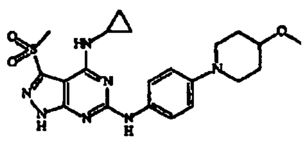 | Free | $^1$H NMR (600 MHz, DMSO-D6) δ ppm 0.51-0.60 (m, 2H), 0.82-0.93 (m, 2H), 1.47-1.58 (m, 2H), 1.89-2.00 (m, 2H), 2.43-2.56 (m, 2H), 2.74-2.85 (m, 2H), 2.98 (m, 1H), 326 (s, 3H), 3.35-3.47 (m, 1H), 3.40 (s, 3H), 6.83-6.93 (m, 2H), 7.57 (s, 1H), 7.62-7.76 (m, 2H), 9.13 (s, 1H), 13.87 (bs, 1H) | 458 | 456 | 1 |

[Table 1-12]

| Compound No. | Compound | Salt | NMR | ESI MS $(M+H)^+$ | ESI MS $(M-H)^-$ | Example No. |
|---|---|---|---|---|---|---|
| 78 | 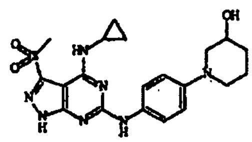 | Free | $^1H$ NMR (600 MHz, DMSO-D6) δ ppm 0.53-0.60 (m, 2H), 0.83-0.92 (m, 2H), 1.22 (m, 1H), 1.54 (m, 1H), 1.73 (m, 1H), 1.87 (m, 1H), 2.41 (dd, J=11.0 and 9.2Hz, 1H), 2.55 (m, 1H), 2.98 (m, 1H), 3.38 (m, 1H), 3.40 (s, 3H), 3.49 (m, 1H), 3.59 (m, 1H), 4.77 (d, J=5.0Hz, 1H), 6.80-6.90 (m, 2H), 7.57 (s, 1H), 7.62-7.75 (m, 2H), 9.13 (s, 1H), 13.87 (bs, 1H) | 444 | 447 | 1 |

(continued)

| Compound No. | Compound | Salt | NMR | ESI MS $(M+H)^+$ | ESI MS $(M-H)^-$ | Example No. |
|---|---|---|---|---|---|---|
| 79 | 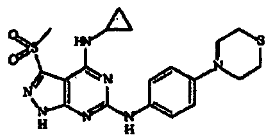 | Free | $^1$H NMR (600 MHz, DMSO-D6) δ ppm 0.52-0.62 (m, 2H), 0.83-0.92 (m, 2H), 2.64-2:74 (m, 4H), 2.97-3.04 (m, 1H), 3.35-3.44 (m, 4H), 3.40 (s, 3H), 6.83-6.91 (m, 2H), 7.57-7.59 (m, 1H), 7.68-7.76 (m, 2H), 9.16 (bs, 1H), 13.88 (bs, 1H) | 446 | 444 | 1 |

(continued)

| Compound No. | Compound | Salt | NMR | ESI MS $(M+H)^+$ | ESI MS $(M-H)^-$ | Example No. |
|---|---|---|---|---|---|---|
| 80 | 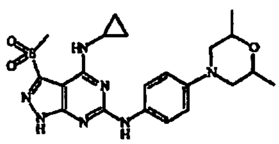 | Free | $^1H$ NMR (600 MHz, DMSO-D6) δppm 0.57-0.59 (m, 2H), 0.84-0.93 (m, 2H), 1.15 (d, J=6.4Hz, 6H), 2.19 (dd, J=11.5, 10.6Hz, 2H), 2.98-3.03 (m, 1H), 3.40 (s, 3H), 3.46-3.51 (m, 2H), 3.63-3.77 (m, 2H), 6.83-6.92 (m, 2H), 7.58-7.60 (m, 1H), 7.67-7.77 (m, 2H), 9.15 (bs, 1H), 13.88 (bs, 1 H) | 458 | 456 | 1 |

(continued)

| Compound No. | Compound | Salt | NMR | ESI MS $(M+H)^+$ | ESI MS $(M-H)^-$ | Example No. |
|---|---|---|---|---|---|---|
| 81 | 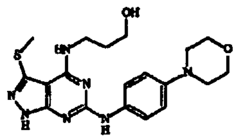 | Free | $^1$H NMR (600 MHz, DMSO-D6) δ ppm 1.72-1.84 (m, 2H), 2.53 (s, 3H), 2.98-3.05 (m, 4H), 3.54-3.58 (m, 2H), 3.60-3.64 (m, 2H), 3.70-3.77 (m, 4H), 4.69 (t, J=5.0Hz, 1H), 6.61(m, 1H), 6.81-0.90 (m, 2H), 7.62-7.68 (m, 2H), 8.84 (s, 1H), 12.80 (bs, 1H) | 416 | 414 | 1 |
| 82 | 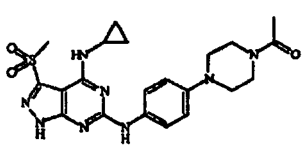 | HCl | $^1$H NMR (600 MHz, DMSO-D6) δ ppm 0.54-0.65 (m, 2H), 0.83-0.95 (m, 2H), 2.06 (s, 3H), 3.06-3.82 (m, 10H), 3.41 (s, 3H), 7.10-7.19 (m, 2H), 7.64 (bs, 1H) 7.79-7.89 (m, 2H), 9.38 (bs, 1H), 13.97 (bs, 1H) | 471 | 469 | 1 |

(continued)

| Compound No. | Compound | Salt | NMR | ESI MS $(M+H)^+$ | ESI MS $(M-H)^-$ | Example No. |
|---|---|---|---|---|---|---|
| 83 | 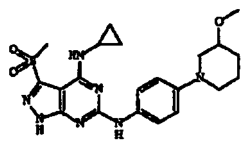 | Free | $^1H$ NMR (600 MHz, DMSO-D6) δ ppm 0.53-0.59 (m, 2H), 0.83-0.92 (m, 2H), 1.27 (m, 1H), 1.55 (m, 1H), 1.77 (m, 1H), 1.98 (m, 1H), 2.55 (m, 1H), 2.66 (m, 1H), 2.99 (m, 1H), 3.28-3.36 (m, 2H), 3.32 (s, 3H), 3.40 (s, 3H), 3.56 (m, 1H), 6.84-6.92 (m, 2H), 7.57 (s, 1H) , 7.64-7.75 (m, 2H), 9.14 (bs, 1H) | 458 | 456 | 1 |

(continued)

| Compound No. | Compound | Salt | NMR | ESI MS $(M+H)^+$ | ESI MS $(M-H)^-$ | Example No. |
|---|---|---|---|---|---|---|
| 84 | 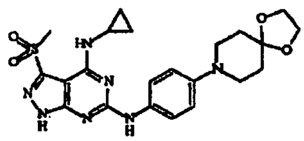 | Free | $^1H$ NMR (600 MHz, DMSO-D6) δ ppm 0.51-0.61 (m, 2H), 0.82-0.92 (m, 2H), 1.67-1.76 (m, 4H), 2.99 (m, 1H), 3.13-3.21 (m, 4H), 3.40 (s, 3H), 3.90 (s, 4H), 6.84-6.95 (m, 2H), 7.57 (s, 1H), 7.63-7.77 (m, 2H), 9.14 (s, 1H), 13.87 (bs, 1H) | 486 | 484 | 1 |

[Table 1-13]

| Compound No. | Compound | Salt | NMR | ESI MS (M+H)+ | ESI MS (M-H)- | Example No. |
|---|---|---|---|---|---|---|
| 85 | 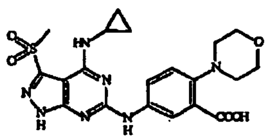 | HCl | $^1$H NMR (600 MHz, DMSO-D6) δ ppm 0.52-0.62 (m, 2H), 0.92-1.00 (m, 2H), 3.04-3.11 (m, 1H), 3.10-3.18 (m, 4H), 3.42 (s, 3H), 3.75-3.90 (m, 4H), 4.99- 5.02 (m, 1H), 7.67-7.75 (m, 2H), 8.13-8.19 (m, 1H), 8.69 (bs, 1H), 9.74 (bs, 1H), 14.08 (bs, 1H) | 474 | 472 | 1 |

(continued)

| Compound No. | Compound | Salt | NMR | ESI MS $(M+H)^+$ | ESI MS $(M-H)^-$ | Example No. |
|---|---|---|---|---|---|---|
| 86 | 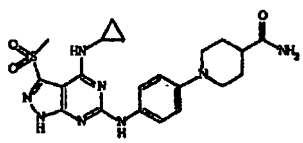 | Free | $^1H$ NMR (600 MHz, DMSO-D6) δ ppm 0.53-0.60 (m, 2H), 0.84-0.91 (m, 2H), 1.65 (dq, J=4.1, 12.4Hz, 2H), 1.73-1.81 (m, 2H), 2.20 (tt, J=4.1, 11.9Hz, 1H), 2.58 (dt, J=2.3, 12.4Hz, 2H), 2.99 (m, 1H), 3.40 (s, 3H), 3.56-3.63 (m, 2H), 6.77 (s, 1H), 6.83-6.92 (m, 2H), 7.27 (s, 1H), 7.57 (s, 1H), 7.70 (m, 2H), 9.13 (s, 1H), 13.87 (bs, 1H) | 471 | 469 | 1 |

(continued)

| Compound No. | Compound | Salt | NMR | ESI MS $(M+H)^+$ | ESI MS $(M-H)^-$ | Example No. |
|---|---|---|---|---|---|---|
| 87 | 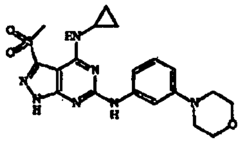 | Free | $^1$H NMR (600 MHz, DMSO-D6) δ ppm 0.54-0.60 (m, 2H), 0.84-0.90 (m, 2H), 3.03 (m, 1H), 3.05-3.10 (m, 4H), 3.41 (s, 3H), 3.71-3.77 (m, 4H), 6.55 (dd, J=8.3, 1.8Hz, 1H), 7.11 (t, J=8.0Hz, 1H), 7.44 (m, 1H), 7.50 (m, 1H), 7.62 (m, 1H), 9.21 (s, 1H), 13.95 (bs, 1H) | 430 | 428 | 1 |

(continued)

| Compound No. | Compound | Salt | NMR | ESI MS $(M+H)^+$ | ESI MS $(M-H)^-$ | Example No. |
|---|---|---|---|---|---|---|
| 88 | 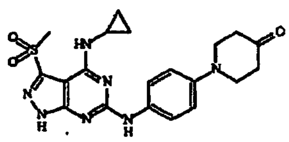 | Free | $^1H$ NMR (600 MHz, DMSO-D6) δ ppm 0.54-0.59 (m, 2H), 0.85-0.91 (m, 2H), 2.42 (t, J=6.0Hz, 4H), 3.00 (m, 1H), 3.40 (s, 3H), 3.51 (t, J=6.0Hz, 4H), 6.94-7.02 (m, 2H), 7.58 (s, 1H), 7.68-7.80 (m, 2H), 9.17 (s, 1H), 13.88 (bs, 1H) | 442 | 440 | 1 |

(continued)

| Compound No. | Compound | Salt | NMR | ESI MS $(M+H)^+$ | ESI MS $(M-H)^-$ | Example No. |
|---|---|---|---|---|---|---|
| 89 | 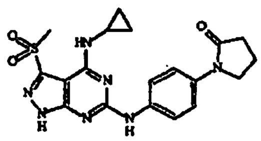 | Free | $^1H$ NMR (600 MHz, DMSO-D6) δ ppm 0.56-0.65 (m, 2H), 0.89-0.95 (m, 2H), 2.02-2.10 (m, 2H), 2.47 (t, J=8.0Hz, 2H), 3.02 (m, 1H), 3.42 (s, 3H), 3.82 (t, J=7.1Hz, 2H), 7.51-7.60 (m, 2H), 7.72 (s, 1H), 7.84-7.91 (m, 2H), 9.54 (s, 1H), 14.05 (bs, 1H) | 428 | 426 | 1 |

(continued)

| Compound No. | Compound | Salt | NMR | ESI MS $(M+H)^+$ | ESI MS $(M-H)^-$ | Example No. |
|---|---|---|---|---|---|---|
| 90 | 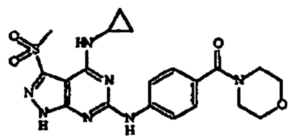 | HCl | $^1$H NMR (600 MHz, DMSO-D6) δ ppm 0.56-0.66 (m, 2H), 0.88-0.99 (m, 2H), 3.00-3.08 (m, 1H), 3.43 (s, 3H), 3.46-3.58 (m, 4H), 3.56-3.68 (m, 4H), 7.32-7.44 (m, 2H), 7.72-7.81 (bs, 1H), 7.95-8.05 (m, 2H), 9.75-9.87 (m, 1H), 14.14 (bs, 1H) | 458 | 456 | 1 |
| 91 | 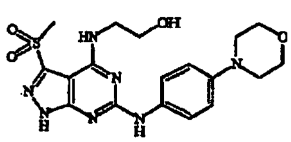 | Free 4H), | $^1$H NMR (600 MHz, DMSO-D6) δ ppm 297-3.09 (m, 4H), 3.39 (s, 3H), 3.56-3.68 (m, 4H), 3.69-3.78 (m, 4.83-4.88 (m, 1H), 6.84-6.92 (m, 2H), 7.59-7.65 (m, 2H), 7.65-7.71 (m, 1H), 9.08 (bs, 1H), 13.86 (bs, 1H) | 434 | 432 | 1 |

[Table 1-14]

| Compound No. | Compound | Salt | NMR | ESI MS $(M+H)^+$ | ESI MS $(M-H)^-$ | Example No. |
|---|---|---|---|---|---|---|
| 92 | 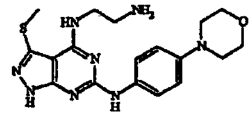 | HCl | $^1H$ NMR (600 MHz, DMSO-D6) δ ppm 2.59 (s, 3H), 3.07-3.15 (m, 2H), 3.27-3.53 (m, 4H), 3.73-3.87 (m, 2H), 3.87-4.13 (m, 4H), 7.39-7.65 (m, 2H) 7.67-7.92 (m, 2H), 8.05-8.30 (m, 4H), 10.03 (bs, 1H) | 401 | 399 | 6 |
| 93 | 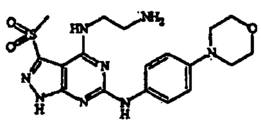 | Free | $^1H$ NMR (600 MHz, DMSO-D6) δ ppm 2.84 (t, J=6.2Hz, 2H), 3.05 (t, J=4.8Hz, 4H), 3.41 (s, 3H), 3.50-3.60 (m, 2H), 3.73-3.77 (m, 4H), 6.85-6.94 (m, 2H), 7.60-7.72 (m, 3H), 9.04 (s, 1H) | 433 | Not detected | 4 |

(continued)

| Compound No. | Compound | Salt | NMR | ESI MS $(M+H)^+$ | ESI MS $(M-H)^-$ | Example No. |
|---|---|---|---|---|---|---|
| 94 | 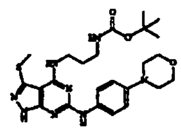 | Free | $^1H$ NMR (600 MHz, DMSO-D6) δ ppm 1.37 (s, 9H), 1.67-1.76 (m, 2H), 2.52 (s, 3H), 2.99-3.06 (m, 6H), 3.52 (q, J=6.4Hz, 2H), 3.71-3.75 (m, 4H), 6.51-6.60 (m, 1H), 6.83-6.88 (m, 2H), 6.90 (t, J=5.7Hz, 1H), 7.58-7.68 (m, 2H), 8.82 (s, 1H), 12.79 (bs, 1H) | 515 | 513 | 6 |
| 95 | 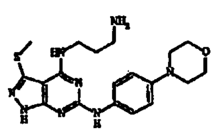 | Free | $^1H$ NMR (600 MHz, DMSO-D6) δ ppm 1.64-1.71 (m, 2H), 2.51 (s, 3H), 2.66 (t, J=6.4Hz, 2H), 2.98-3.05 (m, 4H), 3.56-3.64 (m, 2H), 3.70-3.76 (m, 4H), 6.80-6.89 (m, 3H), 7.60-7.67 (m, 2H), 8.81 (bs, 1H) | 415 | 413 | 6 |

(continued)

| Compound No. | Compound | Salt | NMR | ESI MS $(M+H)^+$ | ESI MS $(M-H)^-$ | Example No. |
|---|---|---|---|---|---|---|
| 96 | 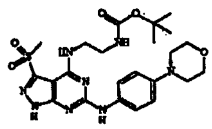 | Free | $^1H$ NMR (600 MHz, DMSO-D6) δ ppm 1.35 (s, 9H), 2.99-3.07 (m, 4H), 3.17-3.24 (m, 2H), 3.38 (s, 3H), 3.56-3.64 (m, 2H), 3.70-3.76 (m, 4H), 6.86-6.91 (m, 2H), 6.97 (t, J=5.7Hz, 1H), 7.53 (m, 1H), 7.58-7.66 (m, 2H), 9.09 (bs, 1H) | 533 | 531 | 4 |
| 97 | 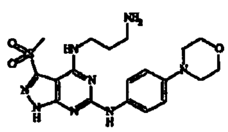 | HCl | $^1H$ NMR (600 MHz, DMSO-D6) δ ppm 1.89-1.99 (m, 2H), 2.83-2.92 (m, 2H), 3.01-3.09 (m, 4H), 3.44 (s, 3H), 3.61-3.66 (m, 2H), 3.71-3.78 (m, 4H), 6.86-6.94 (m, 2H), 7.52 (m, 1H), 7.60-7.69 (m, 2H), 7.92-8.13 (m, 3H), 9.19 (s, 1H), 13.95 (bs, 1H) | 447 | 445 | 4 |

(continued)

| Compound No. | Compound | Salt | NMR | ESI MS $(M+H)^+$ | ESI MS $(M-H)^-$ | Example No. |
|---|---|---|---|---|---|---|
| 98 | 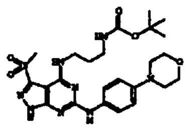 | Free | $^1H$ NMR (600 MHz, DMSO-D6) δ ppm 1.36 (s, 9H), 1.71-1.75(m, 2H), 3.01-3.07 (m, 6H), 3.40 (s, 3H), 3.49-3.57 (m, 2 H), 3.71-3.76 (m, 4H), 6.85-6.93 (m, 3H), 7.46 (m, 1H), 7.60-7.66 (m, 2H), 9.07 (bs, 1H) | 547 | 545 | 4 |

[Table 1-15]

| Compound No. | Compound | Salt | NMR | ESI MS $(M+H)^+$ | ESI MS $(M-H)^-$ | Example No. |
|---|---|---|---|---|---|---|
| 99 | 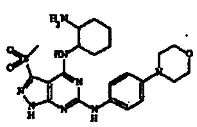 | Free | $^1H$ NMR (600 MHz, DMSO-D6) δ ppm 1.29-1.44 (m, 2H), 1.46-1.68 (m, 5H), 1.76 (m, 1H), 2.99-3.08 (m, 5H), 3.40 (s, 3H), 3.71-3.77 (m, 4H), 4.33 (m, 1H), 6.84-6.90 (m, 2H), 7.60-7.66 (m, 2H), 7.70 (m, 1H), 8.99 (bs, 1H) | 487 | 485 | 4 |

(continued)

| Compound No. | Compound | Salt | NMR | ESI MS $(M+H)^+$ | ESI MS $(M-H)^-$ | Example No. |
|---|---|---|---|---|---|---|
| 100 | 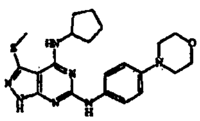 | Free | $^1H$ NMR (600 MHz, DMSO-D6) δ ppm 1.49-1.67 (m, 4H), 1.68-1.79 (m, 2H), 1.99-2.11 (m, 2H), 2.53 (s, 3H), 2.97-3.06 (m, 4H), 3.68-3.78 (m, 4H), 4.50 (m, 1H), 5.97 (d, J=7.3Hz, 1H), 6.81-6.90 (m, 2H), 7.60-7.69 (m, 2H), 8.87 (s, 1H) 12.84 (bs, 1H) | 426 | 424 | 6 |
| 101 | 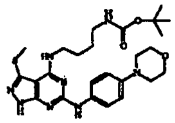 | Free | $^1H$ NMR (600 MHz, DMSO-D6) δ ppm 1.36 (s, 9H), 1.41-1.51 (m, 2H), 1.55-1.65 (m, 2H), 2.52 (s, 3H), 2.92-2.98 (m, 2H), 2.99-3.05 (m, 4H), 3.51 (q, J=6.7Hz, 2H), 3.70-3.77 (m, 4H), 6.41 (m, 1H), 6.82 (t, J=5.5Hz, 1H), 6.83-6.89 (m, 2H), 7.60-7.67 (m, 2H), 8.82 (s, 1H), 12.78 (bs, 1H) | 529 | 527 | 6 |

(continued)

| Compound No. | Compound | Salt | NMR | ESI MS (M+H)+ | ESI MS (M-H)- | Example No. |
|---|---|---|---|---|---|---|
| 102 | 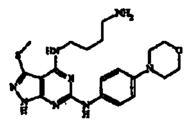 | HCl | $^1$H NMR (600 MHz, DMSO-D6) δ ppm 1.59-1.78 (m, 4H), 2.61 (s, 3H), 2.68-2.92 (m, 2H), 3.20-3.46 (m, 4H), 3.54-3.72 (m, 2H), 3.81-4.06 (m, 4H), 7.22-7.53 (m, 2H), 7.55-7.80 (m, 2H), 7.94-8.21 (m, 3H) | 429 | 427 | 6 |
| 103 | 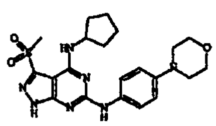 | Free | $^1$H NMR (600 MHz, DMSO-D6) δ ppm 1.47-1.57 (m, 2H), 1.59-1.68 (m, 2H), 1.68-1.78 (m, 2H), 1.98-2.11 (m, 2H), 3.00-3.09 (m, 4H), 3.42 (s, 3H), 3.70-3.78 (m, 4H), 4.47 (m, 1H), 6.84-6.93 (m, 2H), 7.57 (d, J=6.9Hz, 1H), 7.61-7.71 (m, 2H), 9.08 (s, 1H), 13.85 (bs, 1H) | 458 | 456 | 1 |

(continued)

| Compound No. | Compound | Salt | NMR | ESI MS (M+H)+ | ESI MS (M-H)- | Example No. |
|---|---|---|---|---|---|---|
| 104 | 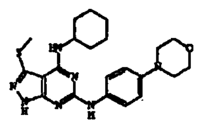 | Free | $^1$H NMR (600 MHz, DMSO-D6) δ ppm 1.27 (m, 1H), 1.34-1.46 (m, 4 H), 1.62 (m, 1H), 1.68-1.78 (m, 2H), 1.94-2.06 (m, 2H), 2.54 (s, 3H), 2.96-3.10 (m, 4H), 3.71-3.77 (m, 4H), 4.12 (m, 1H), 5.92 (d, J=7.8Hz, 1H), 6.82-6.89 (m, 2H), 7.61-7.67 (m, 2H), 8.87 (s, 1H), 12.85 (bs, 1H) | 440 | 438 | 6 |
| 105 | 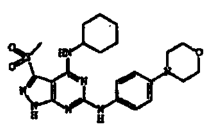 | Free | $^1$H NMR (600 MHz, DMSO-D6) δ ppm 1.24-1.50 (m, 5H), 1.55 (m, 1H), 1.64-1.78 (m, 2H), 1.88-2.06 (m, 2H), 2.95-3.08 (m, 4H), 3.41 (s, 3H), 3.68-3.80 (m, 4H), 4.05-4.27 (m, 1H), 6.79-6.95 (m, 2H), 7.54 (d, J=7.3Hz, 1H), 7.58-7.71 (m, 2H), 9.06 (s, 1H), 13.83 (bs, 1H) | 472 | 470 | 1 |

[Table 1-16]

| Compound No. | Compound | Salt | NMR | ESI MS $(M+H)^+$ | ESI MS $(M-H)^-$ | Example No. |
|---|---|---|---|---|---|---|
| 106 | 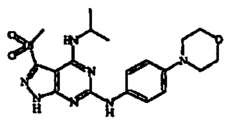 | Free | $^1H$ NMR (600 MHz, DMSO-D6) δ ppm 1.25 (d, J=6.4Hz, 6H), 2.99-3.06 (m, 4H), 3.41 (s, 3H), 3.69-3.77 (m, 4H), 4.35 (m, 1H), 6.80-6.94 (m, 2H), 7.42 (d, J=6.9Hz, 1H), 7.59-7.68 (m, 2H), 9.07 (s, 1H), 13.85 (bs, 1H) | 432 | 430 | 1 |
| 107 | 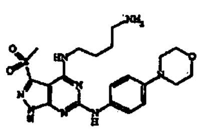 | Free | $^1H$ NMR (600 MHz, DMSO-D6) δ ppm 1.43-1.55 (m, 2H), 1.57-1.71 (m, 2H), 2.58-2.69 (m, 2H), 2.94-3.07 (m, 4H), 3.36 (s, 3H), 3.48-3.59 (m, 2H), 3.67-3.78 (m, 4H), 6.82-6.92 (m, 2H), 7.45 (m, 1H), 7.61-7.72 (m, 2H), 8.89 (s, 1H) | 461 | 459 | 4 |

(continued)

| Compound No. | Compound | Salt | NMR | ESI MS $(M+H)^+$ | ESI MS $(M-H)^-$ | Example No. |
|---|---|---|---|---|---|---|
| 108 | 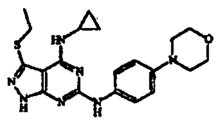 | Free | $^1H$ NMR (600 MHz, DMSO-D6) δ ppm 0.64-0.70 (m, 2H), 0.80-0.88 (m, 2H), 1.24 (t, J=7.3Hz, 3H), 2.93-3.00 (m, 1H), 2.96 (q, J=7.3Hz, 2H), 3.00-3.07 (m, 4H), 3.69-3.77 (m, 4H), 6.42 (bs, 1H), 6.82-6.90 (m, 2H), 7.68-7.80 (m, 2H), 8.94 (s, 1H), 12.92 (s, 1H) | 412 | 410 | 6 |
| 109 | 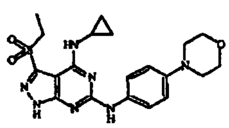 | Free | $^1H$ NMR (600 MHz, DMSO-D6) δ ppm 0.49-0.62 (m, 2H), 0.79-0.93 (m, 2H), 1.18 (t, J=7.3Hz, 3H), 2.95-3.10 (m, 5H), 3.47 (q, J=7.3Hz, 2H), 3.66-3.79 (m, 4H), 6.83-6.92 (m, 2H), 7.63 (bs, 1H), 7.66-7.81 (m, 2H), 9.16 (s, 1H), 13.93 (bs, 1H) | 444 | 442 | 1 |

(continued)

| Compound No. | Compound | Salt | NMR | ESI MS $(M+H)^+$ | ESI MS $(M-H)^-$ | Example No. |
|---|---|---|---|---|---|---|
| 110 | 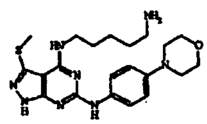 | HCl | $^1$H NMR (600 MHz, DMSO-D6) δ ppm 1.28-1.47 (m, 2H), 1.53-1.74 (m, 4H), 2.61 (s, 3H), 2.69-2.89 (m, 2H), 3.18-3.49 (m, 4H), 3.50-3.67 (m, 2H), 3.80-4.05, (m, 4H), 7.10-7.83 (m, 4H), 7.90-8.12 (m, 3H) | 443 | 441 | 6 |
| 111 | 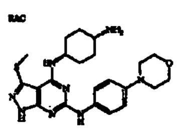 | Free | $^1$H NMR (600 MHz, DMSO-D6) δ ppm 1.06-129 (m, 2H), 1.31-1.51 (m, 2H), 1.69-1.89 (m, 2H), 1.93-2.10 (m, 2H), 2.52 (s, 3H), 2.54-2.67 (m, 1H), 2.95-3.09 (m, 4H), 3.66-3.80 (m, 4H), 3.93-4.11 (m, 1H), 5.83 (d, J=8.3Hz, 1H), 6.78-6.90 (m, 2H), 7.56-7.71 (m, 2H), 8.86 (s, 1H) | 455 | 453 | 6 |

(continued)

| Compound No. | Compound | Salt | NMR | ESI MS (M+H)+ | ESI MS (M-H)- | Example No. |
|---|---|---|---|---|---|---|
| 112 | 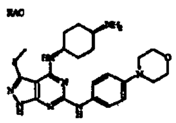 | Free | $^1$H NMR (600 MHz, DMSO-D6) δ ppm 1.29-1.49 (m, 2H), 1.58-1.73 (m, 4H), 1.78-1.97 (m, 2H), 2.56 (s, 3H), 2.76-2.93 (m, 1H), 2.95-3.09 (m, 4H), 3.67-3.84 (m, 4H), 4.15-4.35 (m, 1H), 6.06 (d, J=7.8Hz, 1H), 6.86 (d, J=8.7Hz, 2H), 7.54-7.77 (m, 2H), 8.89 (s, 1H) | 455 | 453 | 6 |

[Table 1-17]

| Compound | Compound | Salt | NMR | ESI MS $(M+H)^+$ | ESI MS $(M-H)^-$ | Example No. |
|---|---|---|---|---|---|---|
| 113 | 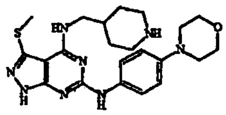 | Free | $^1H$ NMR (600 MHz, DMSO-D6) δ ppm 1.03-1.16 (m, 2H), 1.55-1.68 (m, 2H), 1.69-1.83 (m, 1H), 2.41 (m, 2H), 2.53 (s, 3H), 2.87-2.98 (m, 2H), 2.98-3.06 (m, 4H), 3.42 (t, J=6.4Hz, 2H), 3.69-3.78 (m, 4H), 6.29-6.45 (m, 1H), 6.80-6.90 (m, 2H), 7.59-7.68 (m, 2H), 8.84 (s, 1H) | 455 | 453 | 6 |
| 114 | 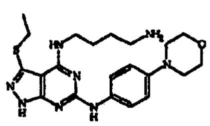 | HCl | $^1H$ NMR (600 MHz, DMSO-D6) δ ppm 1.27 (t, J=7.3Hz, 3H), 1.53-1.78 (m, 4H), 2.70-2.91 (m, 2H), 3.06 (q, J=7.3Hz, 2H), 3.15-3.46 (m, 4H), 3.53-3.70 (m, 2H), 3.76-4.04 (m, 4H), 7.07-7.81 (m, 4H), 7.89-8.15 (m, 3H) | 443 | Not detected | 6 |

(continued)

| Compound | Compound | Salt | NMR | ESI MS $(M+H)^+$ | ESI MS $(M-H)^-$ | Example No. |
|---|---|---|---|---|---|---|
| 115 | 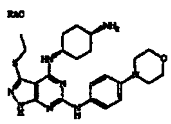 | Free | $^1H$ NMR (600 MHz, DMSO-D6) δ ppm 1.26 (t, J=7.3Hz, 3H), 1.30-1.45 (m, 2H), 1.58-1.75 (m, 4H), 1.78-1.91 (m, 2H), 2.76-2.90 (m, 1H), 2.98 (q, J=7.3Hz, 2H), 2.98-3.09 (m, 4H), 3.66-3.81 (m, 4H), 4.17-4.36 (m, 1H), 6.34 (d, J=7.8Hz, 1H), 6.79-6.96 (m, 2H), 7.55-7.73 (m, 2H), 8.90 (s, 1H) | 469 | 467 | 6 |
| 116 | 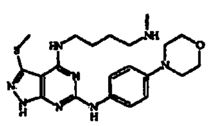 | HCl | $^1H$ NMR (600 MHz, DMSO-D6) δ ppm 1.64-1.74 (m, 4H), 2.49 (s, 3H), 2.61 (s, 3H), 2.80-2.97 (m, 2H), 3.19-3.47 (m, 4H), 3.54-3.68 (m, 2H), 3.79-4.03 (m, 4H), 7.13-7.83 (m, 4H), 8.84-9.07 (m, 3H) | 443 | Not detected | 6 |

(continued)

| Compound | Compound | Salt | NMR | ESI MS $(M+H)^+$ | ESI MS $(M-H)^-$ | Example No. |
|---|---|---|---|---|---|---|
| 117 | 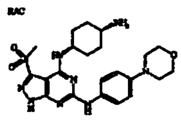 | Free | $^1$H NMR (600 MHz, DMSO-D6) δ ppm 1.33-1.49 (m, 2H), 1.58-1.76 (m, 4H), 1.76-1.99 (m, 2H), 2.79-2.89 (m, 1H), 2.95-3.09 (m, 4H), 3.40 (s, 3H), 3.69-3.79 (m, 4H), 4.19-4.34 (m, 1H), 6.80-6.95 (m, 2H), 7.59-7.72 (m, 3H), 8.95 (s, 1H) | 487 | 485 | 4 |
| 118 | 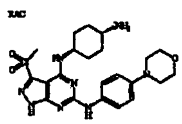 | Free | $^1$H NMR (600 MHz, DMSO-D6) δ ppm 1.14-1.42 (m, 4H), 1.74-1.92 (m, 2H), 1.99-2.17 (m, 2H), 2.64-2.77 (m, 1H), 2.99-3.09 (m, 4H), 3.37 (s, 3H), 3.69-3.80 (m, 4H), 3.88-4.15 (m, 1H), 6.70-6.99 (m, 2H), 7.40 (d, J=7.8Hz, 1H), 7.61-7.73 (m, 2H), 8.92 (bs, 1H) | 487 | 485 | 4 |

(continued)

| Compound | Compound | Salt | NMR | ESI MS $(M+H)^+$ | ESI MS $(M-H)^-$ | Example No. |
|---|---|---|---|---|---|---|
| 119 | 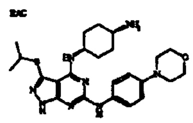 | Free | $^1H$ NMR (600 MHz, DMSO-D6) δ ppm 128 (d, J=6.4Hz, 6H), 1.31-1.45 (m, 2H), 1.59-1.76 (m, 4H), 1.77-1.91 (m, 2H), 2.73-2.91 (m, 1H), 2.95-3.08 (m, 4H), 3.32-3.42 (m, 1H), 3.65-3.78 (m, 4H), 4.18-4.33 (m, 1H), 6.55 (d, J=7.8Hz, 1H), 6.80-6.96 (m, 2H), 7.60-7.70 (m, 2H), 8.90 (s, 1H) | 483 | 481 | 6 |

[Table 1-18]

| Compound | Compound | Salt | NMR | ESI MS $(M+H)^+$ | ESI MS $(M-H)^+$ | Example No. |
|---|---|---|---|---|---|---|
| 120 | 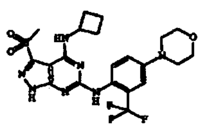 | Free | $^1H$ NMR (600 MHz, CHLOROFORM-D) δ ppm 1.74-1.85 (m, 2H), 1.98-2.06 (m, 2H), 2.38-2.45 (m, 2H), 3.15-3.20 (m, 4H), 3.30 (s, 3H), 3.86-3.89 (m, 4H), 4.57-4.63 (m, 1H), 6.91 (bs, 1H), 7.06-7.10 (m, 1H), 7.13-7.15 (m, 1H), 7.80 (d, J=7.3 Hz, 1H), 7.90 (d, J=8.7 Hz, 1H) | 512 | 510 | 3 |

(continued)

| Compound | Compound | Salt | NMR | ESI MS $(M+H)^+$ | ESI MS $(M-H)^+$ | Example No. |
|---|---|---|---|---|---|---|
| 121 | 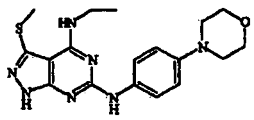 | HCl | $^1H$ NMR (600 MHz, DMSO-D6) δ ppm 123 (t, J=7.1Hz, 3H), 2.61 (s, 3H), 3.28-3.43 (m, 4H), 3.58-3.70 (m, 2H), 3.86-3.98 (m, 4H), 7.35-7.41 (m, 2H), 7.62-7.72 (m, 2H), 10.41 (s, 1H) | 386 | 385 | 6 |
| 122 | 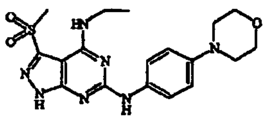 | HCl | $^1H$ NMR (600 MHz, DMSO-D6) δ ppm 1.26 (t, J=7.1Hz, 3H), 3.45 (s, 3H), 3.48-3.52 (m, 4H), 3.57-3.65 (m, 2H), 4.03-4.08 (m, 4H), 5.65-5.72 (m, 1H), 7.62-7.69 (m, 2H), 7.90-7.96 (m, 2H), 9.68 (bs, 1H), 14.13 (bs, 1H) | 418 | 416 | 1 |
| 123 | 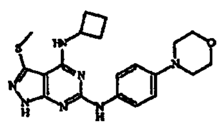 | HCl | $^1H$ NMR (600 MHz, DMSO-D6) δ ppm 1.67-1.79 (m, 2H), 2.18-2.26 (m, 2H), 2.32-2.39 (m, 2H), 2.61 (s, 3H), 3.35 (bs, 4H), 3.47-4.19 (m, 4H), 4.63-4.72 (m, 1H), 7.39 (bs, 2H), 7.71 (bs, 2H) | 412 | 411 | 6 |
| 124 | 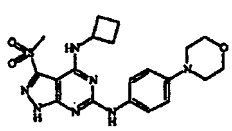 | HCl | $^1H$ NMR (600 MHz, DMSO-D6) δ ppm 1.73-1.85 (m, 2H), 1.92-2.01 (m, 2H), 2.41-2.48 (m, 2H), 3.46 (s, 3H), 3.49 (bs, 4H), 3.73-4.38 (m, 4H), 4.61-4.69 (m, 1H), 7.64 (bs, 1H), 7.84-7.94 (m, 4H), 9.63 (s, 1H), 14.12 (bs, 1H) | 444 | 442 | 1 |

(continued)

| Compound | Compound | Salt | NMR | ESI MS $(M+H)^+$ | ESI MS $(M-H)^+$ | Example No. |
|---|---|---|---|---|---|---|
| 125 | 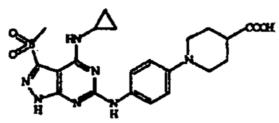 | Free | $^1H$ NMR (600 MHz, DMSO-D6) δ ppm 0.52-0.62 (m, 2H), 0.83-0.93 (m, 2H), 1.59-1.74 (m, 2H), 1.85-1.95 (m, 2H), 2.31-2.41 (m, 1H), 2.63-2.75 (t, J=11.0 Hz, 2H), 2.91-3.10 (m, 1H), 3.40 (s, 3H), 3.47-3.58 (m, 2H), 6.77-6.95 (m, 2H), 7.58 (s, 1H), 7.64-7.78 (m, 2H), 9.16 (s, 1H), 12.22 (s, 1H), 13.89 (s, 1H) | 472 | 470 | 1 |
| 126 | 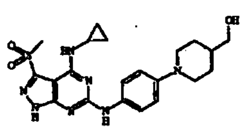 | Free | $^1H$ NMR (600 MHz, DMSO-D6) δ ppm 0.54-0.58 (m, 2H), 0.85-0.90 (m, 2H), 1.19-1.28 (m, 2H), 1.42-1.50 (m, 1H), 1.70-1.76 (m, 2H), 2.52-2.58 (m, 2H), 2.99 (bs, 1H), 3.27-3.30 (m, 2H), 3.40 (s, 3H), 3.56-3.61 (m, 2H), 4.48 (t, J=5.3Hz, 1H), 6.85-6.89 (m, 2H), 7.57 (s, 1H), 7.65-7.71 (m, 2H), 9.14 (s, 1H), 13.88 (bs, 1H) | 458 | 456 | 1 |

[Table 1-19]

| Compound | Compound | Salt | NMR | ESI MS $(M+H)^+$ | ESI MS $(M-H)^-$ | Example No. |
|---|---|---|---|---|---|---|
| 127 | 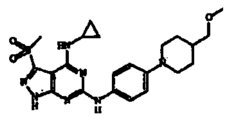 | Free | $^1$H NMR (600 MHz, DMSO-D6) δ ppm 0.54-0.58 (m, 2H), 0.85-0.90 (m, 2H), 1.23-1.33 (m, 2H), 1.60-1.69 (m, 1H), 1.69-1.75 (m, 2H), 2.53-2.59 (m, 2H), 2.99 (bs, 1H), 3.21 (d, J=6.4Hz, 2H), 3.24 (s, 3H), 3.40 (s, 3H), 3.55-3.61 (m, 2H), 6.84-6.89 (m, 2H), 7.57 (s, 1H), 7.66-7.71 (m, 2H), 9.13 (s, 1H), 13.88 (bs, 1H) | 472 | 470 | 1 |

(continued)

| Compound | Compound | Salt | NMR | ESI MS $(M+H)^+$ | ESI MS $(M-H)^-$ | Example No. |
|---|---|---|---|---|---|---|
| 128 | 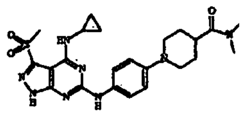 | Free | $^1H$ NMR (600 MHz, DMSO-D6) δ ppm 0.50-0.60 (m, 2H), 0.83-0.92 (m, 2H), 1.59-1.76 (m, 4H), 2.59-2.77 (m, 3H), 2.82 (s, 3H), 2.96-3.02 (m, 1H), 3.04 (s, 3H), 3.39 (s, 3H), 3.56-3.66 (m, 2H), 6.83-6.92 (m, 2H), 7.56 (s, 1H), 7.64-7.75 (m, 2H), 9.11 (s, 1H), 13.88(bs, 1H) | 499 | 497 | 1 |
| 129 | 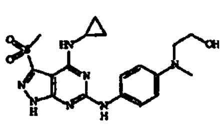 | Free | $^1H$ NMR (600 MHz, DMSO-D6) δ ppm 0.51-0.59 (m, 2H), 0.82-0.90 (m, 2H), 2.88 (s, 3H), 2.94-3.03 (m, 1H), 3.39 (s, 3H), 3.31-3.37 (m, 2H), 3.47-3.60 (m, 2H), 4.61 (t, J=5.5 Hz, 1H), 6.58-6.69 (m, 2H), 7.46-7.70 (m, 3H), 8.99 (s, 1H), 13.82 (bs, 1H) | 418 | 416 | 1 |

(continued)

| Compound | Compound | Salt | NMR | ESI MS $(M+H)^+$ | ESI MS $(M-H)^-$ | Example No. |
|---|---|---|---|---|---|---|
| 130 | 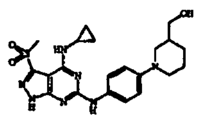 | Free | $^1H$ NMR (600 MHz, DMSO-D6) δ ppm 0.50-0.62 (m, 2H), 0.82-0.91 (m, 2H), 0.97-1.09 (m, 1H), 1.50-1.61 (m, 1H), 1.65-1.77 (m, 3H), 2.28-2.36 (m, 1H), 2.52-2.60 (m, 1H), 2.94-3.04 (m, 1H), 3.25-3.38 (m, 2H), 3.40 (s, 3H), 3.44-3.50 (m, 1H), 3.54-3.61 (m, 1H), 4.52 (t, J=5.27 Hz, 1H), 6.81-6.90 (m, 2H), 7.53-7.61 (m, 1H), 7.64-7.76 (m, 2H), 9.14 (bs, 1H), 13.87 (bs, 1H) | 458 | 456 | 1 |

(continued)

| Compound | Compound | Salt | NMR | ESI MS $(M+H)^+$ | ESI MS $(M-H)^-$ | Example No. |
|---|---|---|---|---|---|---|
| 131 | 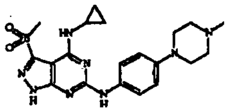 | Free | $^1H$ NMR (600 MHz, DMSO-D6) δ ppm 0.48-0.62 (m, 2H), 0.88 (m, 2H), 2.21 (s, 3H), 2.34-2.48 (m, 4H), 2.96-3.03 (m, 1H), 3.02-3.14 (m, 4H), 3.40 (s, 3H), 6.81-6.95 (m, 2H), 7.57 (s, 1H), 7.63-7.84 (m, 2H), 9.14 (s, 1H), 13.88 (bs, 1H) | 443 | 441 | 3 |

(continued)

| Compound | Compound | Salt | NMR | ESI MS (M+H)+ | ESI MS (M-H)- | Example No. |
|---|---|---|---|---|---|---|
| 132 | 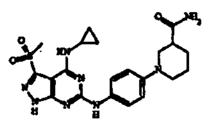 | Free | 1H NMR (600 MHz, DMSO-D6) δ ppm 0.52-0.61 (m, 2H), 0.82-0.94 (m, 2H), 1.40-1.51 (m, 1H), 1.51-1.62 (m, 1H), 1.66-1.76 (m, 1H), 1.78-1.89 (m, 1H) 2.39-2.47 (m, 1H) 2.53-2.61 (m, 1H), 2.62-2.71 (m, 1H), 2.92-3.06 (m, 1H), 3.40 (s, 3H), 3.45-3.54 (m, 1H), 3.55-3.63 (m, 1H), 6.79-6.86 (m, 1H), 6.86-6.92 (m, 2H), 7.29-7.40 (m, 1H), 7.51-7.61 (m, 1H), 7.63-7.76 (m, 2H), 9.14 (bs, 1H), 13.87 (bs, 1H) | 471 | 469 | 1 |

(continued)

| Compound | Compound | Salt | NMR | ESI MS $(M+H)^+$ | ESI MS $(M-H)^-$ | Example No. |
|---|---|---|---|---|---|---|
| 133 | 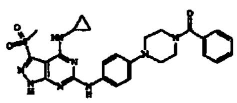 | Free | $^1H$ NMR (600 MHz, DMSO-D6) δ ppm 0.52-0.61 (m, 2H), 0.81-0.92 (m, 2H), 2.94-3.24 (m, 5H), 3.40 (s, 3H), 3.41-3.57 (m, 2H), 3.68-3.87 (m, 2H), 6.87-6.96 (m, 2H), 7.39-7.51 (m, 5H), 7.54-7.63 (m, 1H), 7.69-7.79 (m, 2H), 9.19 (bs, 1H), 13.88 (bs, 1H) | Not detected | 531 | 1 |

[Table 1-20]

| Compound | Compound | Salt | NMR | ESI MS (M+H)+ | ESI MS(M-H)- | Example No. |
|---|---|---|---|---|---|---|
| 134 | 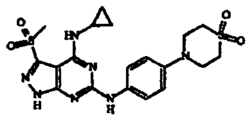 | Free | $^1H$ NMR (600 MHz, DMSO-D6) δ ppm 0.52-0.62 (m, 2H), 0.85-0.93 (m, 2H), 2.94-3.04 (m, 1H), 3.09-3.17 (m, 4H), 3.40 (s, 3H), 3.63-3.73 (m, 4H), 6.92-7.02 (m, 2H), 7.55-7.63 (m, 1H), 7.71-7.82 (m, 2H), 922 (bs, 1H), 13.90 (bs, 1H) | 478 | 476 | 1 |
| 135 | 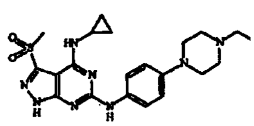 | Free | $^1H$ NMR (600 MHz, DMSO-D6) δ ppm 0.43-0.68 (m, 2H), 0.81-0.95 (m, 2H), 1.03 (t, J=7.1Hz, 3H), 2.36 (q, J=7.1Hz, 2H), 2.44-2.55 (m, 4H), 2.94-3.03 (m, 1H), 3.03-3.10 (m, 4H), 3.40 (s, 3H), 6.82-6.93 (m, 2H), 7.58 (bs, 1H), 7.64-7.78 (m, 2H), 9.14 (s, 1H), 13.88 (bs, 1H) | 457 | 455 | 3 |

(continued)

| Compound | Compound | Salt | NMR | ESI MS $(M+H)^+$ | ESI MS$(M-H)^-$ | Example No. |
|---|---|---|---|---|---|---|
| 136 | 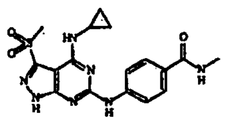 | HCl | $^1$H NMR (600 MHz, DMSO-D6) δ ppm 0.55-0.70 (m, 2H), 0.87-0.98 (m, 2H), 2.76 (d, J=4.1Hz, 3H), 2.97-3.08 (m, 1H), 3.43 (s, 3H), 7.72 (d, J=3.7Hz, 1H), 7.74-7.82 (m, 2H), 7.92-8.07 (m, 2H), 8.20-8.29 (m,1H), 9.72 (s,1H), 14.10 (bs, 1H) | 402 | 400 | 1 |
| 137 | 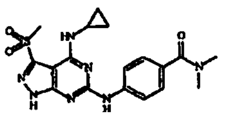 | Free | $^1$H NMR (600 MHz, DMSO-D6) δ ppm 0.56-0.65 (m, 2H), 0.85-0.97 (m, 2H), 2.97 (s, 6H), 3.00-3.07 (m, 1H), 3.42 (s, 3H), 7.33-7.40 (m, 2H), 7.69 (d, J=3.2Hz, 1H), 7.95-8.01 (m, 2H), 9.63 (s, 1H), 14.05 (bs, 1H) | 416 | 414 | 1 |

(continued)

| Compound | Compound | Salt | NMR | ESI MS $(M+H)^+$ | ESI MS$(M-H)^-$ | Example No. |
|---|---|---|---|---|---|---|
| 138 | 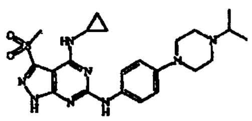 | Free | $^1H$ NMR (600 MHz, DMSO-D6) δ ppm 0.45-0.71 (m, 2H), 0.83-0.95 (m, 2H), 1.02 (d, J=6.4Hz, 6H), 2.55-2.65 (m, 4H), 2.64-2.80 (m, 1H), 2.93-3.14 (m, 5H), 3.42 (s, 3H), 6.80-6.95 (m, 2H), 7.59 (bs, 1H), 7.65-7.82 (m, 2H), 9.15 (s, 1H), 13.89 (bs, 1H) | 471 | 469 | 3 |

(continued)

| Compound | Compound | Salt | NMR | ESI MS (M+H)+ | ESI MS(M-H)- | Example No. |
|---|---|---|---|---|---|---|
| 139 | 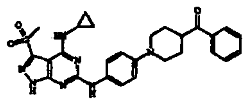 | Free | $^1$H NMR (600 MHz, DMSO-D6) δ ppm 0.49-0.61 (m, 2H), 0.83-0.94 (m, 2H), 1.64-1.77 (m, 2H), 1.83-1.91 (m, 2H), 2.73-2.87 (m, 2H), 2.96-3.05 (m, 1H), 3.40 (s, 3H), 3.52-3.62 (m, 1H), 3.62-3.72 (m, 2H), 6.86-6.96 (m, 2H), 7.49-7.61 (m, 3H), 7.62-7.70 (m, 1H), 7.68-7.77 (m, 2H), 7.98-8.05 (m, 2H), 9.14 (bs, 1H), 13.88 (bs, 1H) | 532 | 530 | 1 |

(continued)

| Compound | Compound | Salt | NMR | ESI MS $(M+H)^+$ | ESI MS$(M-H)^-$ | Example No. |
|---|---|---|---|---|---|---|
| 140 | 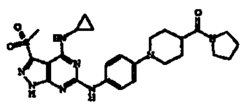 | Free | $^1H$ NMR (600 MHz, DMSO-D6) δ ppm 0.54-0.59 (m, 2H), 0.85-0.91 (m, 2H), 1.63-1.75 (m, 4H), 1.74-1.80 (m, 2H), 1.85-1.91 (m, 2H), 2.50-2.59 (m, 1H), 2.58-2.72 (m, 2H), 2.92-3.07 (m, 1H), 3.23-3.31 (m, 2H), 3.40 (s, 3H), 3.46-3.53 (m, 2H), 3.57-3.68 (m, 2H), 6.86-6.92 (m, 2H), 7.54-7.62 (m, 1H), 7.65-7.74 (m, 2H), 9.15 (bs, 1H), 13.88 (bs, 1H) | 525 | Not detected | 1 |

[Table 1-21]

| Compound | Compound | Salt | NMR | ESI MS $(M+H)^+$ | ESI MS $(M-H)^-$ | Example No. |
|---|---|---|---|---|---|---|
| 141 | 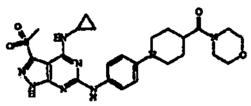 | Free | $^1H$ NMR (600 MHz, DMSO-D6) δ ppm 0.52-0.60 (m, 2H), 0.82-0.93 (m, 2H), 1.63-1.75 (m, 4H), 2.62-2.70 (m, 2H), 2.69-2.77 (m, 1H), 2.95-3.04 (m, 1H), 3.40 (s, 3H) 3.41-3.49 (m, 2H), 3.50-3.65 (m, 8H), 6.84-6.91 (m, 2H), 7.52-7.60 (m, 1H), 7.64-7.77 (m, 2H), 9.13 (bs, 1H), 13.87 (bs, 1H) | 471 | 539 | 1 |

(continued)

| Compound | Compound | Salt | NMR | ESI MS $(M+H)^+$ | ESI MS$(M-H)^-$ | Example No. |
|---|---|---|---|---|---|---|
| 142 | 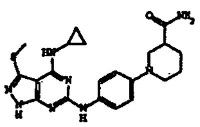 | Free | $^1H$ NMR (600 MHz, DMSO-D6) δ ppm 0.62-0.70 (m, 2H), 0.78-0.86 (m, 2H), 1.41-1.51 (m, 1H), 1.52-1.62 (m, 1H), 1.67-1.76 (m, 1H), 1.79-1.87 (m, 1H), 2.38-2.47 (m, 1H), 2.50 (s, 3H), 2.52-2.60 (m, 1H), 2.61-2.69 (m, 1H), 2.91-2.99 (m, 1H), 3.43-3.53 (m, 1H), 3.53-3.60 (m, 1H), 6.28-6.36 (m, 1H), 6.79-6.90 (m, 3H), 7.35 (bs, 1H), 7.67-7.77 (m, 2H), 8.89 (bs, 1H), 12.82 (bs, 1H) | 439 | 437 | 6 |

(continued)

| Compound | Compound | Salt | NMR | ESI MS (M+H)+ | ESI MS(M-H)- | Example No. |
|---|---|---|---|---|---|---|
| 143 | 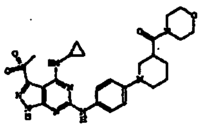 | Free | $^1$H NMR (600 MHz, DMSO-D6) δ ppm 0.53-0.59 (m, 2H), 0.82-0.91 (m, 2H), 1.38-1.49 (m, 1H), 1.61-1.75 (m, 2H), 1.76-1.85 (m, 1H), 2.52-2.61 (m, 1H), 2.65-2.72 (m, 1H), 2.83-2.91 (m, 1H), 2.96-3.04 (m, 1H), 3.35-3.44 (m, 2H), 3.40 (s, 3H), 3.45-3.65 (m, 8H), 6.84-6.91 (m, 2H), 7.54-7.62 (m, 1H), 7.65-7.75 (m, 2H), 9.16 (bs, 1H) | 541 | 539 | 1 |

(continued)

| Compound | Compound | Salt | NMR | ESI MS $(M+H)^+$ | ESI MS $(M-H)^-$ | Example No. |
|---|---|---|---|---|---|---|
| 144 | 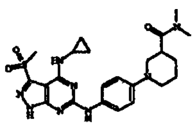 | Free | $^1H$ NMR (600 MHz, DMSO-D6) δ ppm 0.52-0.59 (m, 2H), 0.82-0.91 (m, 2H), 1.39-1.49 (m, 1H), 1.59-1.75 (m, 2H), 1.77-1.85 (m, 1H), 2.53-2.60 (m, 1H), 2.62-2.69 (m, 1H), 2.82 (s, 3H), 2.83-2.91 (m, 1H), 2.95-3.03 (m, 1H), 3.04 (s, 3H), 3.40 (s, 3H), 3.52-3.62 (m, 2H), 6.84-6.91 (m, 2H), 7.54-7.61 (m, 1H), 7.65-7.75 (m, 2H), 9.15 (bs, 1H) 13.88 (bs, 1H) | 499 | 467 | 1 |

(continued)

| Compound | Compound | Salt | NMR | ESI MS $(M+H)^+$ | ESI MS$(M-H)^-$ | Example No. |
|---|---|---|---|---|---|---|
| 145 | 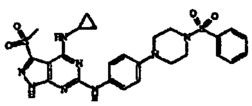 | Free | $^1$H NMR (600 MHz, DMSO-D6) δ ppm 0.51-0.60 (m, 2H), 0.80-0.92 (m, 2H), 2.93-3.05 (m, 5H), 3.07-3.18 (m, 4H), 3.39 (s, 3H), 6.80-6.89 (m, 2H), 7.53-7.63 (m, 1H), 7.63-7.82 (m, 7H), 9.18 (bs, 1H), 13.89 (bs, 1H) | 569 | 567 | 1 |
| 146 | 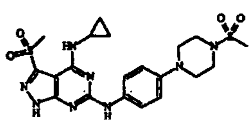 | Free | $^1$H NMR (600 MHz, DMSO-D6) δ ppm 0.54-0.59 (m, 2H), 0.85-0.92 (m, 2H), 2.93 (s, 3H), 2.96-3.03 (m, 1H), 3.13-3.19 (m, 4H), 3.21-3.27 (m, 4H), 3.40 (s, 3H), 6.90-6.95 (m, 2H), 7.56-7.62 (m, 1H), 7.71-7.79 (m, 2H), 9.20 (bs, 1H) 13.90 (bs, 1H) | 507 | 505 | 1 |

(continued)

| Compound | Compound | Salt | NMR | ESI MS (M+H)+ | ESI MS(M-H)- | Example No. |
|---|---|---|---|---|---|---|
| 147 | 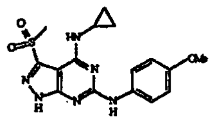 | Free | $^1$H NMR (600 MHz, DMSO-D6) δ ppm 0.54-0.59 (m, 2H), 0.85-0.91 (m, 2H), 2.96-3.03 (m, 1H), 3.40 (s, 3H), 3.72 (s, 3H,) 6.84-6.89 (m, 2H), 7.56-7.62 (m, 1H), 7.73-7.81 (m, 2H), 9.21 (bs, 1H), 13.90 (bs, 1H) | 375 | 373 | 1 |

[Table 1-22]

| Compound No. | Compound | Salt | NMR | ESI MS $(M+H)^+$ | ESI MS $(M-H)^-$ | Example No. |
|---|---|---|---|---|---|---|
| 148 | 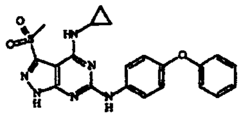 | Free | $^1$H NMR (600 MHz, DMSO-D6) δ ppm 0.54-0.59 (m, 2H), 0.85-0.91 (m, 2H), 2.98-3.04 (m, 1H), 3.41 (s, 3H), 6.93-6.98 (m, 2H), 6.97-7.02 (m, 2H), 7.04-7.09 (m, 1H), 7.33-7.38 (m, 2H), 7.61-7.66 (m, 1H), 7.89-7.95 (m, 2H), 9.46 (bs, 1H), 13.97 (bs, 1H) | 437 | 435 | 1 |
| 149 | 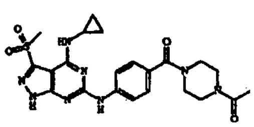 | Free | $^1$H NMR (600 MHz, DMSO-D6) δ ppm 0.56-0.63 (m, 2H), 0.88-0.95 (m, 2H), 2.03 (s, 3H), 3.00-3.09 (m, 1H), 3.43 (s, 3H), 3.44-3.54 (m, 8H), 7.34-7.41 (m, 2H), 7.67-7.72 (m, 1H), 7.98-8.04 (m, 2H), 9.67 (bs, 1H) | 499 | 497 | 1 |

(continued)

| Compound No. | Compound | Salt | NMR | ESI MS $(M+H)^+$ | ESI MS $(M-H)^-$ | Example No. |
|---|---|---|---|---|---|---|
| 150 | 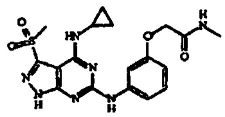 | Free | $^1H$ NMR (600 MHz, DMSO-D6) δ ppm 0.56-0.61 (m, 2H), 0.87-0.94 (m, 2H), 2.66 (d, J=4.59 Hz, 3H), 3.02-3.09 (m, 1H), 3.43 (s, 3H), 4.43 (s, 2H), 6.50-6.55 (m, 1H), 7.16-7.21 (m, 1H), 7.47-7.54 (m, 1H), 7.62-7.72 (m, 2H), 7.96-8.05 (m, 1H), 9.45 (bs, 1H), 14.05 (bs, 1H) | 432 | 430 | 1 |
| 151 | 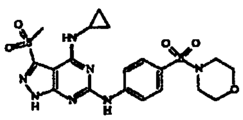 | Free | $^1H$ NMR (600 MHz, DMSO-D6) δ ppm 0.57-0.66 (m, 2H), 0.91-0.99 (m, 2H), 2.80-2.90 (m, 4H), 3.01-3.10 (m, 1H), 3.45 (s, 3H), 3.61-3.68 (m, 4H,) 7.61-7.70 (m, 2H), 7.75-7.82 (m, 1H), 8.18-8.26 (m, 2H), 10.01 (bs, 1H), 14.14 (bs, 1H) | 494 | 492 | 3 |

(continued)

| Compound No. | Compound | Salt | NMR | ESI MS $(M+H)^+$ | ESI MS $(M-H)^-$ | Example No. |
|---|---|---|---|---|---|---|
| 152 | 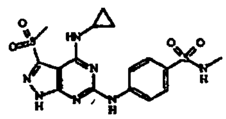 | Free | $^1H$ NMR (600 MHz, DMSO-D6) δ ppm 0.58-0.63 (m, 2H), 0.90-0.96 (m, 2H), 239 (d, J=5.0 Hz, 3H), 3.01-3.07 (m, 1H), 3.43 (s, 3H), 7.18-7.23 (m, 1H), 7.65-7.70 (m, 2H), 7.72-7.77 (m, 1H), 8.10-8.16 (m, 2H), 9.89 (bs, 1H), 14.12 (bs, 1H) | 438 | 436 | 1 |

(continued)

| Compound No. | Compound | Salt | NMR | ESI MS $(M+H)^+$ | ESI MS $(M-H)^-$ | Example No. |
|---|---|---|---|---|---|---|
| 153 | 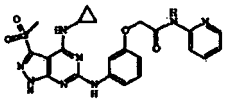 | Free | $^1H$ NMR (600 MHz, DMSO-D6) δ ppm 0.56-0.60 (m, 2H), 0.88-0.93 (m, 2H), 3.01-3.06 (m, 1H), 3.41 (s, 3H), 4.76 (s, 2H), 6.51-6.55 (m, 1H), 7.12-7.15 (m, 1H), 7.18 (t, J=8.3Hz, 1H), 7.45-7.49 (m, 1H), 7.64-7.67 (m, 1H), 7.74-7.77 (m, 1H), 7.79-7.83 (m, 1H), 8.04-8.09 (m, 1H), 8.33-8.35 (m, 1H), 9.44 (bs, 1H), 10.44 (bs, 1H), 14.04 (bs, 1H) | 495 | 493 | 1 |
| 154 | 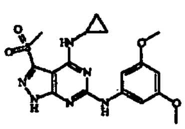 | Free | $^1H$ NMR (600 MHz, DMSO-D6) δ ppm 0.52-0.63 (m, 2H), 0.78-1.00 (m, 2 H), 2.90-3.08 (m, 1H), 3.40 (s, 3H), 3.72 (s, 6H), 6.07-6.16 (m, 1H), 7.16-7.29 (m, 2 H), 7.64 (s, 1H), 9.29 (s, 1H), 14.00 (s, 1H) | 405 | 403 | 1 |

[Table 1-23]

| Compound No. | Compound | Salt | NMR | ESI MS $(M+H)^+$ | ESI MS $(M-H)^-$ | Example No. |
|---|---|---|---|---|---|---|
| 155 | 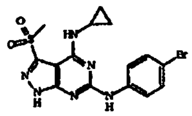 | Free | $^1H$ NMR (600 MHz, DMSO-D6) δ ppm 0.56-0.60 (m, 2H), 0.88-0.93 (m, 2H), 2.98-3.04 (m, 1H), 3.42 (s, 3H), 7.43-7.47 (m, 2H), 7.68 (d, J=3.2 Hz, 1H), 7.89-7.93 (m, 2H), 9.57 (s, 1H), 14.04 (bs, 1H) | 423 | 421 | 1 |
| 156 | 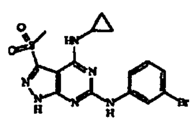 | Free | $^1H$ NMR (600 MHz, DMSO-D6) δ ppm 0.58-0.63 (m, 2H), 0.93-0.98 (m, 2H), 2.97-3.03 (m, 1H), 3.42 (s, 3H), 7.08-7.12 (m, 1H), 7.22 (t, J=8.0 Hz, 1H), 7.68-7.75 (m, 2H), 8.48 (s, 1H), 9.62 (s, 1H), 14.06 (bs, 1H) | 423 | 421 | 1 |

(continued)

| Compound No. | Compound | Salt | NMR | ESI MS $(M+H)^+$ | ESI MS $(M-H)^-$ | Example No. |
|---|---|---|---|---|---|---|
| 157 | 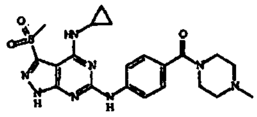 | Free | $^1H$ NMR (600 MHz, DMSO-D6) δ ppm 0.53-0.65 (m, 2H), 0.84-0.96 (m, 2H), 2.19 (s, 3H), 2.25-2.36 (m, 4H), 2.96-3.10 (m, 1H), 3.38-3.61 (m, 4H), 3.42 (s, 3H), 7.28-7.41 (m, 2H), 7.69 (d, J=3.7Hz, 1H), 7.94-8.06 (m, 2H), 9.64 (s, 1H), 14.04 (bs, 1H) | 471 | 469 | 3 |

(continued)

| Compound No. | Compound | Salt | NMR | ESI MS $(M+H)^+$ | ESI MS $(M-H)^-$ | Example No. |
|---|---|---|---|---|---|---|
| 158 | 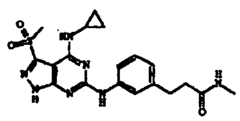 | Free | $^1H$ NMR (600 MHz, DMSO-D6) δ ppm 0.54-0.64 (m, 2H), 0.84-0.96 (m, 2H), 2.35 (t, J=8.0Hz, 2H), 2.56 (d, J=4.6Hz, 3H), 2.77 (t, J=8.0Hz, 2H), 3.00-3.10 (m, 1H), 3.42 (s, 3H), 6.78 (d, J=7.8Hz, 1H), 7.17 (t, J=7.8Hz, 1H), 7.64 (d, J=3.2Hz, 1H), 7.68-7.73 (m, 1H), 7.73-7.82 (m, 2H), 9.33 (s, 1H), 13.96 (bs, 1H) | 430 | 428 | 1 |

(continued)

| Compound No. | Compound | Salt | NMR | ESI MS $(M+H)^+$ | ESI MS $(M-H)^-$ | Example No. |
|---|---|---|---|---|---|---|
| 159 | 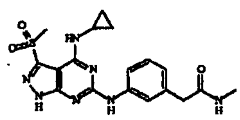 | Free | $^1H$ NMR (600 MHz, DMSO-D6) δ ppm 0.53-0.66 (m, 2H), 0.87-0.93 (m, 2H), 2.57 (d, J=4.6Hz, 3H), 2.99-3.09 (m, 1H), 3.35 (s, 2H), 3.41 (s, 3H), 6.83 (d, J=7.8Hz, 1H), 7.19 (t, J=7.8Hz, 1H), 7.64 (d, J=3.2Hz, 1H), 7.72-7.82 (m, 2H), 7.83-7.94 (m, 1H), 9.36 (s, 1H), 13.95 (bs, 1H) | 416 | 414 | 1 |

(continued)

| Compound No. | Compound | Salt | NMR | ESI MS (M+H)+ | ESI MS (M-H)- | Example No. |
|---|---|---|---|---|---|---|
| 160 | 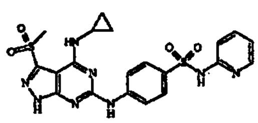 | Free | 1H NMR (600 MHz, DMSO-D6) d ppm 0.55-0.65 (m, 2H), 0.88-0.95 (m, 2H), 2.99-3.07 (m, 1H), 3.30-3.57 (m, 1H), 3.42 (s, 3H), 6.84-6.97 (m, 1H), 7.09-7.17 (m, 1H), 7.66-7.72 (m, 1H), 7.72-7.76 (m, 1H), 7.76-7.81 (m, 2H), 8.02-8.11 (m, 3H), 9.86 (bs, 1H), 14.10 (bs, 1H) | 501 | 499 | 1 |
| 161 | 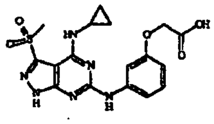 | Free | 1H NMR (600 MHz, DMSO-D6) δ ppm 0.55-0.61 (m, 2H), 0.88-0.95 (m, 2H), 2.99-3.06 (m, 1H), 3.42 (s, 3H), 4.63 (s, 2H), 6.48 (dd, J=8.1, 2.1Hz, 1H), 7.16 (t, J=8.1Hz, 1H), 7.47 (dd, J=8.1, 1.8Hz, 1H), 7.62-7.73 (m, 2H), 9.42 (s, 1H), 12.91-13.02 (m, 1H), 14.04 (s, 1H) | 419 | 417 | 1 |

[Table 1-24]

| Compound No. | Compound | Salt | NMR | ESI MS $(M+H)^+$ | ESI MS $(M-H)^-$ | Example No. |
|---|---|---|---|---|---|---|
| 162 | 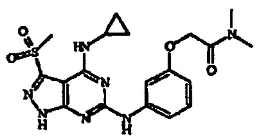 | Free | $^1H$ NMR (600 MHz, DMSO-D6) δ ppm 0.55-0.63 (m, 2H), 0.88-0.95 (m, 2H), 2.85 (s, 3H), 3.00 (s, 3H), 3.01-3.06 (m, 1H), 3.42 (s, 3H), 4.74 (s, 2H), 6.50 (dd, J=8.1, 2.1Hz, 1H), 7.15 (t, J=8.1Hz, 1H), 7.42-7.49 (m, 1H), 7.62-7.68 (m, 2H), 9.38 (s, 1H), 14.02 - 14.09 (m, 1H) | 446 | 444 | 1 |
| 163 | 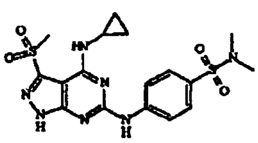 | Free | $^1H$ NMR (600 MHz, DMSO-D6) δ ppm 0.58-0.62 (m, 2H), 0.92-0.97 (m, 2H), 2.59 (s, 6H), 3.03-3.08 (m, 1H), 3.44 (s, 3H), 7.63-7.68 (m, 2H), 7.76 (d, J=3.7Hz, 1H), 8.17-8.22 (m, 2H), 9.97 (s, 1H), 14.15 (bs, 1H) | 452 | 450 | 1 |

(continued)

| Compound No. | Compound | Salt | NMR | ESI MS $(M+H)^+$ | ESI MS $(M-H)^-$ | Example No. |
|---|---|---|---|---|---|---|
| 164 | 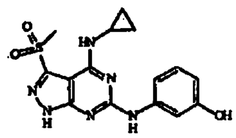 | Free | $^1$H NMR (600 MHz, DMSO-D6) δ ppm 0.55-0.60 (m, 2H), 0.87-0.92 (m, 2H), 3.01-3.06 (m, 1H), 3.41 (s, 3H), 6.35-6.39 (m, 1H), 7.03 (t, J=8.0 Hz, 1H), 7.30-7.34 (m, 1H), 7.41 (s, 1H), 7.62 (d, J=3.2Hz, 1H), 9.18 (s, 1H), 9.27 (s, 1H), 13.96 (bs, 1 H) | 361 | 359 | 1 |

[Table 1-25]

| Compound No. | Compound | Salt | ESI MS $(M+H)^+$ | ESI MS $(M-H)^-$ | Example No. |
|---|---|---|---|---|---|
| 165 | 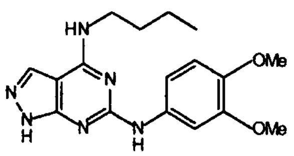 | Free | 343 | 341 | 8 |
| 166 | 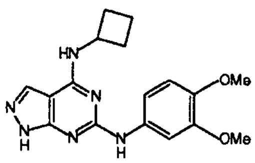 | Free | 341 | 339 | 8 |
| 167 | 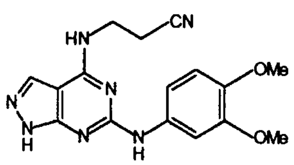 | Free | 340 | 338 | 8 |
| 168 | 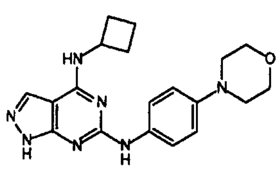 | Free | 366 | 364 | 8 |

(continued)

| Compound No. | Compound | Salt | ESI MS (M+H)+ | ESI MS (M-H)- | Example No. |
|---|---|---|---|---|---|
| 169 | 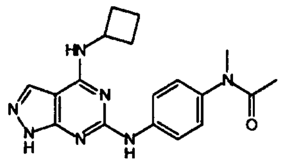 | Free | 352 | 350 | 8 |
| 170 | 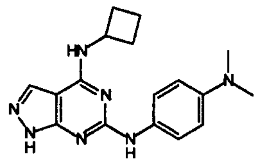 | Free | 324 | 322 | 8 |
| 171 | 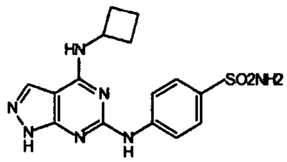 | Free | 360 | 358 | 8 |
| 172 | 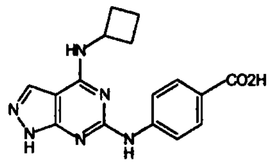 | Free | 325 | Not detected | 8 |
| 173 | 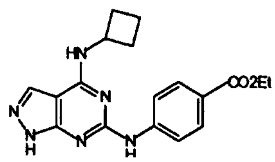 | Free | 353 | 351 | 8 |

[Table 1-26]

| Compound No. | Compound | Salt | ESI MS (M+H)+ | ESI MS (M-H)- | Example No. |
|---|---|---|---|---|---|
| 174 | 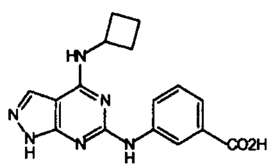 | Free | 325 | 323 | 8 |
| 175 | 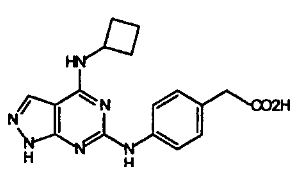 | Free | 339 | Not detected | 8 |

(continued)

| Compound No. | Compound | Salt | ESI MS (M+H)+ | ESI MS (M-H)- | Example No. |
|---|---|---|---|---|---|
| 176 | 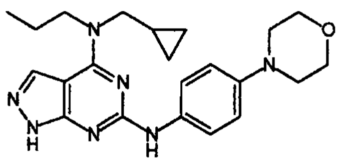 | Free | 408 | 406 | 8 |
| 177 | 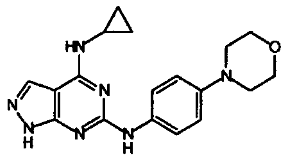 | Free | 352 | 350 | 8 |
| 178 | 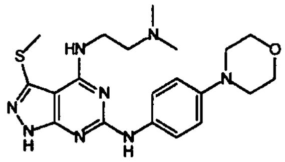 | Free | 430 | Not detected | 6 |
| 179 | 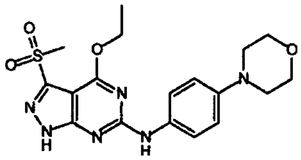 | Free | 419 | 417 | 10 |
| 180 | 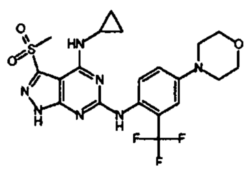 | Free | 498 | 496 | 1 |
| 181 | 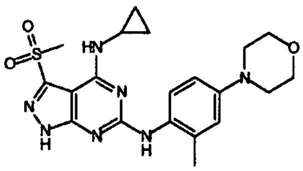 | HCl | 444 | 442 | 1 |
| 182 | 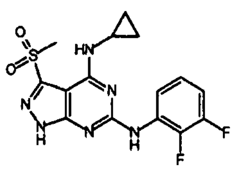 | HCl | 381 | 379 | 1 |

[Table 1-27]

| Compound No. | Compound | Salt | ESI MS (M+H)+ | ESI MS (M-H)- | Example No. |
|---|---|---|---|---|---|
| 183 | 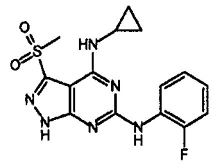 | HCl | 363 | 361 | 1 |
| 184 | 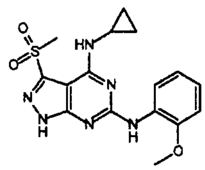 | HCl | 375 | 373 | 1 |
| 185 | 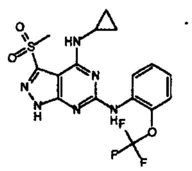 | Free | 429 | 427 | 1 |
| 186 | 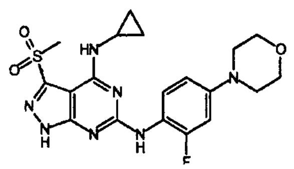 | HCl | 448 | 446 | 1 |
| 187 | 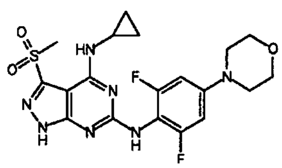 | HCl | 466 | 464 | 1 |
| 188 | 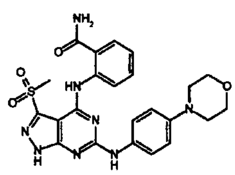 | Free | 509 | 507 | 13 |
| 189 | 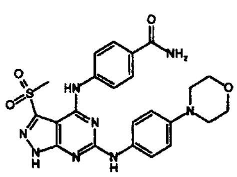 | Free | 509 | 507 | 13 |
| 190 | 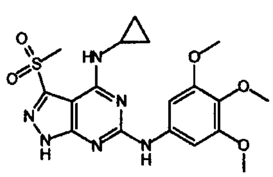 | HCl | 435 | 433 | 1 |

(continued)

| Compound No. | Compound | Salt | ESI MS (M+H)+ | ESI MS (M-H)- | Example No. |
|---|---|---|---|---|---|
| 191 | 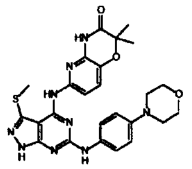 | Free | 534 | 532 | 12 |

[Table 1-28]

| Compound No. | Compound | Salt | ESI MS (M+H)+ | ESI MS (M-H)- | Example No. |
|---|---|---|---|---|---|
| 192 | 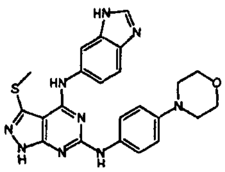 | HCl | 474 | 472 | 12 |
| 193 | 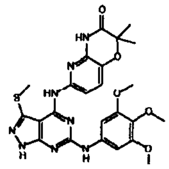 | HCl | 539 | 537 | 12 |
| 194 | 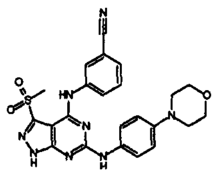 | HCl | 491 | 489 | 13 |
| 195 | 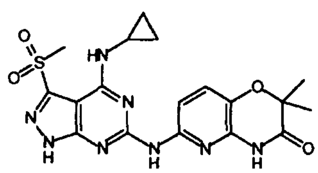 | Free | 445 | 443 | 1 |
| 196 | 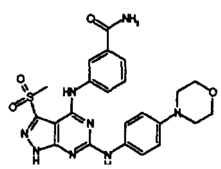 | Free | 509 | 507 | 13 |
| 197 | 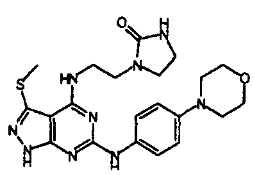 | HCl | 470 | 468 | 6 |

(continued)

| Compound No. | Compound | Salt | ESI MS (M+H)+ | ESI MS (M-H)- | Example No. |
|---|---|---|---|---|---|
| 198 | 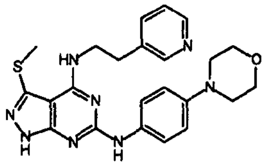 | Free | 463 | 461 | 6 |
| 199 | 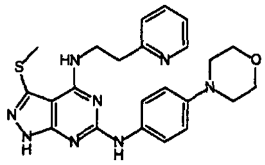 | Free | 463 | 461 | 6 |
| 200 | 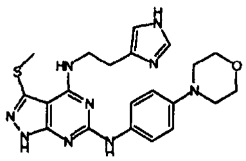 | Free | 452 | 450 | 6 |

[Table 1-29]

| Compound No. | Compound | Salt | ESI MS (M+H)+ | ESI MS (M-H)- | Example No. |
|---|---|---|---|---|---|
| 201 | 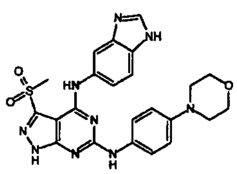 | HCl | 506 | 504 | 13 |
| 202 | 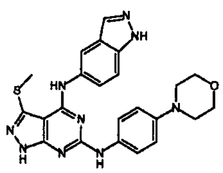 | Free | 474 | 472 | 12 |
| 203 | 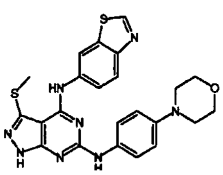 | HCl | 491 | 489 | 12 |
| 204 | 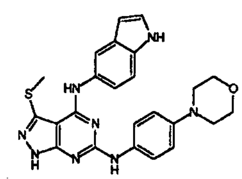 | HCl | 473 | 471 | 12 |

(continued)

| Compound No. | Compound | Salt | ESI MS (M+H)+ | ESI MS (M-H)- | Example No. |
|---|---|---|---|---|---|
| 205 | 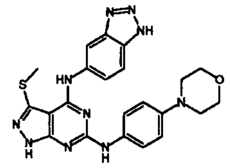 | Free | 475 | 473 | 12 |
| 206 | 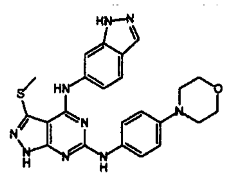 | Free | 474 | 472 | 12 |
| 207 | 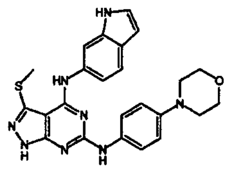 | Free | 473 | 471 | 12 |
| 208 | 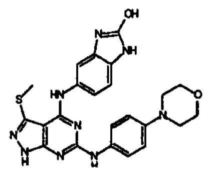 | Free | 490 | 488 | 12 |
| 209 | 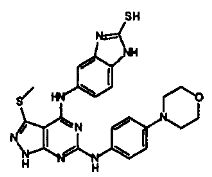 | Free | 506 | 504 | 12 |

[Table 1-30]

| Compound No. | Compound | Salt | ESI MS (M+H)+ | ESI MS (M-H)- | Example No. |
|---|---|---|---|---|---|
| 210 | 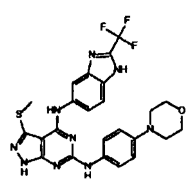 | HCl | 542 | 540 | 12 |
| 211 | 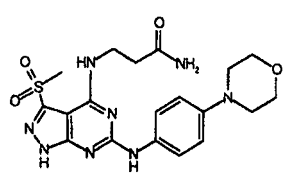 | Free | 461 | 459 | 1 |

(continued)

| Compound No. | Compound | Salt | ESI MS (M+H)+ | ESI MS (M-H)- | Example No. |
|---|---|---|---|---|---|
| 212 | 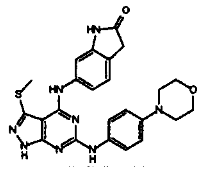 | HCl | 489 | 487 | 12 |
| 213 | 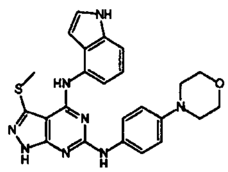 | HCl | 473 | 471 | 12 |
| 214 | 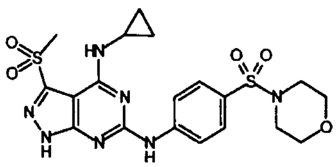 | Free | 494 | 492 | 1 |
| 215 | 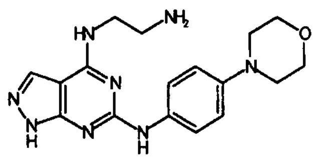 | Free | 355 | 353 | 9 |
| 216 | 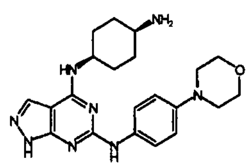 | Free | 409 | 407 | 9 |
| 217 | 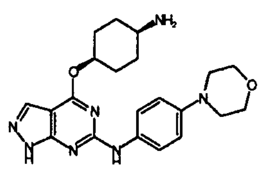 | Free | 410 | Not detected | 11 |
| 218 | 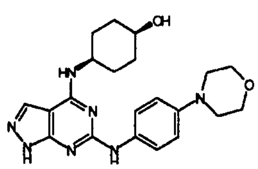 | Free | 410 | 408 | 8 |

[Table 1-31]

| Compound No. | Compound | Salt | ESI MS $(M+H)^+$ | ESI MS $(M-H)^-$ | Example No. |
|---|---|---|---|---|---|
| 219 | 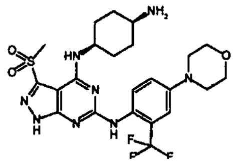 | Free | 555 | 553 | 4 |
| 220 | 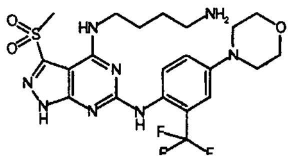 | Free | 529 | 527 | 4 |
| 221 | 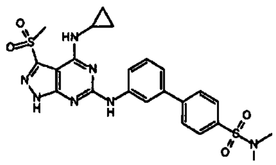 | Free | Not detected | 526 | 1 |
| 222 | 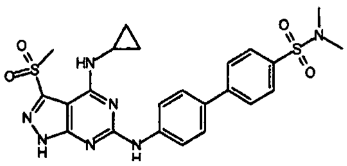 | Free | 528 | 526 | 1 |
| 223 | 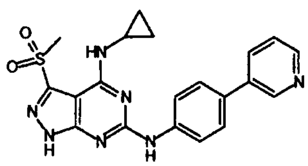 | Free | 422 | 420 | 1 |
| 224 | 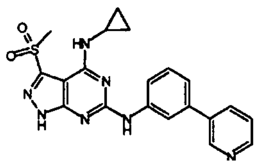 | Free | 422 | Not detected | 1 |
| 225 | 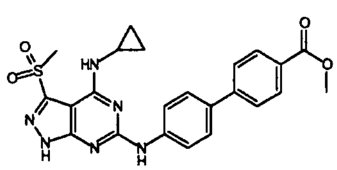 | Free | 479 | 477 | 1 |
| 226 | 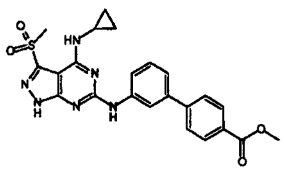 | Free | 479 | 477 | 1 |

(continued)

| Compound No. | Compound | Salt | ESI MS (M+H)+ | ESI MS (M-H)- | Example No. |
|---|---|---|---|---|---|
| 227 | 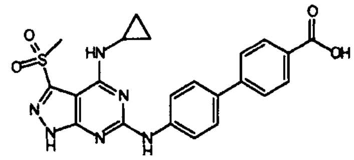 | HCl | 465 | 463 | 1 |

[Table 1-32]

| Compound No. | Compound | Salt | ESI MS (M+H)+ | ESI MS (M-H)- | Example No. |
|---|---|---|---|---|---|
| 228 | 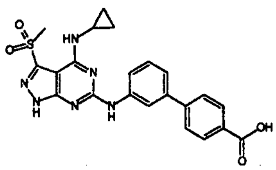 | HCl | 465 | 463 | 1 |
| 229 | 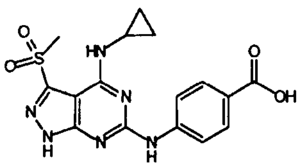 | Free | 389 | 387 | 1 |
| 230 | 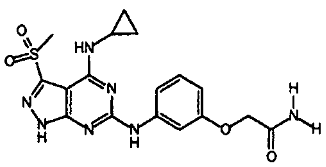 | Free | 418 | 416 | 1 |
| 231 | 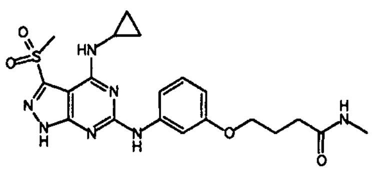 | Free | 460 | 458 | 1 |
| 232 | 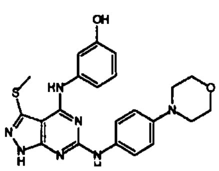 | Free | 450 | 448 | 12 |
| 233 | 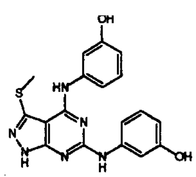 | Free | 381 | 379 | 12 |

(continued)

| Compound No. | Compound | Salt | ESI MS (M+H)+ | ESI MS (M-H)- | Example No. |
|---|---|---|---|---|---|
| 234 | 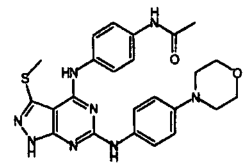 | Free | 491 | 489 | 12 |
| 235 | 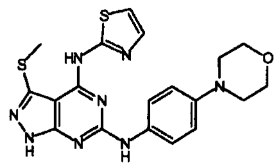 | Free | 441 | 439 | 14 |
| 236 | 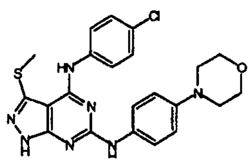 | Free | 468 | 466 | 12 |

[Table 1-33]

| Compound No. | Compound | Salt | ESI MS (M+H)+ | ESI MS (M-H)- | Example No. |
|---|---|---|---|---|---|
| 237 | 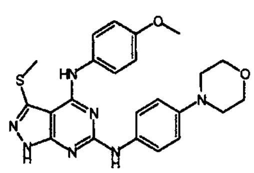 | Free | 464 | 462 | 12 |
| 238 | 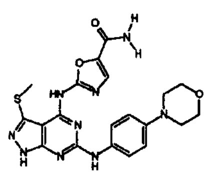 | Free | 468 | 466 | 14 |
| 239 | 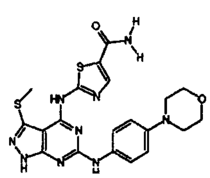 | Free | 484 | 482 | 14 |
| 240 | 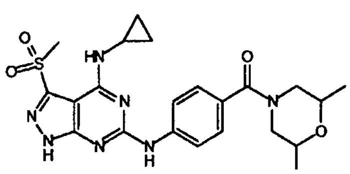 | Free | 486 | 484 | 1 |

(continued)

| Compound No. | Compound | Salt | ESI MS $(M+H)^+$ | ESI MS $(M-H)^-$ | Example No. |
|---|---|---|---|---|---|
| 241 | 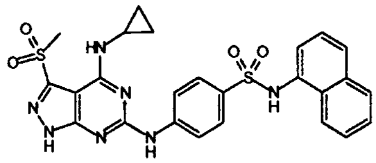 | Free | 550 | 548 | 1 |

Test Example

The effect of the compound of the present invention was confirmed by the following pharmacological test. The abbreviations in the Test Example are shown below.
ATP: Adenosine triphosphate
Tris: Tris(hydroxymethyl)aminomethane-hydrochloride buffer solution
DTT: Dithiothreitol
CHAPS: 3-[(3-Cholamidopropyl)dimethylammonio]propanesulfonate
BSA: Bovine serum albumin
PBS: Phosphate buffer solution
MOPS: 3-Morpholinopropanesulfonic acid
EDTA: Ethylenediaminetetraacetic acid
Brij-35: Polyoxyethylene (23) lauryl ether

1. Syk inhibition test

10 ng/10 μl/well of a Syk (Wako Pure Chemical Industries) solution, 15 μl of a poly(L-glu-L-tyr) substrate peptide solution (Sigma, final concentration 5 μM), 20 μl of an ATP mixture (ATP: Oriental Yeast, final concentration 5 μM, [γ-33P]ATP: GE Healthcare Biosciences, 0.1 μCi/well) and 5 μl of a test substance solution were added to a 96-well Corning reaction plate, followed by incubation at room temperature for 30 minutes. An assay buffer containing 50 mM Tris pH 7.5, 10 mM $MgCl_2$, 0.1 mM $Na_3VO_4$, 1 mM DTT, 0.01% CHAPS and 100 μg/ml BSA was used. The test substance solution was made by dissolving the test substance in DMSO at each concentration. The final concentration of DMSO was 1%. The reaction was terminated by adding 100 μl of a 120 mM phosphoric acid solution as a reaction terminator. Then, the total reaction solution was transferred to MultiScreen (Millipore) and washed with 200 μl of 100 mM phosphoric acid four times.
After drying, 20 μl of MicroScint-O (PerkinElmer) was added and the radioactivity was measured by TopCount (PerkinElmer). The ratio of the radioactivity when the test substance was added to the radioactivity when the test substance was not added was determined. The $IC_{50}$ value was calculated from the inhibition rate at each concentration and used as an index of inhibitory activity.
The compounds of Compound Nos. 6 and 112 had Syk inhibitory activity with $IC_{50}$ values of 0.16 μM and 0.020 μM, respectively, and the other compounds of Compound Nos. 74, 102, 107, 114, 115, 128, 130, 131, 134, 135, 138, 143 and 146 each had inhibitory activity with an $IC_{50}$ value of 0.2 μM or less.

2. Degranulation test using human mast cell line LAD cells

Human mast cell line LAD cells were sensitized with human IgE (Cosmo Bio; final concentration 1 μg/ml) overnight. The cells were harvested, and then washed with PBS (Invitrogen) and washed again with Tyrode's buffer solution (Sigma) containing 0.1% BSA (Sigma). The cells were seeded into a 96-well plate at $10^5$ cells/well and then the compound (0.1% final concentration DMSO solution) was added, followed by incubation at 37°C for 15 minutes. Rabbit anti-human IgE (Dako Japan; final concentration 10 μg/ml) or control Ig (Dako Japan; final concentration 10 μg/ml) was added, followed by incubation at 37°C for 30 minutes. The supernatant was recovered and p-nitrophenyl-2-acetamido-2-deoxy-β-D-glucopyranoside (Sigma; final concentration 4 mM), a substrate of β-hexosaminidase which is an index of degranulation, was added, followed by incubation at 37°C for 45 minutes. Glycine (Wako; final concentration 0.2 M, pH 10.4) was added and the reaction was terminated. Then, the absorbance at 405 nm was measured.
The inhibition rate (%) was calculated as (anti-IgE antibody degranulation rate - compound-added group degranulation rate)/(anti-IgE antibody degranulation rate - control antibody degranulation rate) × 100. The $IC_{50}$ value was

calculated from the inhibition rate at each concentration and used as an index of inhibitory activity.

The compounds of Compound Nos. 7 and 112 had inhibitory activity with $IC_{50}$ values of 0.024 μM and 0.019 μM, respectively, and the other compounds of Compound Nos. 4, 13, 74, 88, 102, 107, 128, 130, 131, 134, 135, 138 and 143 each had inhibitory activity with an $IC_{50}$ value of 0.1 μM or less.

3. Rat passive cutaneous anaphylaxis (PCA) test

The back of seven-week-old male Wistar rats was clipped under light anesthesia with ether, and the rats were sensitized by intradermal administration of 100 μl of 25 ng/ml anti-dinitrophenyl-IgE (DNP-IgE) (Sigma) to the back. 24 hours after the sensitization, 1 ml of a 0.5% Evans blue solution containing 1 mg of DNP-BSA was intravenously administered. Further, 30 minutes after the administration, the rats were sacrificed and the back skin was collected. The test compound, or only a vehicle as a control, was orally administered four hours prior to the antigen challenge. The length and breadth of the skin blue spot were calipered, and the area of the blue spot was calculated as length × breadth.

The PCA inhibition rate by the test substance was calculated by the following formula. In the formula,
C represents: a blue spot area by PCA reaction when only the vehicle was administered, and
X represents: a blue spot area by PCA reaction when the test compound was administered.

$$\text{Inhibition rate (\%)} = (C - X) \times 100/C$$

The compound of Compound No. 6 excellently inhibited PCA reaction.

4. Mouse collagen-induced arthritis (CIA) test

Seven-week-old male DBA/1 mice were sensitized by intradermal administration of bovine type II collagen/complete Freund's adjuvant emulsion (150 μg/0.1 ml/mouse) to the tail head. 21 days after the primary sensitization, the mice were similarly boosted by intradermal administration of bovine type II collagen/complete Freund's adjuvant emulsion (100 μg/0.1 ml/mouse) to the tail head. After the booster, the degree of arthritis was scored by observation for each extremity twice a week on the following scale.

0: No arthritis
1: Edema in one finger joint
2: Edema in two or more finger joints or instep
3: Edema in tarsus or ankle
4: Severe edema or joint deformation

Oral administration of the test compound, or only a vehicle as a control, was started 30 minutes prior to the booster, and administration was carried out for 42 consecutive days thereafter. For each day passed, the total score for the extremities was provided as an arthritis score for one individual (0 to 16), and the arthritis onset inhibitory effect was evaluated as an average value for eight cases using the vehicle-administered group as a control group.

The compound of Compound No. 7 excellently inhibited the onset of arthritis in the CIA model.

5. Abl inhibition test

5 ng/5 μl/well of an Abl (Wako Pure Chemical Industries) solution, 30 μl of an Abltide substrate peptide solution (Wako Pure Chemical Industries; final concentration 5 μM) and 5 μl of a test substance solution were added to a 96-well plate (Corning), followed by incubation at room temperature for 15 minutes. Then, 10 μl of an ATP mixture (ATP: Oriental Yeast, final concentration 6 μM, [γ-33P]ATP: GE Healthcare Biosciences, 0.06 μCi/well, MgAcetate : Cosmo Bio, final concentration 10 mM) was added, followed by incubation at room temperature for 40 minutes. The Abl solution was prepared using a kinase buffer (20 mM MOPS pH 7.0, 1 mM EDTA, 0.1% β-mercaptoethanol, 0.01% Brij-35, 5% glycerol, 1 mg/ml BSA). The substrate peptide solution and the ATP mixture were prepared using 8 mM MOPS pH 7.0 and 0.2 mM EDTA. The test substance solution was made by dissolving the test substance in DMSO at each concentration. The final concentration of DMSO was 1%. The reaction was terminated by adding 100 μl of a 100 mM phosphoric acid solution as a reaction terminator. Then, the total reaction solution was transferred to a 96-well MultiScreen plate (Millipore) and washed with 200 μl of 100 mM phosphoric acid three times. After drying, 20 μl of MicroScint-O (PerkinElmer) was added and the radioactivity was measured by TopCount (PerkinElmer). After subtracting the radioactivity

in the well to which the Abltide substrate peptide was not added from each radioactivity, the ratio of the radioactivity when the test substance was added to the radioactivity when the test substance was not added was determined. The $IC_{50}$ value was calculated from the inhibition rate at each concentration and used as an index of inhibitory activity.

The compounds of Compound Nos. 55, 74, 78, 112, 115, 117, 119, 126, 130, 131, 132, 134, 143, 146, 152, 200, 216 and 217 each had inhibitory activity with an $IC_{50}$ value of 10 nM or less. The compounds of Compound Nos. 6, 21, 38, 60, 102, 103, 107, 108, 110, 114, 116, 128, 150, 188, 192, 206, 208, 227 and 240 each had inhibitory activity with an $IC_{50}$ value of 50 nM or less.

From the test results of 1 to 4 above, it was confirmed that the compound of the present invention has a Syk inhibitory effect and a degranulation inhibitory effect and inhibits anaphylaxis and arthritis. Therefore, the compound is assumed to be useful as a prophylactic or therapeutic agent for diseases involving Syk such as allergic disease, autoimmune disease and arthritis.

From the test results of 5 above, it was confirmed that the compound of the present invention has an Abl inhibitory effect. Therefore, the compound is assumed to be useful as a prophylactic or therapeutic agent for diseases involving Abl such as cancer.

Formulation Examples are shown below.

Formulation Example 1

Granules containing the following ingredients are prepared.

| | |
|---|---|
| Ingredients Compound represented by formula [I] | 10 mg |
| Lactose | 700 mg |
| Corn starch | 274 mg |
| HPC-L | 16 mg |
| | 1000 mg |

The compound represented by the formula [I] and lactose are allowed to pass through a 60-mesh sieve. Corn starch is allowed to pass through a 120-mesh sieve. They are mixed in a V-shape mixer. A low-viscosity hydroxypropylcellulose (HPC-L) solution is added to the mixed powder. The mixture is kneaded, granulated (extrusion granulation, pore size 0.5 to 1 mm) and then dried. The resulting dry granules are sieved through a vibrating sieve (12/60 mesh) to obtain granules.

Formulation Example 2

Encapsulation powder containing the following ingredients is prepared.

| | |
|---|---|
| Ingredients Compound represented by formula [I] | 10 mg |
| Lactose | 79 mg |
| Corn starch | 10 mg |
| Magnesium stearate | 1 mg |
| | 100 mg |

The compound represented by the formula [I] and lactose are allowed to pass through a 60-mesh sieve. Corn starch is allowed to pass through a 120-mesh sieve. These ingredients and magnesium stearate are mixed in a V-shape mixer. A No. 5 hard gelatin capsule is filled with 100 mg of the 10% powder.

Formulation Example 3

Encapsulation granules containing the following ingredients are prepared.

| | |
|---|---|
| Ingredients Compound represented by formula [I] | 15 mg |
| Lactose | 90 mg |
| Corn starch | 42 mg |
| HPC-L | 3 mg |
| | 150 mg |

The compound represented by the formula [I] and lactose are allowed to pass through a 60-mesh sieve. Corn starch is allowed to pass through a 120-mesh sieve. They are mixed in a V-shape mixer. A low-viscosity hydroxypropylcellulose (HPC-L) solution is added to the mixed powder. The mixture is kneaded, granulated and then dried. The resulting dry granules are sieved and size-regulated through a vibrating sieve (12/60 mesh) and a No. 4 hard gelatin capsule is filled with 150 mg of the resulting granules.

Formulation Example 4

A tablet containing the following ingredients is prepared.

| | |
|---|---|
| Ingredients Compound represented by formula [I] | 10 mg |
| Lactose | 90 mg |
| Microcrystalline cellulose | 30 mg |
| Magnesium stearate | 5 mg |
| CMC-Na | 15 mg |
| | 150 mg |

The compound represented by the formula [I], lactose, microcrystalline cellulose and CMC-Na (sodium carboxymethylcellulose) are allowed to pass through a 60-mesh sieve and mixed. Magnesium stearate is added to the mixed powder to obtain a mixed powder for formulation. The mixed powder is directly compressed to obtain 150 mg of a tablet.

Formulation Example 5

An intravenous formulation is prepared as follows.

| | |
|---|---|
| Compound represented by formula [I] | 100 mg |
| Saturated fatty acid glyceride | 1000 ml |

Typically, the solution having the above ingredients is intravenously administered to a patient at a rate of 1 ml per minute.

INDUSTRIAL APPLICABILITY

The compound of the present invention has Syk and/or Abl inhibitory activity and can be used as a prophylactic or therapeutic agent for Syk- and/or Abl-related diseases, specifically, a prophylactic or therapeutic agent for diseases such as allergic disease, autoimmune disease and arthritis, or cancer.

## Claims

**1.** A pyrazolopyrimidine compound represented by the formula [I]:

[Formula 1]

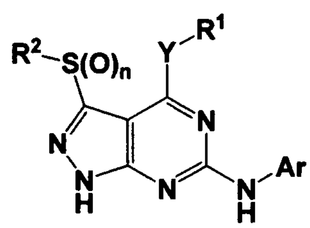

[I]

wherein

$R^1$ represents a $C_{1-8}$ alkyl group, a $C_{3-8}$ cycloalkyl group, a $C_{2-9}$ heterocyclyl group, a $C_{1-9}$ heteroaryl group or

a phenyl group (wherein the $C_{1-8}$ alkyl group, the $C_{3-8}$ cycloalkyl group, the $C_{2-9}$ heterocyclyl group, the $C_{1-9}$ heteroaryl group and the phenyl group are unsubstituted or substituted with 1 to 4 substituents selected from the following Substituent Group $A^1$);
Substituent Group $A^1$ represents
a group consisting of a halogen atom, a hydroxy group, an amino group, a cyano group, a carboxy group, a $C_{1-6}$ alkyl group, a trifluoromethyl group, a $C_{3-8}$ cycloalkyl group, a $C_{1-6}$ alkoxy group (wherein the $C_{1-6}$ alkoxy group is unsubstituted or substituted with a hydroxy group), a sulfanyl group, a $C_{1-6}$ alkylsulfanyl group, a $C_{1-6}$ alkoxycarbonyl group, a $C_{3-8}$ cycloalkoxy group, a ($C_{1-6}$ alkyl) amino group, a di($C_{1-6}$ alkyl) amino group, a $C_{1-6}$ alkoxycarbonylamino group, a hydroxyaminocarbonyl group, a ureido group, a carbamoyl group, a $C_{2-9}$ heterocyclyl group (wherein the $C_{2-9}$ heterocyclyl group is unsubstituted or substituted with a $C_{1-6}$ alkyl group or an oxo group), a $C_{1-9}$ heteroaryl group, a ($C_{1-6}$ acyl)amino group and an oxo group;
$R^2$ represents a $C_{1-6}$ alkyl group;
n represents an integer of 0, 1 or 2;
Y represents -O- or -$NR^3$- (wherein $R^3$ represents a hydrogen atom or a $C_{1-6}$ alkyl group);
Ar represents a $C_{1-9}$ heteroaryl group (wherein the $C_{1-9}$ heteroaryl group is unsubstituted or substituted with 1 to 3 substituents selected from the group consisting of a sulfanyl group, a hydroxy group, a $C_{1-6}$ alkoxy group, a morpholinyl group, a trifluoromethyl group and an oxo group) or a phenyl group (wherein the phenyl group is unsubstituted or substituted with the same or different 1 to 3 $R^a$s); and
$R^a$ represents a hydroxy group, a cyano group, a carboxy group, a sulfanyl group, a $C_{1-6}$ alkoxycarbonyl group, a $C_{1-6}$ alkyl group (wherein the $C_{1-6}$ alkyl group is unsubstituted or substituted with a carboxy group or - $CONR^4R^5$ (wherein $R^4$ and $R^5$ are the same or different and each represent a hydrogen atom or a $C_{1-6}$ alkyl group)), a $C_{3-8}$ cycloalkyl group, a $C_{1-6}$ alkoxy group (wherein the $C_{1-6}$ alkoxy group is unsubstituted or substituted with a carboxy group or -$CONR^6R^7$ (wherein $R^6$ and $R^7$ are the same or different and each represent a hydrogen atom, a $C_{1-6}$ alkyl group, a $C_{3-8}$ cycloalkyl group, a $C_{1-6}$ alkoxy group, a $C_{2-9}$ heterocyclyl group or a $C_{1-9}$ heteroaryl group)), a $C_{1-9}$ heteroaryl group, a $C_{3-8}$ cycloalkoxy group, a halogen atom, a trifluoromethyl group, a trifluoromethoxy group, a phenoxy group, a phenyl group (wherein the phenyl group is unsubstituted or substituted with 1 to 3 substituents selected from the group consisting of a carboxy group, a $C_{1-6}$ alkoxycarbonyl group and -$SO_2NR^{14}R^{15}$ (wherein $R^{14}$ and $R^{15}$ are the same or different and each represent a hydrogen atom or a $C_{1-6}$ alkyl group)), -$NR^8R^9$, -$CONR^8R^9$, -$NR^8SO_2R^9$ or -$SO_2NR^8R^9$ (wherein $R^8$ and $R^9$ are the same or different and each represent a hydrogen atom, a $C_{1-6}$ acyl group, a $C_{1-6}$ alkoxycarbonyl group, a toluyl group, a naphthyl group, a $C_{1-9}$ heteroaryl group or a $C_{1-6}$ alkyl group (wherein the $C_{1-6}$ alkyl group is unsubstituted or substituted with 1 to 3 substituents selected from the group consisting of a hydroxy group, a carboxy group and a $C_{1-6}$ alkoxy group),
or when $R^8$ and $R^9$ are substituents on the adjacent nitrogen atom, they, together with the adjacent nitrogen atom, represent the formula [II]:

[Formula 2]

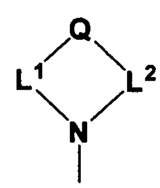

[II]

wherein Q represents -O-, -$NR^{10}$-, -$CHR^{11}$-, -CO- (wherein the -CO- is unprotected or protected with ethylene ketal), -S-, -SO- or -$SO_2$-;
$L^1$ and $L^2$ are the same or different and each represent a linear $C_{1-3}$ alkylene group (wherein the linear $C_{1-3}$ alkylene group is unsubstituted or substituted with 1 to 3 substituents selected from the group consisting of a $C_{1-6}$ alkyl group and an oxo group);
$R^{10}$ represents a hydrogen atom, a $C_{1-6}$ acyl group, a $C_{1-6}$ alkyl group, a $C_{1-6}$ alkylsulfonyl group, a trifluoromethylsulfonyl group, a phenylcarbonyl group or a phenylsulfonyl group (wherein the phenylcarbonyl group and the phenylsulfonyl group are unsubstituted or substituted with 1 to 2 substituents selected from Substituent Group B);
Substituent Group B represents a group consisting of a halogen atom, a $C_{1-6}$ alkyl group and a $C_{1-6}$ alkoxy

group; and
$R^{11}$ represents a hydrogen atom, a hydroxy group, a $C_{1-6}$ alkyl group (wherein the $C_{1-6}$ alkyl group is unsubstituted or substituted with a $C_{1-6}$ alkoxy group or a hydroxy group), a $C_{1-6}$ alkoxy group, a carboxy group, an amino group, a ($C_{1-6}$ alkyl) amino group, a di($C_{1-6}$ alkyl)amino group, a ($C_{1-6}$ acyl)amino group, a phenylcarbonyl group, $-CONR^{12}R^{13}$ (wherein $R^{12}$ and $R^{13}$ are the same or different and each represent a hydrogen atom or a $C_{1-6}$ alkyl group, or they, together with the adjacent nitrogen atom, represent a $C_{2-9}$ heterocyclyl group) or a ($C_{1-6}$ alkylsulfonyl)amino group);
a tautomer or a stereoisomer of the compound, or a pharmaceutically acceptable salt thereof, or a solvate thereof.

**2.** The pyrazolopyrimidine compound, a tautomer or a stereoisomer of the compound, or a pharmaceutically acceptable salt thereof, or a solvate thereof, according to claim 1, wherein $R^1$ is a $C_{1-8}$ alkyl group, a $C_{3-8}$ cycloalkyl group or a $C_{2-9}$ heterocyclyl group (wherein the $C_{1-8}$ alkyl group, the $C_{3-8}$ cycloalkyl group and the $C_{2-9}$ heterocyclyl group are unsubstituted or substituted with 1 to 4 substituents selected from the following Substituent Group A);
Substituent Group A represents
a group consisting of a halogen atom, a hydroxy group, an amino group, a cyano group, a carboxy group, a $C_{1-6}$ alkyl group, a $C_{3-8}$ cycloalkyl group, a $C_{1-6}$ alkoxy group (wherein the $C_{1-6}$ alkoxy group is unsubstituted or substituted with a hydroxy group), a $C_{1-6}$ alkylsulfanyl group, a $C_{1-6}$ alkoxycarbonyl group, a $C_{3-8}$ cycloalkoxy group, a ($C_{1-6}$ alkyl)amino group, a di($C_{1-6}$ alkyl)amino group, a $C_{1-6}$ alkoxycarbonylamino group, a hydroxyaminocarbonyl group, a ureido group, a carbamoyl group, a $C_{2-9}$ heterocyclyl group (wherein the $C_{2-9}$ heterocyclyl group is unsubstituted or substituted with a $C_{1-6}$ alkyl group or an oxo group) and a $C_{1-9}$ heteroaryl group;
$R^2$ is a linear $C_{1-6}$ alkyl group;
Ar is a $C_{1-9}$ heteroaryl group (wherein the $C_{1-9}$ heteroaryl group is unsubstituted or substituted with 1 to 3 substituents selected from the group consisting of a sulfanyl group, a hydroxy group, a $C_{1-6}$ alkoxy group, a morpholinyl group and a trifluoromethyl group) or a phenyl group (wherein the phenyl group is unsubstituted or substituted with the same or different 1 to 3 $R^a$s); and
$R^a$ is a hydroxy group, a cyano group, a carboxy group, a sulfanyl group, a $C_{1-6}$ alkoxycarbonyl group, a $C_{1-6}$ alkyl group (wherein the $C_{1-6}$ alkyl group is unsubstituted or substituted with a carboxy group or $-CONR^4R^5$ (wherein $R^4$ and $R^5$ are the same or different and each represent a hydrogen atom or a $C_{1-6}$ alkyl group)), a $C_{3-8}$ cycloalkyl group, a $C_{1-6}$ alkoxy group (wherein the $C_{1-6}$ alkoxy group is unsubstituted or substituted with a carboxy group or $-CONR^6R^7$ (wherein $R^6$ and $R^7$ are the same or different and each represent a hydrogen atom, a $C_{1-6}$ alkyl group, a $C_{3-8}$ cycloalkyl group, a $C_{1-6}$ alkoxy group, a $C_{2-9}$ heterocyclyl group or a $C_{1-9}$ heteroaryl group)), a $C_{1-9}$ heteroaryl group, a $C_{3-8}$ cycloalkoxy group, a halogen atom, a trifluoromethyl group, a trifluoromethoxy group, a phenoxy group, $-NR^8R^9$, $-CONR^8R^9$, $-NR^8SO_2R^9$ or $-SO_2NR^8R^9$ (wherein $R^8$ and $R^9$ are the same or different and each represent a hydrogen atom, a $C_{1-6}$ acyl group, a $C_{1-6}$ alkoxycarbonyl group, a toluyl group, a $C_{1-9}$ heteroaryl group or a $C_{1-6}$ alkyl group (wherein the $C_{1-6}$ alkyl group is unsubstituted or substituted with 1 to 3 substituents selected from the group consisting of a hydroxy group, a carboxy group and a $C_{1-6}$ alkoxy group), or when $R^8$ and $R^9$ are substituents on the adjacent nitrogen atom, they, together with the adjacent nitrogen atom, represent the formula [II]:

[Formula 3]

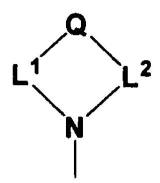

[II]

wherein Q represents -O-, $-NR^{10}-$, $-CHR^{11}-$, -CO- (wherein the -CO- is unprotected or protected with ethylene ketal), -S-, -SO- or $-SO_2-$;
$L^1$ and $L^2$ are the same or different and each represent a linear $C_{1-3}$ alkylene group (wherein the linear $C_{1-3}$ alkylene group is unsubstituted or substituted with 1 to 3 substituents selected from the group consisting of a $C_{1-6}$ alkyl group and an oxo group);
$R^{10}$ represents a hydrogen atom, a $C_{1-6}$ acyl group, a $C_{1-6}$ alkyl group, a $C_{1-6}$ alkylsulfonyl group, a trifluoromethylsulfonyl group, a phenylcarbonyl group or a phenylsulfonyl group (wherein the phenylcarbonyl group and the phenylsulfonyl group are unsubstituted or substituted with 1 to 2 substituents selected from Substituent Group B);

Substituent Group B represents a group consisting of a halogen atom, a $C_{1-6}$ alkyl group and a $C_{1-6}$ alkoxy group; and $R^{11}$ represents a hydrogen atom, a hydroxy group, a $C_{1-6}$ alkyl group (wherein the $C_{1-6}$ alkyl group is unsubstituted or substituted with a $C_{1-6}$ alkoxy group or a hydroxy group), a $C_{1-6}$ alkoxy group, a carboxy group, an amino group, a ($C_{1-6}$ alkyl)amino group, a di($C_{1-6}$ alkyl)amino group, a ($C_{1-6}$ acyl)amino group, a phenylcarbonyl group, $-CONR^{12}R^{13}$ (wherein $R^{12}$ and $R^{13}$ are the same or different and each represent a hydrogen atom or a $C_{1-6}$ alkyl group, or they, together with the adjacent nitrogen atom, represent a $C_{2-9}$ heterocyclyl group) or a ($C_{1-6}$ alkylsulfonyl) amino group).

**3.** The pyrazolopyrimidine compound, a tautomer or a stereoisomer of the compound, or a pharmaceutically acceptable salt thereof, or a solvate thereof according to claim 2, wherein Ar is a phenyl group (wherein the phenyl group is unsubstituted or substituted with the same or different 1 to 3 $R^a$s).

**4.** The pyrazolopyrimidine compound, a tautomer or a stereoisomer of the compound, or a pharmaceutically acceptable salt thereof, or a solvate thereof according to claim 2, wherein Ar is a phenyl group (wherein the phenyl group is substituted at the 3-, 4- or 5-position with the same or different 1 to 3 $R^a$s).

**5.** The pyrazolopyrimidine compound, a tautomer or a stereoisomer of the compound, or a pharmaceutically acceptable salt thereof, or a solvate thereof according to any one of claims 2 to 4, wherein Y is NH.

**6.** The pyrazolopyrimidine compound, a tautomer or a stereoisomer of the compound, or a pharmaceutically acceptable salt thereof, or a solvate thereof according to any one of claims 2 to 5, wherein $R^1$ is a $C_{1-8}$ alkyl group (wherein the $C_{1-8}$ alkyl group is unsubstituted or substituted with an amino group, a ($C_{1-6}$ alkyl)amino group or a di($C_{1-6}$ alkyl) amino group).

**7.** The pyrazolopyrimidine compound, a tautomer or a stereoisomer of the compound, or a pharmaceutically acceptable salt thereof, or a solvate thereof according to any one of claims 2 to 5, wherein $R^1$ is a $C_{3-8}$ cycloalkyl group (wherein the $C_{3-8}$ cycloalkyl group is unsubstituted or substituted with an amino group).

**8.** The pyrazolopyrimidine compound, a tautomer or a stereoisomer of the compound, or a pharmaceutically acceptable salt thereof, or a solvate thereof according to claim 1, wherein $R^1$ is a $C_{1-9}$ heteroaryl group or a phenyl group (wherein the $C_{1-9}$ heteroaryl group and the phenyl group are unsubstituted or substituted with 1 to 3 substituents selected from the group consisting of a halogen atom, a hydroxy group, a cyano group, a $C_{1-6}$ alkyl group, a trifluoromethyl group, a $C_{1-6}$ alkoxy group, a sulfanyl group, a carbamoyl group, a ($C_{1-6}$ acyl)amino group and an oxo group), and Y is NH.

**9.** A pyrazolopyrimidine compound represented by the formula [III]:

[Formula 4]

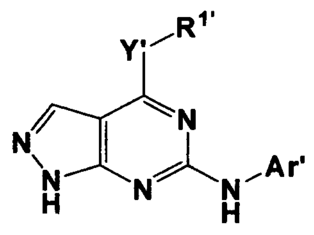

[III]

wherein

$R^{1'}$ represents a $C_{1-8}$ alkyl group or a $C_{3-8}$ cycloalkyl group (wherein the $C_{1-8}$ alkyl group and the $C_{3-8}$ cycloalkyl group are unsubstituted or substituted with 1 to 4 substituents selected from the following Substituent Group A');
Substituent Group A' represents
a group consisting of a halogen atom, a hydroxy group, an amino group, a cyano group, a carboxy group, a $C_{1-6}$ alkyl group, a trifluoromethyl group, a $C_{3-8}$ cycloalkyl group, a $C_{1-6}$ alkoxy group (wherein the $C_{1-6}$ alkoxy group is unsubstituted or substituted with a hydroxy group), a sulfanyl group, a $C_{1-6}$ alkylsulfanyl group, a $C_{1-6}$ alkoxycarbonyl group, a $C_{3-8}$ cycloalkoxy group, a ($C_{1-6}$ alkyl) amino group, a di ($C_{1-6}$ alkyl) amino group, a

$C_{1-6}$ alkoxycarbonylamino group, a hydroxyaminocarbonyl group, a ureido group, a carbamoyl group, a $C_{2-9}$ heterocyclyl group (wherein the $C_{2-9}$ heterocyclyl group is unsubstituted or substituted with a $C_{1-6}$ alkyl group or an oxo group), a $C_{1-9}$ heteroaryl group, a ($C_{1-6}$ acyl)amino group and an oxo group;

Y' represents -O- or -$NR^{16}$- (wherein $R^{16}$ represents a hydrogen atom or a $C_{1-6}$ alkyl group);

Ar' represents a $C_{1-9}$ heteroaryl group (wherein the $C_{1-9}$ heteroaryl group is unsubstituted or substituted with 1 to 3 substituents selected from the group consisting of a sulfanyl group, a hydroxy group, a $C_{1-6}$ alkoxy group, a morpholinyl group, a trifluoromethyl group and an oxo group) or a phenyl group (wherein the phenyl group is unsubstituted or substituted with the same or different 1 to 3 $R^b$s); and

$R^b$ represents a hydroxy group, a cyano group, a carboxy group, a sulfanyl group, a $C_{1-6}$ alkoxycarbonyl group, a $C_{1-6}$ alkyl group (wherein the $C_{1-6}$ alkyl group is unsubstituted or substituted with a carboxy group or -$CONR^{17}R^{18}$ (wherein $R^{17}$ and $R^{18}$ are the same or different and each represent a hydrogen atom or a $C_{1-6}$ alkyl group)), a $C_{3-8}$ cycloalkyl group, a $C_{1-6}$ alkoxy group (wherein the $C_{1-6}$ alkoxy group is unsubstituted or substituted with a carboxy group or -$CONR^{19}R^{20}$ (wherein $R^{19}$ and $R^{20}$ are the same or different and each represent a hydrogen atom, a $C_{1-6}$ alkyl group, a $C_{3-8}$ cycloalkyl group, a $C_{1-6}$ alkoxy group, a $C_{2-9}$ heterocyclyl group or a $C_{1-9}$ heteroaryl group)), a $C_{1-9}$ heteroaryl group, a $C_{3-8}$ cycloalkoxy group, a halogen atom, a trifluoromethyl group, a trifluoromethoxy group, a phenoxy group, a phenyl group (wherein the phenyl group is unsubstituted or substituted with a substituent selected from a carboxy group, a $C_{1-6}$ alkoxycarbonyl group and -$SO_2NR^{21}R^{22}$ (wherein $R^{21}$ and $R^{22}$ are the same or different and each represent a hydrogen atom or a $C_{1-6}$ alkyl group)), - $NR^{23}R^{24}$, -$CONR^{23}R^{24}$, -$NR^{23}SO_2R^{24}$ or -$SO_2NR^{23}R^{24}$ (wherein $R^{23}$ and $R^{24}$ are the same or different and each represent a hydrogen atom, a $C_{1-6}$ acyl group, a $C_{1-6}$ alkoxycarbonyl group, a toluyl group, a naphthyl group, a $C_{1-9}$ heteroaryl group or a $C_{1-6}$ alkyl group (wherein the $C_{1-6}$ alkyl group is unsubstituted or substituted with 1 to 3 substituents selected from the group consisting of a hydroxy group, a carboxy group and a $C_{1-6}$ alkoxy group), or when $R^{23}$ and $R^{24}$ are substituents on the adjacent nitrogen atom, they, together with the adjacent nitrogen atom, represent the formula [IV]:

[Formula 5]

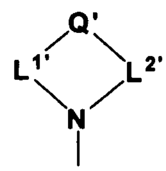

[IV]

wherein Q' represents -O-, -$NR^{25}$- , -$CHR^{26}$-, -CO- (wherein the -CO- is unprotected or protected with ethylene ketal), -S-, -SO- or -$SO_2$-;

$L^{1'}$ and $L^{2'}$ are the same or different and each represent a linear $C_{1-3}$ alkylene group (wherein the linear $C_{1-3}$ alkylene group is unsubstituted or substituted with 1 to 3 substituents selected from the group consisting of a $C_{1-6}$ alkyl group and an oxo group);

$R^{25}$ represents a hydrogen atom, a $C_{1-6}$ acyl group, a $C_{1-6}$ alkyl group, a $C_{1-6}$ alkylsulfonyl group, a trifluoromethylsulfonyl group, a phenylcarbonyl group or a phenylsulfonyl group (wherein the phenylcarbonyl group and the phenylsulfonyl group are unsubstituted or substituted with 1 to 2 substituents selected from Substituent Group B');

Substituent Group B' represents a group consisting of a halogen atom, a $C_{1-6}$ alkyl group and a $C_{1-6}$ alkoxy group; and

$R^{26}$ represents a hydrogen atom, a hydroxy group, a $C_{1-6}$ alkyl group (wherein the $C_{1-6}$ alkyl group is unsubstituted or substituted with a $C_{1-6}$ alkoxy group or a hydroxy group), a $C_{1-6}$ alkoxy group, a carboxy group, an amino group, a ($C_{1-6}$ alkyl) amino group, a di($C_{1-6}$ alkyl)amino group, a ($C_{1-6}$ acyl)amino group, a phenylcarbonyl group, -$CONR^{27}R^{28}$ (wherein $R^{27}$ and $R^{28}$ are the same or different and each represent a hydrogen atom or a $C_{1-6}$ alkyl group, or they, together with the adjacent nitrogen atom, represent a $C_{2-9}$ heterocyclyl group) or a ($C_{1-6}$ alkylsulfonyl)amino group);

a tautomer or a stereoisomer of the compound, or a pharmaceutically acceptable salt thereof, or a solvate thereof.

**10.** The pyrazolopyrimidine compound, a tautomer or a stereoisomer of the compound, or a pharmaceutically acceptable salt thereof, or a solvate thereof according to claim 9, wherein $R^{1'}$ is a $C_{1-8}$ alkyl group (wherein the $C_{1-8}$ alkyl group is unsubstituted or substituted with an amino group, a cyano group, a ($C_{1-6}$ alkyl)amino group or a di($C_{1-6}$ alkyl) amino group).

**11.** The pyrazolopyrimidine compound, a tautomer or a stereoisomer of the compound, or a pharmaceutically acceptable salt thereof, or a solvate thereof according to claim 9, wherein $R^{1'}$ is a $C_{3-8}$ cycloalkyl group (wherein the $C_{3-8}$ cycloalkyl group is unsubstituted or substituted with a hydroxy group or an amino group).

**12.** A Syk inhibitor comprising, as an active ingredient, the pyrazolopyrimidine compound, a tautomer or a stereoisomer of the compound, or a pharmaceutically acceptable salt thereof, or a solvate thereof according to any one of claims 1 to 11.

**13.** A therapeutic agent or prophylactic agent for allergic disease, autoimmune disease or arthritis, comprising, as an active ingredient, the pyrazolopyrimidine compound, a tautomer or a stereoisomer of the compound, or a pharmaceutically acceptable salt thereof, or a solvate thereof according to any one of claims 1 to 11.

**14.** An Abl inhibitor comprising, as an active ingredient, the pyrazolopyrimidine compound, a tautomer or a stereoisomer of the compound, or a pharmaceutically acceptable salt thereof, or a solvate thereof according to any one of claims 1 to 11.

**15.** A therapeutic agent or prophylactic agent for cancer, comprising, as an active ingredient, the pyrazolopyrimidine compound, a tautomer or a stereoisomer of the compound, or a pharmaceutically acceptable salt thereof, or a solvate thereof according to any one of claims 1 to 11.

**INTERNATIONAL SEARCH REPORT**

International application No.
PCT/JP2007/075278

A. CLASSIFICATION OF SUBJECT MATTER
*C07D487/04*(2006.01)i, *A61K31/519*(2006.01)i, *A61K31/5377*(2006.01)i, *A61K31/538*(2006.01)i, *A61K31/541*(2006.01)i, *A61K31/55*(2006.01)i, *A61K31/553*(2006.01)i, *A61P19/02*(2006.01)i, *A61P37/06*(2006.01)i,

According to International Patent Classification (IPC) or to both national classification and IPC

B. FIELDS SEARCHED

Minimum documentation searched (classification system followed by classification symbols)
C07D487/04, A61K31/519, A61K31/5377, A61K31/538, A61K31/541, A61K31/55, A61K31/553, A61P19/02, A61P37/06, A61P37/08, A61P43/00, C07D519/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
CAOLD(STN), CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>A | WO 2005/085249 A1 (F. HOFFMANN-LA ROCHE AG.), 15 September, 2005 (15.09.05), Full text; particularly, Claims & US 2005/197340 A1 & EP 1737865 A1 & JP 2007-523938 A | 1,3-8,12,13<br>2,9-11,14,15 |
| A | JP 2001-501216 A (Novartis AG.), 30 January, 2001 (30.01.01), Full text; particularly, Claims & WO 98/014450 A1 & EP 929553 A1 & US 6251911 B1 | 1-15 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 15 February, 2008 (15.02.08) | 26 February, 2008 (26.02.08) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.
PCT/JP2007/075278

C (Continuation). DOCUMENTS CONSIDERED TO BE RELEVANT

| Category* | Citation of document, with indication, where appropriate, of the relevant passages | Relevant to claim No. |
|---|---|---|
| A | JP 2006-516561 A (Warner-Lambert Company L.L.C.), 06 July, 2006 (06.07.06), Full text; particularly, Claims & WO 2004/065378 A1 & US 2004/236084 A1 & EP 1590341 A1 | 1-15 |
| A | JP 2003-506375 A (Novartis AG.), 18 February, 2003 (18.02.03), Full text; particularly, Claims & WO 2001/009134 A1 & EP 1200435 A1 & US 6589950 B1 | 1-15 |
| A | WO 2005/080393 A1 (IRM L.L.C.), 01 September, 2005 (01.09.05), Full text; particularly, Claims & EP 1713806 A1 & JP 2007-522241 A & US 2007/225306 A1 | 1-15 |

Form PCT/ISA/210 (continuation of second sheet) (April 2007)

**INTERNATIONAL SEARCH REPORT**

International application No.
PCT/JP2007/075278

Continuation of A. CLASSIFICATION OF SUBJECT MATTER
(International Patent Classification (IPC))

*A61P37/08*(2006.01)i, *A61P43/00*(2006.01)i, *C07D519/00*(2006.01)i

(According to International Patent Classification (IPC) or to both national classification and IPC)

Form PCT/ISA/210 (extra sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0183485 A
- WO 0357695 A
- WO 0075113 A
- WO 0109134 A
- JP 2004203748 A
- WO 06047256 A
- WO 06093247 A
- WO 03063794 A
- WO 05085249 A
- WO 05028480 A
- WO 07070872 A
- JP 48099194 A


### Non-patent literature cited in the description


- *Oncogene,* 19 December 1996, vol. 13 (12), 2595-2605
- *J. Pharmacol. Exp. Ther.,* September 2003, vol. 306 (3), 1174-1181
- *J. Immunol.,* 15 July 2002, vol. 169 (2), 1028-1036
- *Proc. Natl. Acad. Sci. USA.,* 20 April 2004, vol. 101 (16), 6158-6163
- *J. Pharmacol. Exp. Ther.,* May 2006, vol. 317 (2), 571-578
- *J. Pharmacol. Exp. Ther.,* 31 August 2006
- *Bioorganic & Medicinal Chemistry Letters,* 2003, vol. 13 (8), 1415
- *Bioorganic & Medicinal Chemistry,* 2005, vol. 13, 4936
- *Oncogene,* 15 November 2007, vol. 26 (52), 7313-7323
- *Cancer Res.,* 01 June 2006, vol. 66 (11), 5648-5655
- *Cancer Res.,* 15 February 2007, vol. 67 (4), 1580-1588
- DESIGN OF PRODRUGS. Elsevier, 1985